# EUROPEAN PATENT APPLICATION

(11) **EP 4 582 146 A2**
(43) Date of publication of application: **09.07.2025**
(21) Application number: 25169707.4
(22) Date of filing: 25.07.2018
(51) Int. Cl.: A61P 35/00

(54) **ENHANCED CHIMERIC ANTIGEN RECEPTORS AND USE THEREOF**

(30) Priority: 25.07.2017 US 201762536934 P
(62) Divisional of application: 18838056.2
(71) Applicant: Board of Regents, The University of Texas System, Austin, TX 78701 (US)
(72) Inventor: ANG, Sonny Oon, Houston, 77030 (US); LIU, Enli, Houston, 77030 (US); SHPALL, Elizabeth, Houston, 77030 (US); REZVANI, Katy, Houston, 77030 (US)
(74) Representative: J A Kemp LLP

(57) **Abstract**

Provided herein are chimeric antigen receptors (CARs) comprising a truncated EGFRvIII (Ev3) in the hinge region of the CAR and/or a humanized scFv. Further provided herein are immune cells expressing the CARs as well as methods of their use in the treatment of immune disorders.

## Description

The present application claims the priority benefit of United States Provisional Application Serial No. 62/536,934, filed July 25, 2017, the entire contents of which are hereby incorporated by reference.

### INCORPORATION OF SEQUENCE LISTING

The sequence listing that is contained in the file named "UTFCP1331WO.txt", which is 289 KB (as measured in Microsoft Windows) and was created on July 23, 2018, is filed herewith by electronic submission and is incorporated by reference herein.

### BACKGROUND

### 1. Field

The present invention relates generally to the fields of immunology and molecular biology. More particularly, it concerns enhanced chimeric antigen receptors (CARs), such as for immune cells.

### 2. Description of Related Art

Despite technological advancements in the diagnosis and treatment options available to patients diagnosed with cancer, the prognosis still often remains poor and many patients cannot be cured. Immunotherapy holds the promise of offering a potent, yet targeted, treatment to patients diagnosed with various tumors with the potential to eradicate the malignant tumor cells without damaging normal tissues. In theory, the T cells of the immune system are capable of recognizing protein patterns specific for tumor cells and to mediate their destruction through a variety of effector mechanisms. The administration of immune cells expressing chimeric antigen receptors (CARs), such as adoptive T cell therapy or NK cell therapy, is an attempt to harness and amplify the tumor-eradicating capacity of a patient's own immune cells and then return these effectors to the patient in such a state that they effectively eliminate residual tumor, however without damaging healthy tissue. Commonly, CARs comprise a single chain variable fragment (scFv) of an antibody specific for a tumor associated antigen (TAA) coupled via hinge and transmembrane regions to cytoplasmic domains of T-cell signaling molecules. However, many drawbacks in the clinical use of immune cells therapy impair the full use of this approach in cancer treatments. Thus, there is an unmet need for improved CARs for use in immune cell therapy.

### SUMMARY

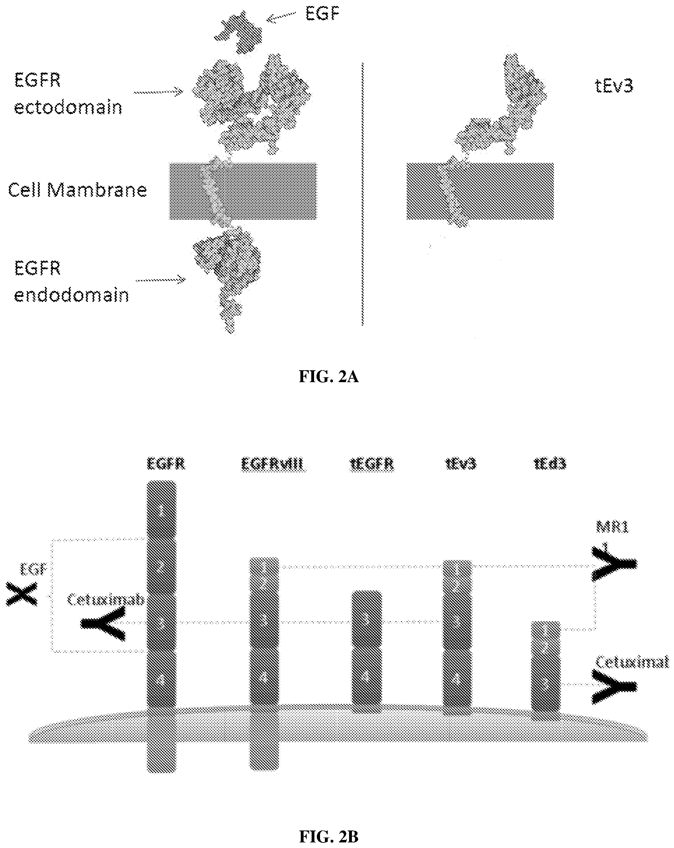

In certain embodiments, the present disclosure provides enhanced chimeric antigen receptors and methods of their use for the treatment of diseases and disorders, including cancer and autoimmune disorders. In one embodiment, there is provided a truncated EGFRvIII, referred to as Ev3 (or tEv3), which does not comprise an endodomain or functional EGF binding domain. In particular aspects, Ev3 comprises truncated domain 1 (L1), truncated domain 2 (CR1), domain 3 (L2), and domain 4 (CR2) of EGFR (as depicted in FIG. 2B). In some aspects, the Ev3 may be located in the hinge region of a CAR and can be used for CAR detection and/or ablation of CAR-expressing cells. In some embodiments, the CAR comprises both a humanized scFv and an Ev3 hinge. In another embodiment, there are provided humanized antigen-binding domains for the CARs, such as humanized single chain variable fragments (scFvs). Further embodiments provide methods of treating immune-associated disorders by administering immune cells, such as T cells or NK cells, which express the CAR(s) of the embodiments to a subject.

In another embodiment, there is provided a chimeric antigen receptor (CAR) comprising a Ev3 in the hinge of said CAR, wherein said Ev3 hinge links an extracellular domain and at least one intracellular immune signaling domain. In some aspects, the Ev3 hinge comprises a transmembrane domain and a non-functional ectodomain of EGFRvIII. In particular aspects, the Ev3 hinge does not comprise EGFRvIII endodomain. In some aspects, the non-functional ectodomain of EGFRvIII has essentially no binding capability to epidermal growth factor (EGF).

In some aspects, the Ev3 hinge comprises an amino acid sequence with at least 80%, such as at least 81, 82, 83, 84, 85, 86, 87, 88, 89, 90, 91, 92, 93, 94, 95, 96, 97, 98, or 99% sequence identity to SEQ ID NO:2. In some aspects, the Ev3 hinge comprises an amino acid sequence of SEQ ID NO:2.

The Ev3 may comprise or have at least 80% *(e.g.,* 81%, 82%, 83%, 84%, 85%, 86%, 87%, 88%, 89%, 90%, 91%, 92%, 93%, 94%, 95%, 96%, 97%, 98%, 99%, or 100%) sequence identity to SEQ ID NO:2 LEEKK-GNYVVTDHGSCVRACGADSYEMEEDGVRKCKKCEGPCRK-VCNGIGIGEFKDSLSINATNIKHFKNCTSISGDLHILPVAFRGDSFTHTPPLDPQELDIL KTVKEITGFLLIQAWPENRTDLHAFENLEIIRGRTKQHGQFSLAVVSLNITSLGLRSLK EISDGDVIISGNKNLCYANTINWKKLFGTSGQKTKIISNRGENSCKATGQ-VCHALCSPEGCWGPEPRDCVSCRNVSRGRECVDKCNLLEGEPREFVENSECIQCHPE CLPQAMNITCTGRGPDNCIQCAHYIDGPHCVKTCPAGVMGENNTLVWKYADAGHV CHLCHPNCTYGCTGPGLEGCPTNGPKIPS (comprising partial Domain 1-partial Domain 2-Domain 3-Domain 4). In certain aspects, the Ev3 may have one, two, three, or more modifications.

In some aspects, the Ev3 hinge is encoded by a nucleotide sequence with at least 80%, such as at least 81, 82, 83, 84, 85, 86, 87, 88, 89, 90, 91, 92, 93, 94, 95, 96, 97, 98, or 99% sequence identity to SEQ ID NO:1. In certain aspects, the Ev3 hinge is encoded by a nucleotide sequence of SEQ ID NO:1.

In certain aspects, the extracellular domain of the CAR comprises an antigen-binding domain selected from the group consisting of F(ab')2, Fab', Fab, Fv, and scFv. In some aspects, the antigen-binding domain comprises a scFv. In specific aspects, the scFv is further defined as a humanized scFv. In some aspects, the humanized scFv is a scFv according to the embodiments.

In some aspects, the antigen binding region of the CAR binds one or more tumor associated antigens. In certain aspects, the one or more tumor associated antigens are selected from the group consisting of CD19, CD319 (CS1), ROR1, CD20, carcinoembryonic antigen, alphafetoprotein, CA-125, MUC-1, epithelial tumor antigen, melanoma-associated antigen, mutated p53, mutated ras, HER2/Neu, ERBB2, folate binding protein, HIV-1 envelope glycoprotein gp120, HIV-1 envelope glycoprotein gp41, GD2, CD5, CD123, CD23, CD30, CD56, c-Met, mesothelin, GD3, HERV-K, IL-11Ralpha, kappa chain, lambda chain, CSPG4, ERBB2, WT-1, TRAIL/DR4, VEGFR2, CD33, CLL-1 and CD99. In particular aspects, the one or more tumor-associated antigens are CD19, CD319, CD123, CD5, ROR1, mesothelin, CD33, CLL-1, and/or CD99. In some aspects, the scFv does not comprise an EGFR binding domain.

In certain aspects, the at least one signaling domain comprises CD3ξ, CD28, OX40/CD134, 4-1BB/CD137, FcεRIγ, ICOS/CD278, ILRB/CD122, IL-2RG/CD132, DAP12, CD70, CD40, or a combination thereof. In particular aspects, the at least one signaling domain comprises DAP12. In some aspects, the CAR comprises IL-15. In one specific aspects, the CAR comprises CD3ζ, CD28, DAP12, and IL-15.

In some aspects, the CAR further comprises a suicide gene. In particular aspects, the suicide gene is inducible caspase 9.

In one embodiment, there are provided isolated antigen-specific humanized single chain variable fragments (scFvs) comprising a light chain variable region (V_{L}) and a heavy chain variable region (V_{H}), wherein the scFv is:
(a) CD19-specific and wherein the V_{L} comprises an amino acid sequence of SEQ ID NO:7 and the V_{H} comprises an amino acid sequence of SEQ ID NO:8;
(b) CD123-specific and wherein the V_{L} comprises an amino acid sequence of SEQ ID NO:13 and the V_{H} comprises an amino acid sequence of SEQ ID NO:14;
(c) mesothelin-specific and wherein the V_{L} comprises an amino acid sequence of SEQ ID NO:19 and the V_{H} comprises an amino acid sequence of SEQ ID NO:20;
(d) ROR1-specific and wherein the V_{L} comprises an amino acid sequence of SEQ ID NO:25 and the V_{H} comprises an amino acid sequence of SEQ ID NO:26;
(e) CD5-specific and wherein the V_{L} comprises an amino acid sequence of SEQ ID NO:31 and the V_{H} comprises an amino acid sequence of SEQ ID NO:32;
(f) CLL-1-specific and wherein the V_{L} comprises an amino acid sequence of SEQ ID NO:59 and the V_{H} comprises an amino acid sequence of SEQ ID NO:60;
(g) CD99-specific and wherein the V_{L} comprises an amino acid sequence of SEQ ID NO:63 and the V_{H} comprises an amino acid sequence of SEQ ID NO:64 or
(h) a sequence with 90% sequence identity to framework regions of any one of (a)-(g).

In some aspects, the scFv sequences comprise 91, 92, 93, 94, 95, 96, 97, 98, or 99% sequence identity to the framework regions of SEQ ID NOs:6, 12, 18, 24, or 30 and 100% sequence identity to the CDRs of said sequences. In certain aspects, the CDRs of said sequences may have one, two, or three modifications.

In some aspects, the scFv is CD19-specific and wherein the V_{L} comprises an amino acid sequence of SEQ ID NO:7 and the V_{H} comprises an amino acid sequence of SEQ ID NO:8. In one specific aspect, the CD19-specific scFv comprises an amino acid sequence of SEQ ID NO:6. In certain aspects, the scFv is CD123-specific and wherein the V_{L} comprises an amino acid sequence of SEQ ID NO:13 and the V_{H} comprises an amino acid sequence of SEQ ID NO:14. In particular aspects, the CD123-specific scFv comprises an amino acid sequence of SEQ ID NO:12. In some aspects, the scFv is mesothelin-specific and wherein the V_{L} comprises an amino acid sequence of SEQ ID NO:19 and the V_{H} comprises an amino acid sequence of SEQ ID NO:20. In specific aspects, the mesothelin-specific scFv comprises an amino acid sequence of SEQ ID NO:18. In some aspects, the scFv is ROR1-specific and wherein the V_{L} comprises an amino acid sequence of SEQ ID NO:25 and the V_{H} comprises an amino acid sequence of SEQ ID NO:26. In specific aspects, the ROR1-specific comprises an amino acid sequence of SEQ ID NO:24. In some asepcts, the scFv is CD5-specific and wherein the V_{L} comprises an amino acid sequence of SEQ ID NO:31 and the V_{H} comprises an amino acid sequence of SEQ ID NO:32. In certain aspects, the CD5-specific comprises an amino acid sequence of SEQ ID NO:30.

In some aspects, the scFv comprises an amino acid sequence with at least 95%, such as at least 96, 97, 98, or 99% sequence identity to framework regions an amino acid sequence of SEQ ID NO:6, 12, 18, 24, or 30.

Further provided herein are isolated polynucleotides encoding the isolated scFvs of the above embodiments. In some aspects, the polynucleotide comprises SEQ ID NO:3, 9, 15, 21, or 27. In other aspects, the polynucleotide comprises a sequence with at least 90, 91, 92, 93, 94, 95, 96, 97, 98, or 99% sequence identity to SEQ ID NO:3, 9, 15, 21, or 27.

Also provided herein are expression vectors comprising isolated nucleotides of the embodiments. In some aspects, the vector is further defined as a viral vector.

Further provided herein are isolated nucleic acids comprising a nucleotide sequence encoding the CAR according to any one of the present embodiments.

Also provided herein are host cells engineered to express a CAR comprising a humanized scFv of the present embodiments and/or a truncated EGFRvIII (Ev3) in the hinge region of said CAR. In some aspects, the CAR is according to any one of the present embodiments. In some aspects, DNA encoding the CAR is integrated into the genome of the cell.

In some aspects, the host cell is further defined as a CAR immune cell. In particular aspects, the immune cell is a T cell, peripheral blood lymphocyte, NK cell, invariant NK cell, NKT cell, or stem cell. In specific aspects, the immune cell is a T cell or a NK cell. In some aspects, the stem cell is a mesenchymal stem cell (MSC) or an induced pluripotent stem (iPS) cell. In certain aspects, the immune cell is derived from an iPS cell. In some aspects, the T cell is a CD8⁺ T cell, CD4⁺ T cell, or gamma-delta T cell. In certain aspects, the T cell is a cytotoxic T lymphocyte (CTL). In some aspects, the immune cell is allogeneic or autologous. In some aspects, the immune cell is isolated from peripheral blood, cord blood, or bone marrow. In certain aspects, the immune cell is isolated from cord blood. In particular aspects, the cord blood is pooled from 2 or more individual cord blood units.

Further provided herein are pharmaceutical compositions comprising a population of immune cells according to any of the present embodiments. Also provided herein are compositions comprising a population of immune cells of any one of present embodiments for use in the treatment of an immune-related disorder.

In another embodiment, there is provided a method of treating an immune-related disorder in a subject comprising administering an effective amount of CAR immune cells of any one of the present embodiments, such as comprising a humanized scFv and/or a Ev3 hinge.

In some aspects of the above embodiments, the immune-related disorder is a cancer, autoimmune disorder, graft versus host disease, allograft rejection, or inflammatory condition. In certain aspects, the immune-related disorder is an inflammatory condition and the immune cells have essentially no expression of glucocorticoid receptor. In particular aspects, the immune-related disorder is a cancer. In some aspects, the immune cells are autologous or allogeneic. In some aspects, the cancer is a solid cancer or a hematologic malignancy.

In additional aspects, the method further comprises administering at least a second therapeutic agent. In some aspects, the at least a second therapeutic agent comprises chemotherapy, immunotherapy, surgery, radiotherapy, or biotherapy. In certain aspects, the immune cells and/or the at least a second therapeutic agent are administered intravenously, intraperitoneally, intratracheally, intratumorally, intramuscularly, endoscopically, intralesionally, percutaneously, subcutaneously, regionally, or by direct injection or perfusion.

In some aspects, the method further comprises administering an anti-EGFR antibody. In some aspects, the anti-EGFR antibody is a monoclonal antibody. In certain aspects, the anti-EGFR antibody is cetuximab or panitumumab. In some aspects, the anti-EGFR antibody selectively ablates Ev3-CAR immune cells through antibody-dependent cell-mediated cytotoxicity (ADCC). In certain aspects, the anti-EGFR antibody is fused to a detectable tag and/or a cytotoxic agent. In some aspects, the method further comprises imaging the detectable tag, thereby detecting the Ev3-CAR immune cells. In certain aspects, the anti-EGFR antibody is fused to a cytotoxic agent. In some aspects, the cytotoxic agent induces activation of the suicide gene in the Ev3-CAR immune cells. In some aspects, the cytotoxic agent results in the ablation of the Ev3-CAR immune cells.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

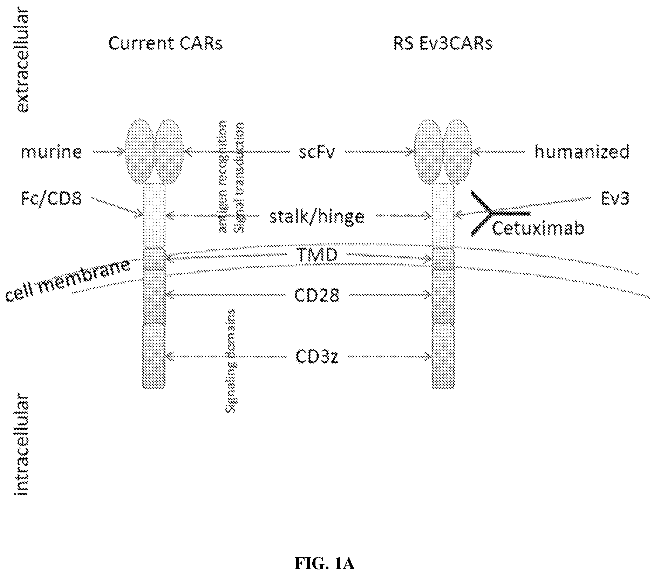

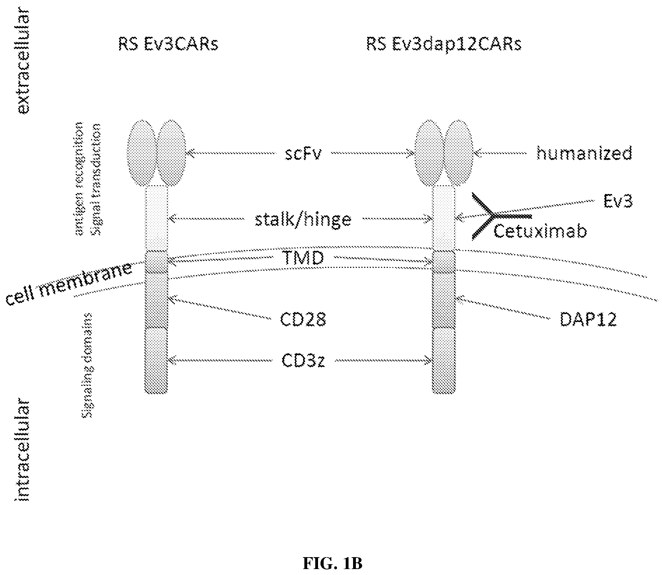

**FIGS. 1A-1B****:** Comparisons between current CARs and Ev3-CAR designs. **(A)** The Ev3 stalk serves as an integrative structure to transduce signals from antigen binding, such as by scFv, as well as an "off" switch, such that it can be recognized by clinically accessible antibodies such as cetuximab to ablate CAR-positive immune cells. **(B)** As an enhancement over CARs, such as T cell use, DAP12 may be utilized as the signal booster in Ev3-DAP12 CARs to increase the efficacies of CAR functions in NK cells.
**FIGS. 2A-2C****: (A)** Schematic depicting Ev3 as compared to EGFR which comprise the endodomain and the EGF binding domain. **(B)** Schematic depicting EGFR variants and fragmets. (C) Sequence of Ev3 comprising only extracellular domains.

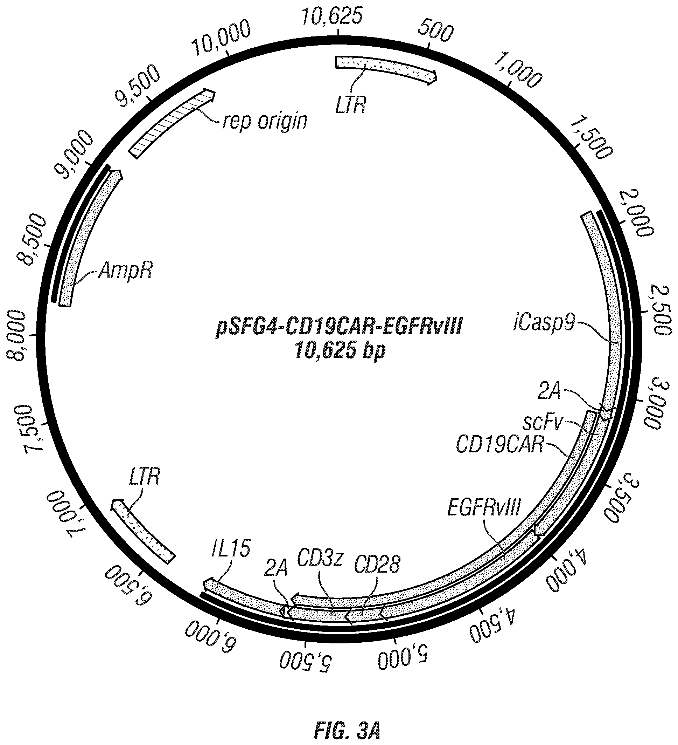

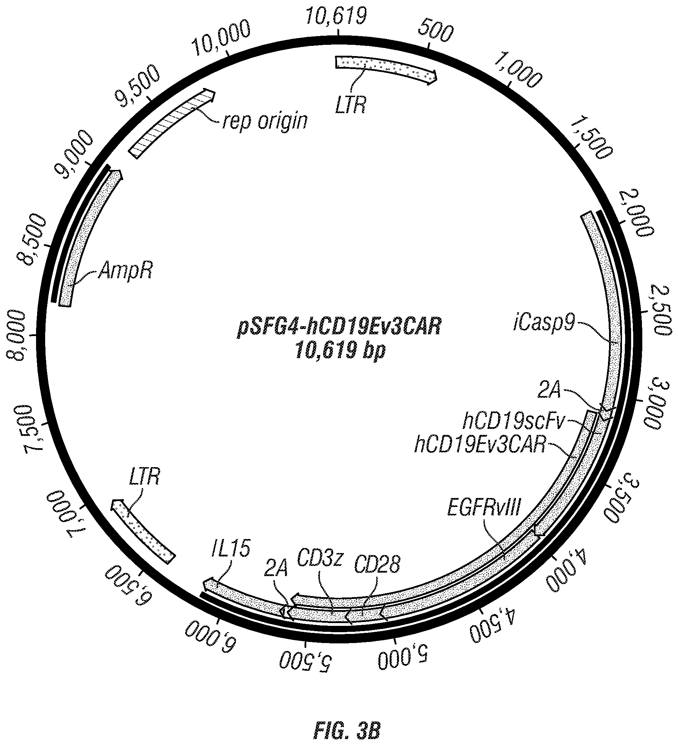

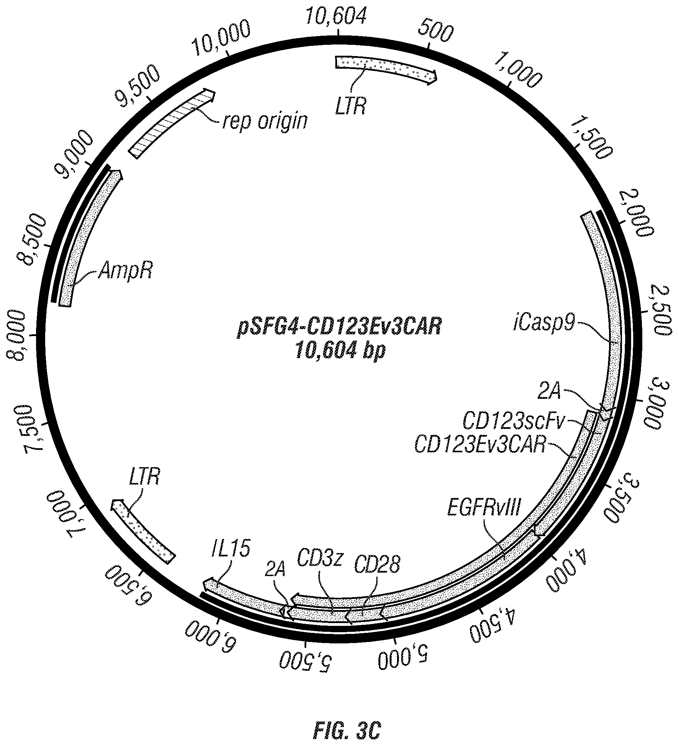

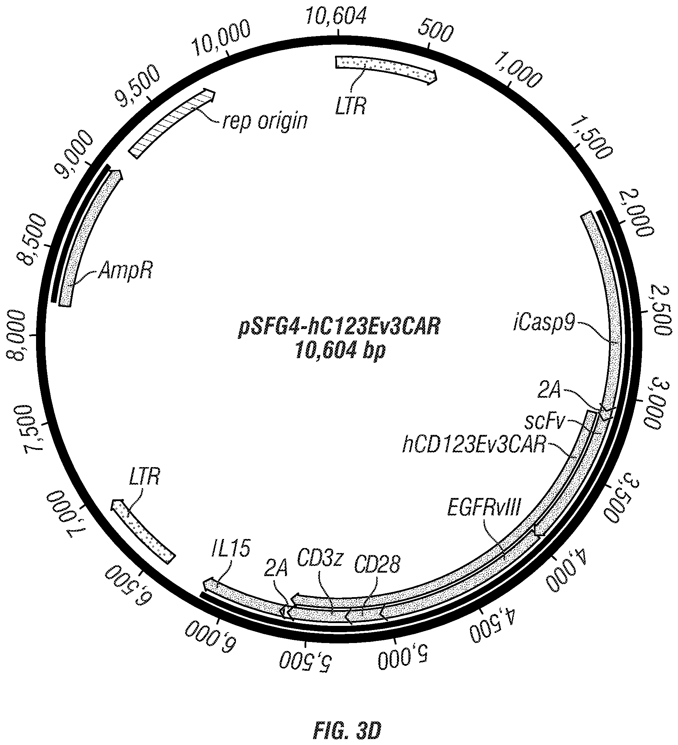

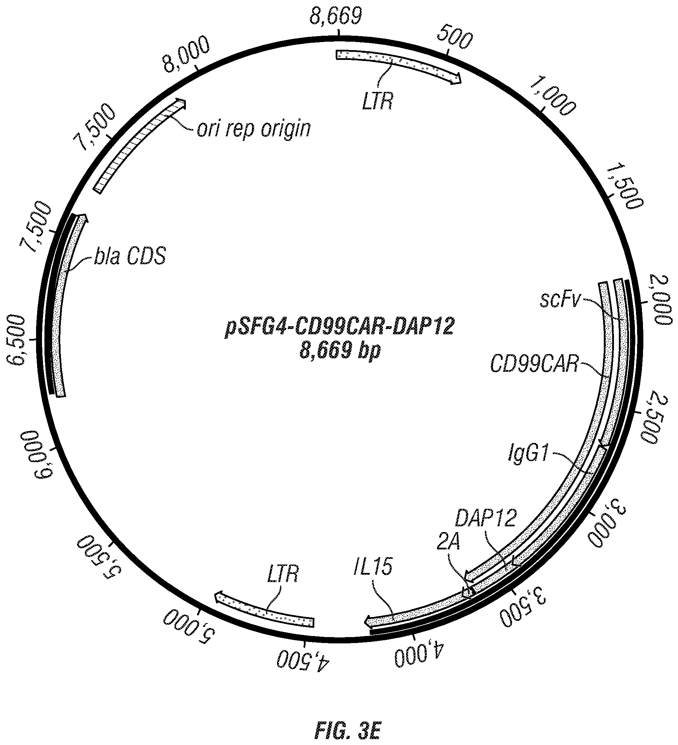

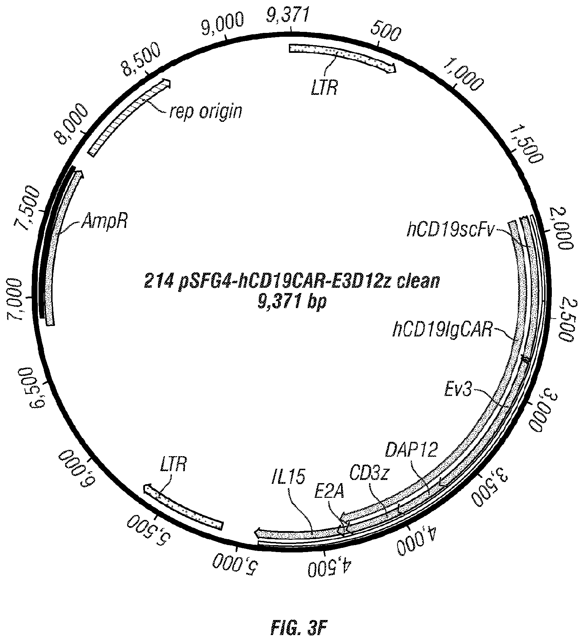

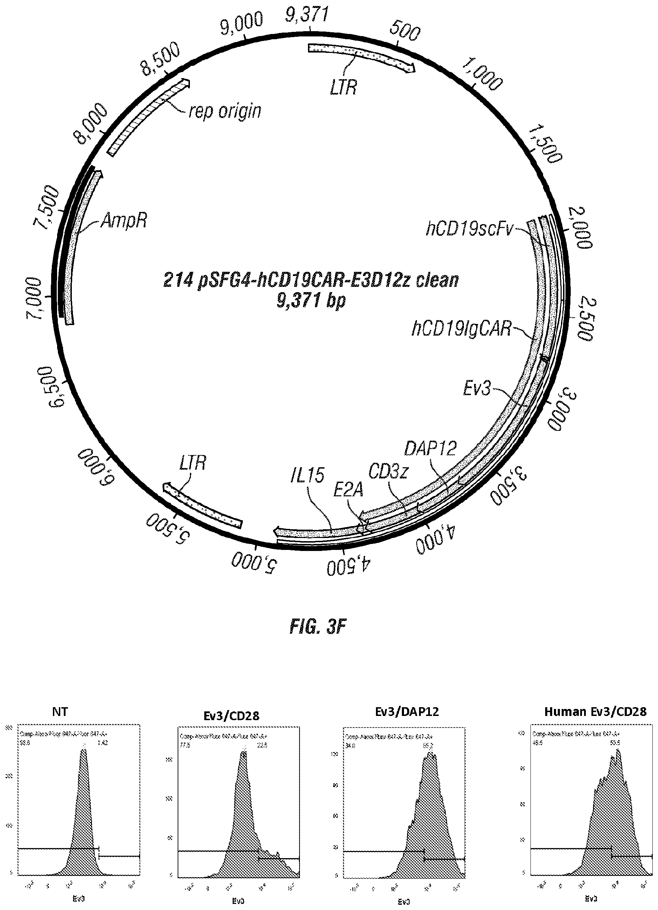

**FIGS. 3A-3F****:** Diagrams for retroviral vectors for the antigens CD19 **(A),** hCD19 **(B),** CD123 **(C),** hCD123 **(D),** and CD99 **(E). (F)** Construct map for CAR 2.0 using humanized scFv against CD19 which can be replaced with other scFvs.

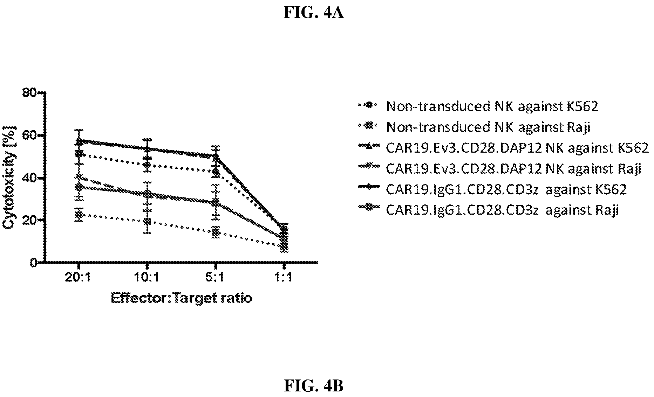

**FIGS. 4A-4B****: (A)** Ev3 antibody recognizes CAR19.Ev3.CD28.CD3ζ, CAR19.Ev3.DAP12.CD3ζ or humanized CAR19.Ev3.CD3ζ transduced NK cells. **(B)** In chromium release assays to assess the killing activity of CAR.19 transduced NK cells against CD19⁺ Raji tumor targets, it was shown that CAR19.Ev3.CD28.DAP12 NK cells are as effective as CAR19.IgG1.CD28.CD3z transduced NK cells in killing CD19⁺ Raji cells at multiple effector:target ratios.

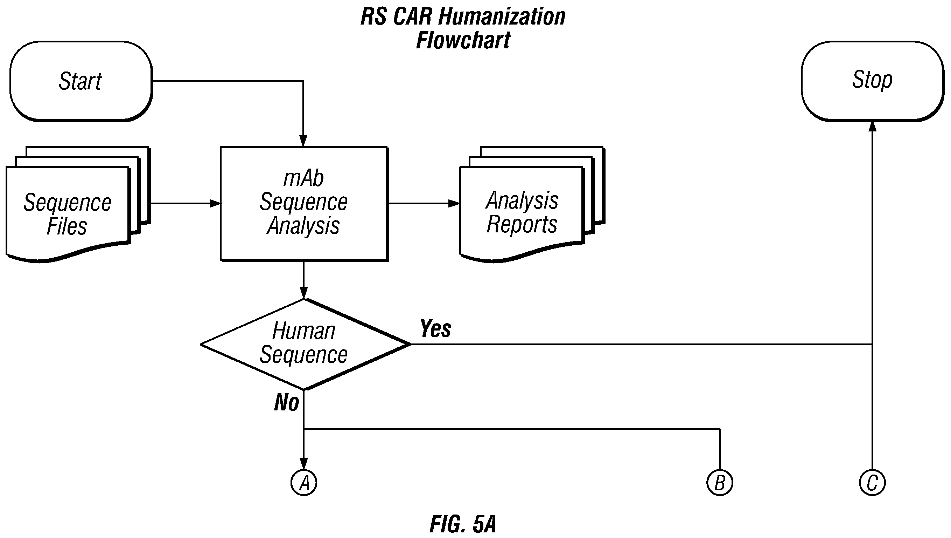

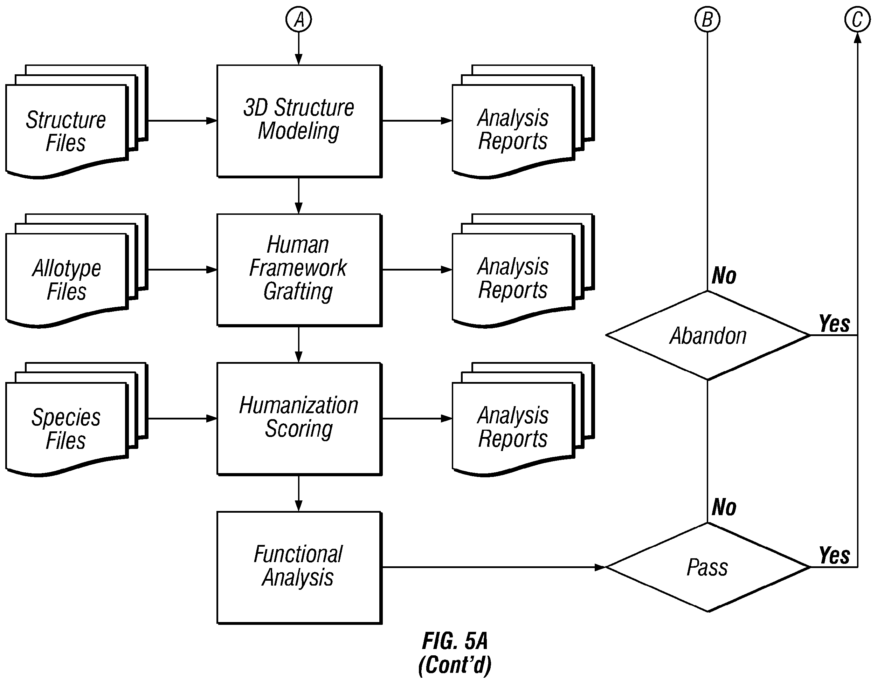

**FIGS. 5A-5B****: (A)** Schematic depicting platform for humanization of CARs. **(B)** Monoclonal antibody sequence analysis.

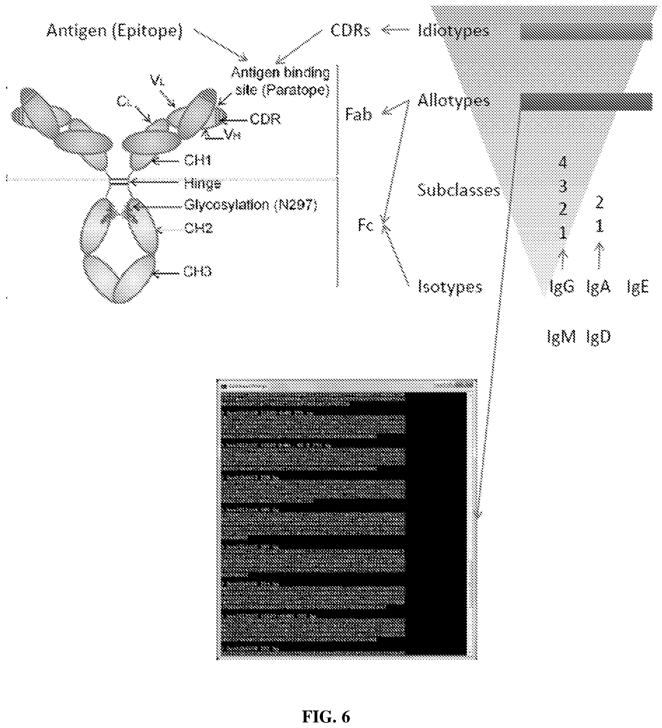

**FIG. 6****:** Schematic depicting isotypes, allotypes, and idiotypes of antibodies. Structural determinants of antibodies are pictured to define the elements isolated and used for the analysis and subsequent CDR/framework graftings. 5 major isotypes for human immunoglobulins are further subclassified as indicated (IgG1, 2, 3, 4, IgA1, 2).

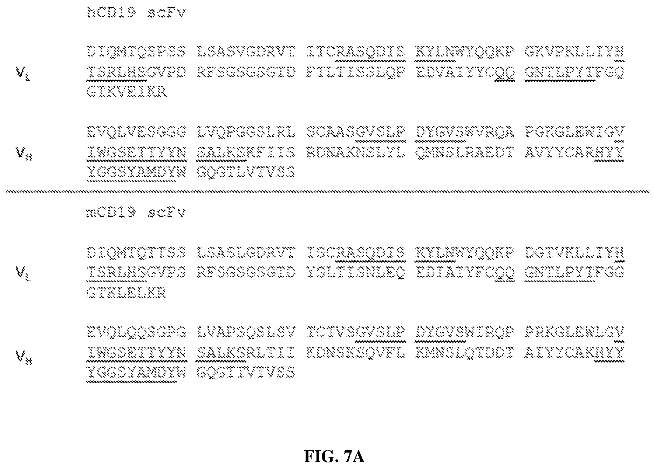

**FIGS. 7A-7C****: (A)** Amino acid sequences for hCD19 scFv and mCD19 scFv including V_{L} and V_{H}. The CDR regions are underlined. **(B)** BLAST analysis of hCD19 scFv and mCD19 scFv to verify their respective species origins by sequence identities or homologies. **(C)** Summary of analysis comparing mCD19 scFv vs humanized CD19 scFv.

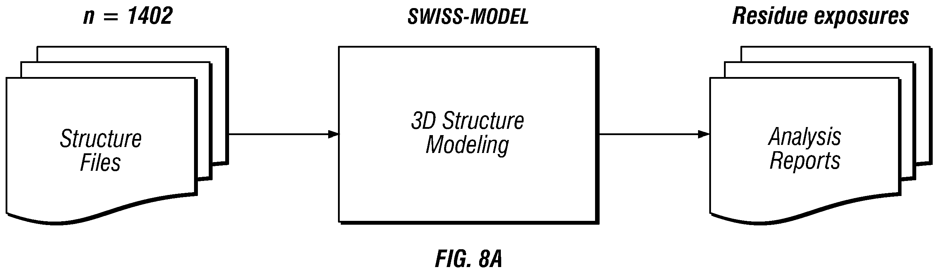

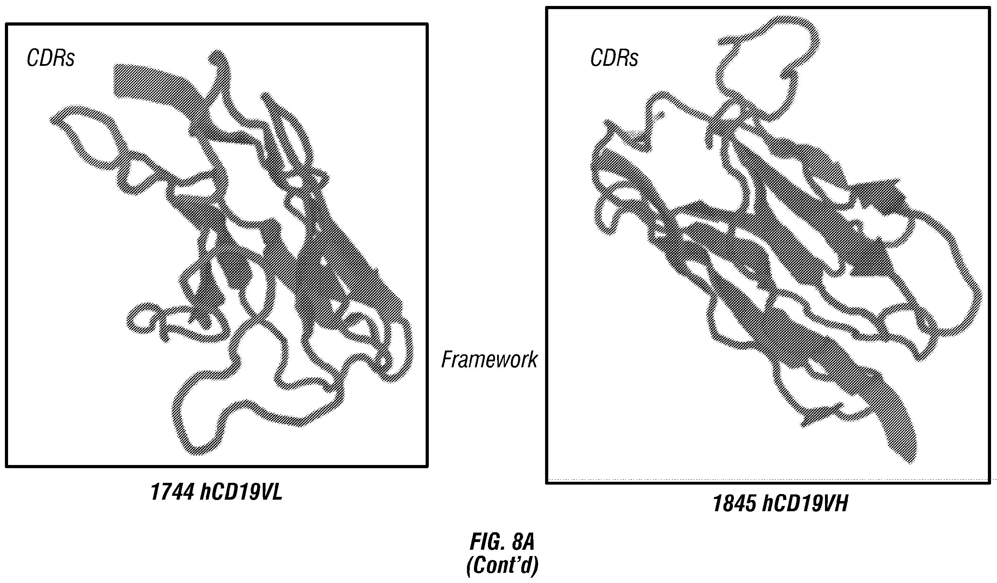

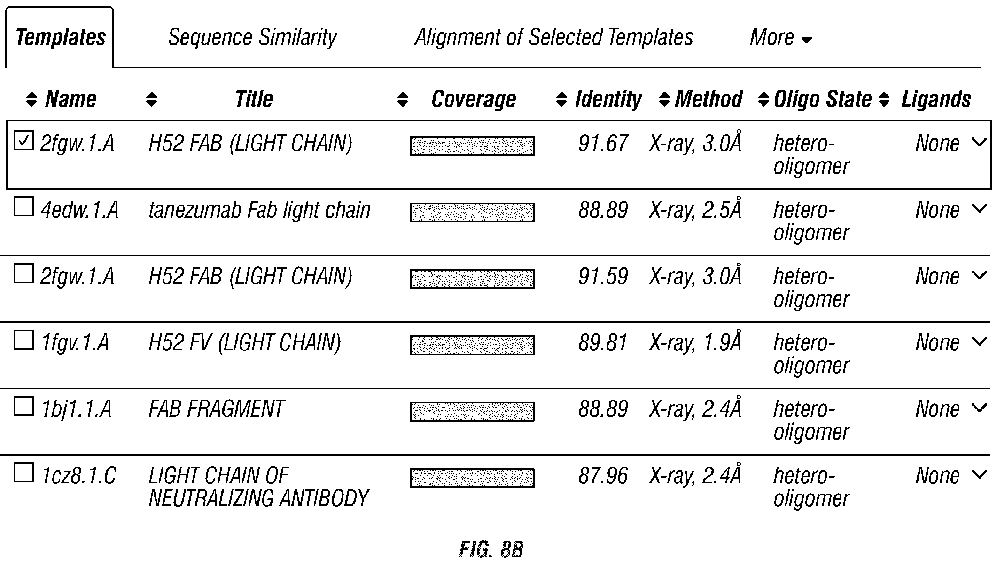

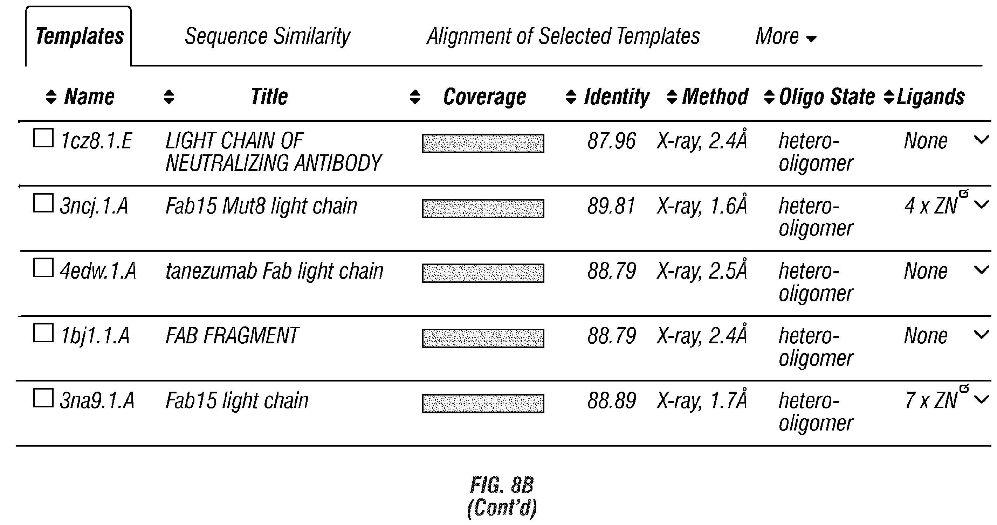

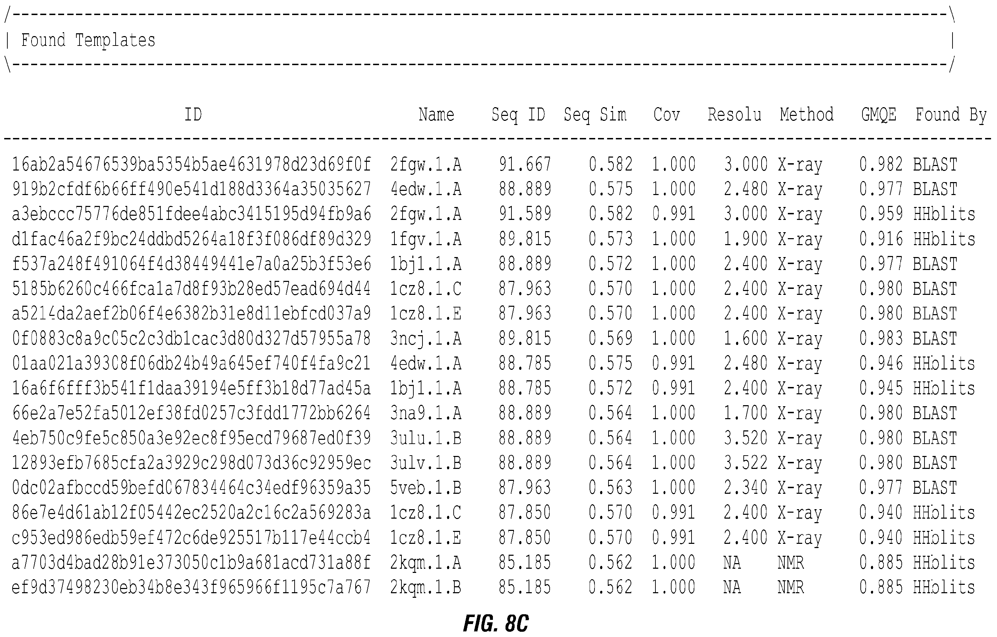

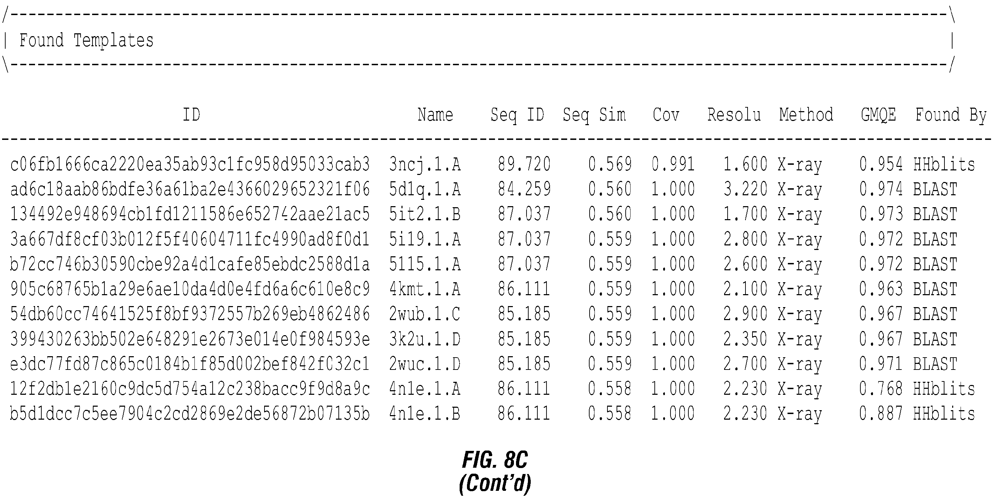

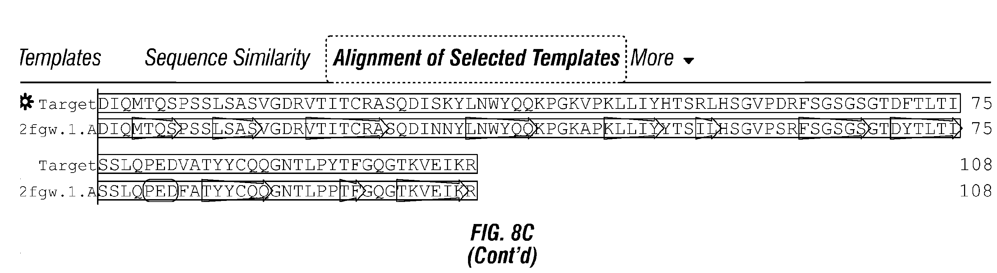

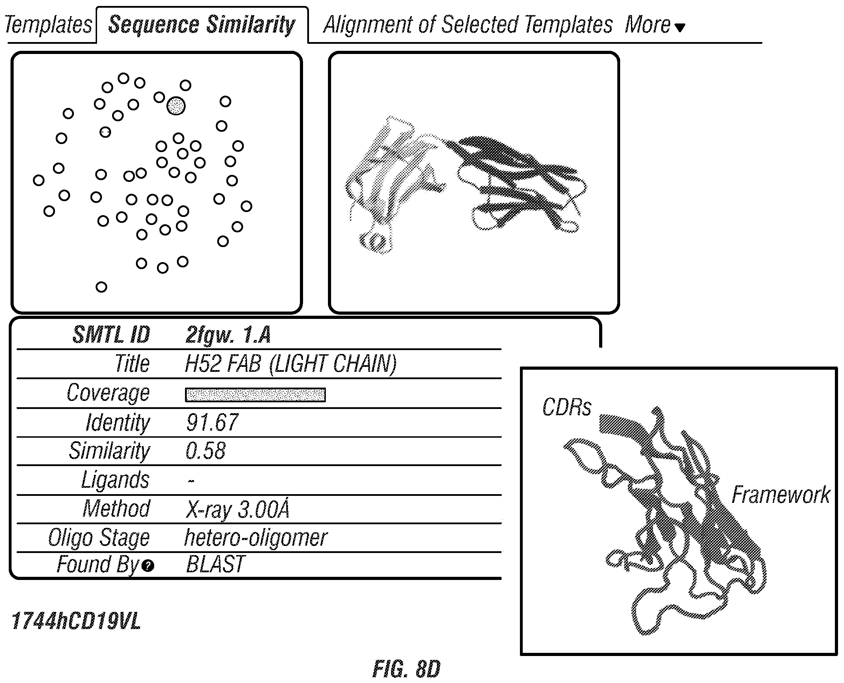

**FIGS. 8A-8D****: (A)** Schematic depicting 3D structure modeling. **(B-D)** 3D Structure modeling of human CD19 variable light chain. Using SWISS-MODEL*, a fully automated protein structure homology-modeler, 3-dimensional models of humanized V_{L} and V_{H} domains were generated (anti-CD19 as specific example shown). Structure files were extracted from SAbDab, a structural antibody database, as templates for homology modeling, as well as using PDB structure files which are automatically accessed within SWISS-MODEL.

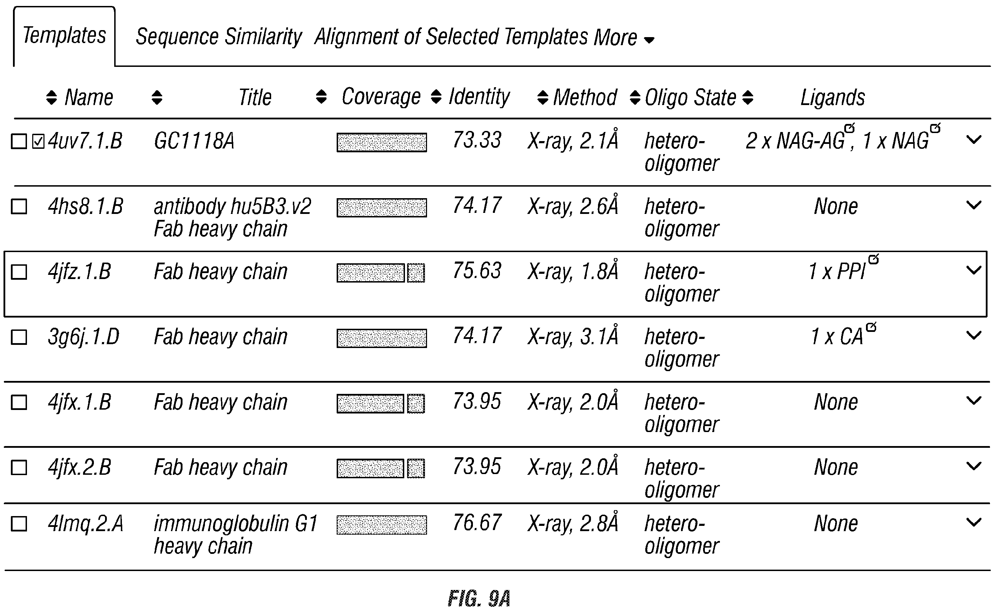

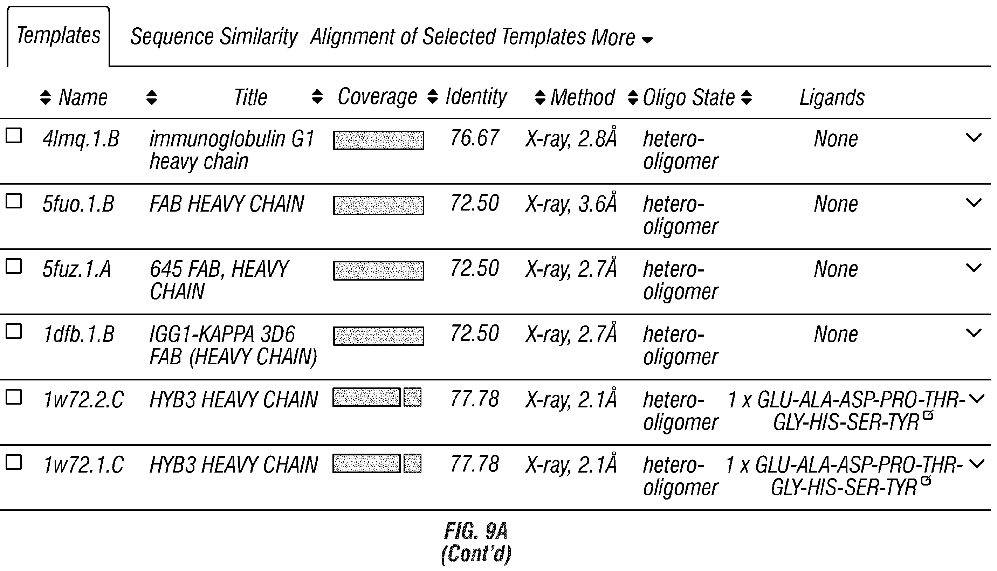

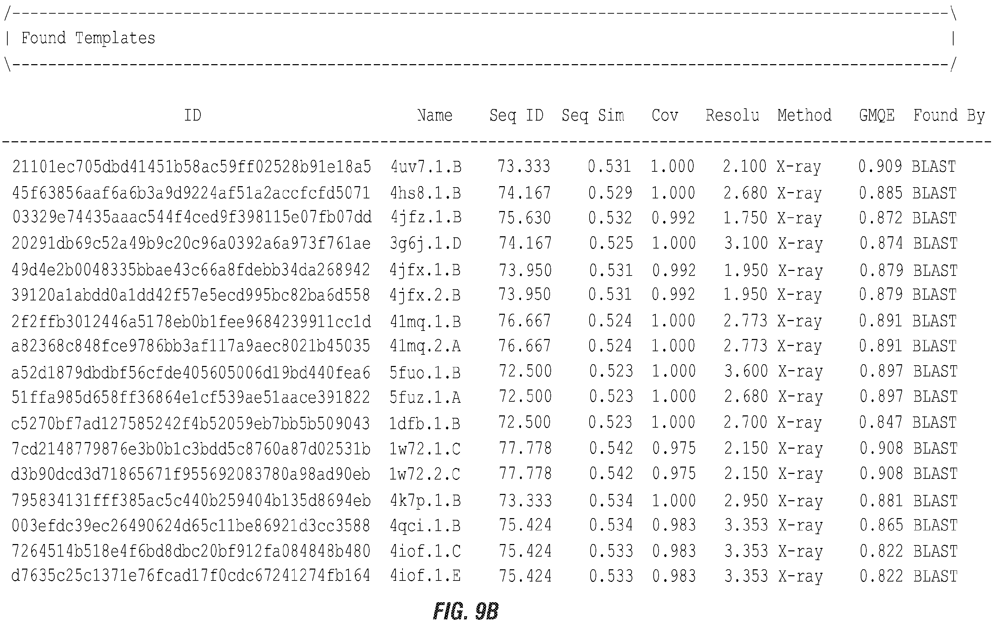

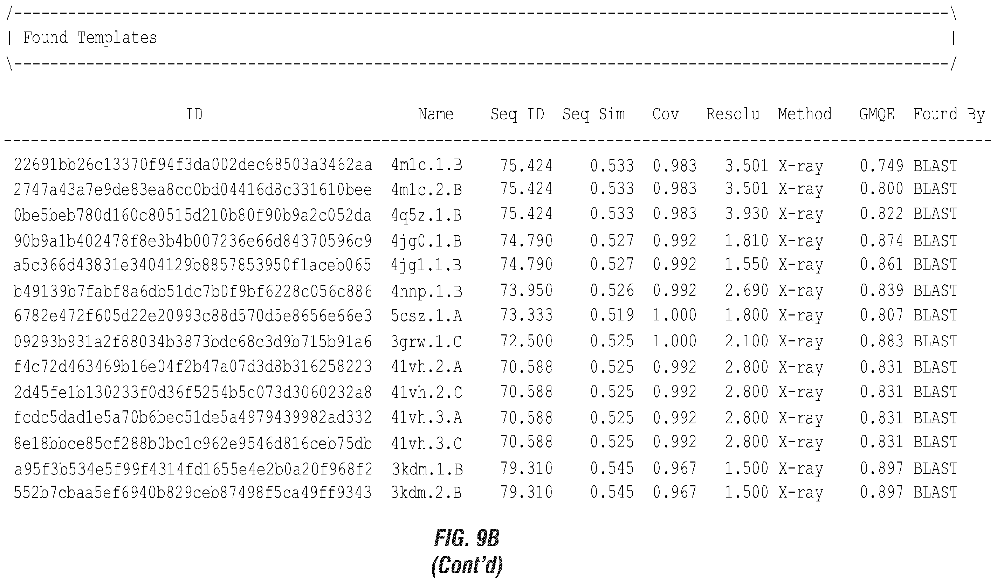

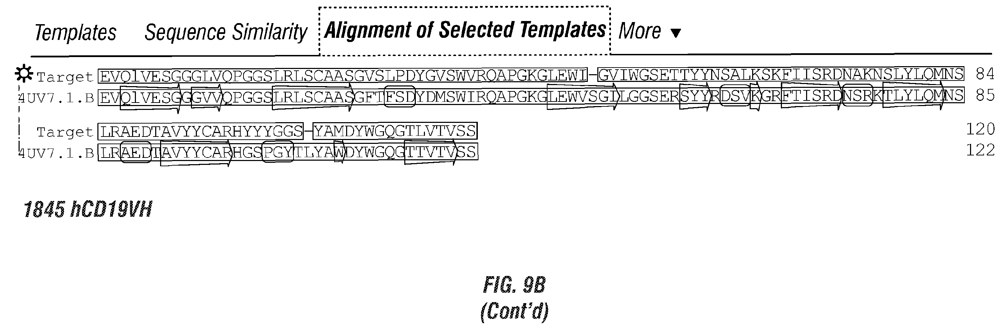

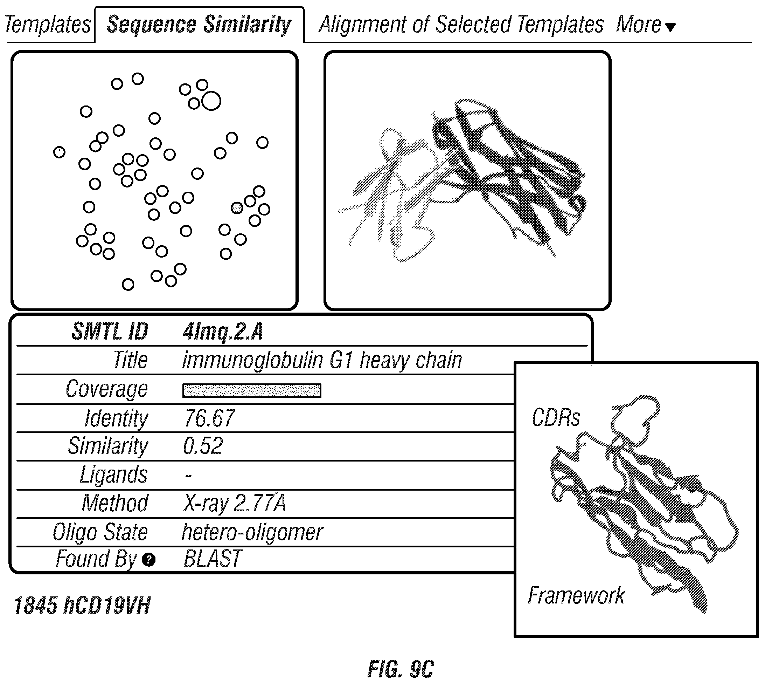

**FIGS. 9A-9C****:** 3D structure modeling of human CD19 variable heavy chain. For the specific example of hCD19V_{L} domain, **(A)** a list was generated in SWISS-MODEL for the best matching (structurally homologous) structure with their scores (GMQE) and matching methods (BLAST or HHblits). **(B-C)** The best scoring structure (2fgw) was then aligned with the hCD19V_{L} domain ("target") as shown in the bottom panel.

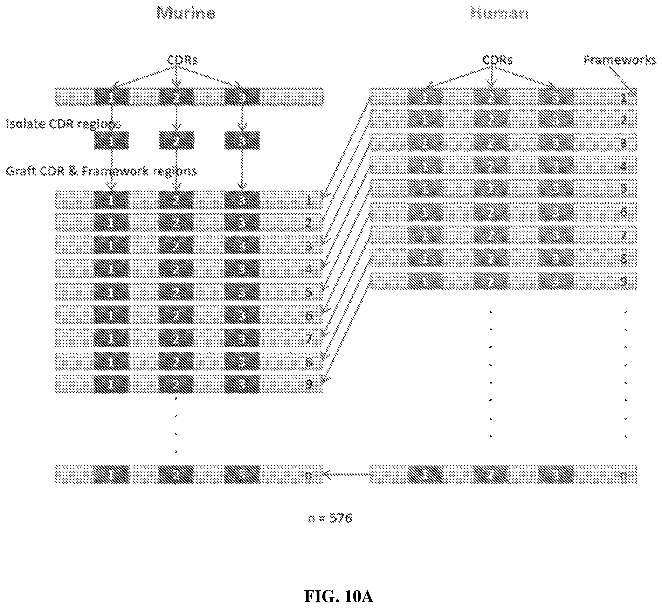

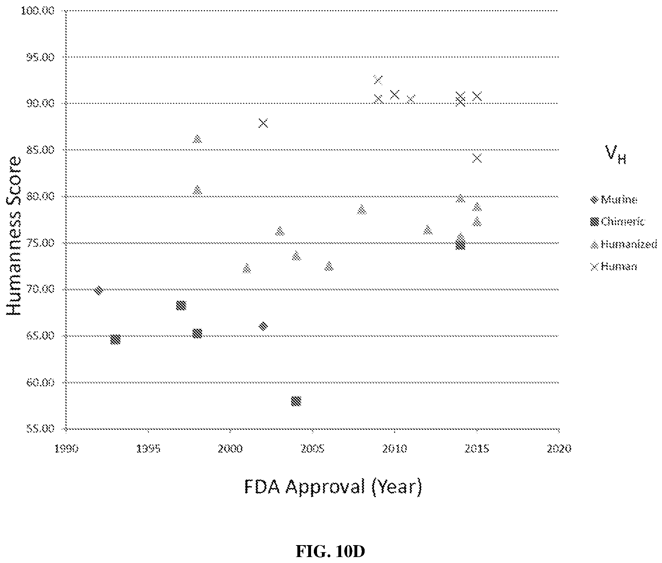

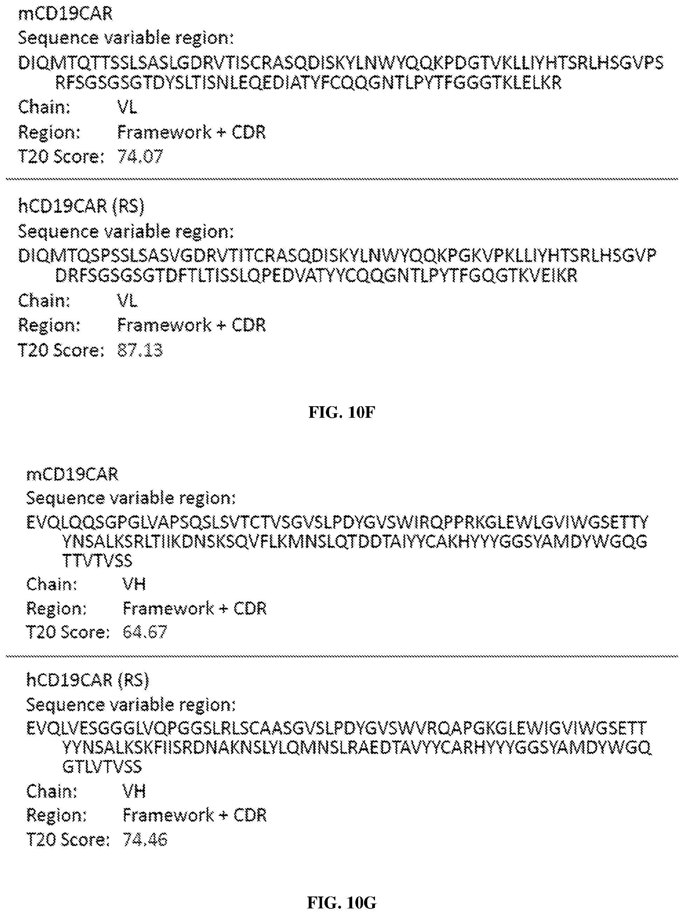

**FIGS. 10A-10G****: (A)** Schematic depicting human framework grafting showing identification and isolation of the CDR regions from mouse antibodies (left top), distillation of human framework regions (right panel), and human framework/murine CDR grafting (left bottom). The CDRs and framework regions are gleaned from sequence analysis as described above. **(B)** Baseline humanization scoring of monoclonal antibody using T20 scoring system (Gao *et al.,* 2013) to enumerate the 'humanness' of particular Ig domains. As controls, V_{L} and V_{H} domains were extracted from antibodies which are currently in the market place for scoring and comparisons. The humanness scores for the indicated antibodies (right most columns) are listed for V_{L} and V_{H} domains. **(C)** Early therapeutic antibodies approved for the commercial marketpace are either murine or chimeric antibodies (variable mouse regions + human constant regions), while later approvals tend to be humanized or human antibodies. The humanness scores tend to cluster for antibodies of the same type (murine, chimeric, humanized, or human), with increasing humanness scores correlative to human contents. **(D)** Humanization scoring of variable heavy chains by year of FDA approval of antibody. The humanness scores tend to cluster for antibodies of the same type (murine, chimeric, humanized, or human), with increasing humanness scores correlative to human contents. Due to the larger sizes of the V_{H} domains, the humanness scores tend to be lower (compared to V_{L}). **(E)** Humanization scoring for indicated antibodies. The frequency of anti-therapeutic antibody responses is shown as % and the size of the patient group evaluated is given in brackets. In general, the more human or humanized an antibody, the less immunogenicity is reported in clinical use. **(F)** Humanization scoring of variable light chain of hCD19-CAR (SEQ ID NO:1) and mCD19-CAR (SEQ ID NO:3). **(G)** Humanization scoring of variable heavy chain of hCD19-CAR (SEQ ID NO:2) and mCD19-CAR (SEQ ID NO:4)

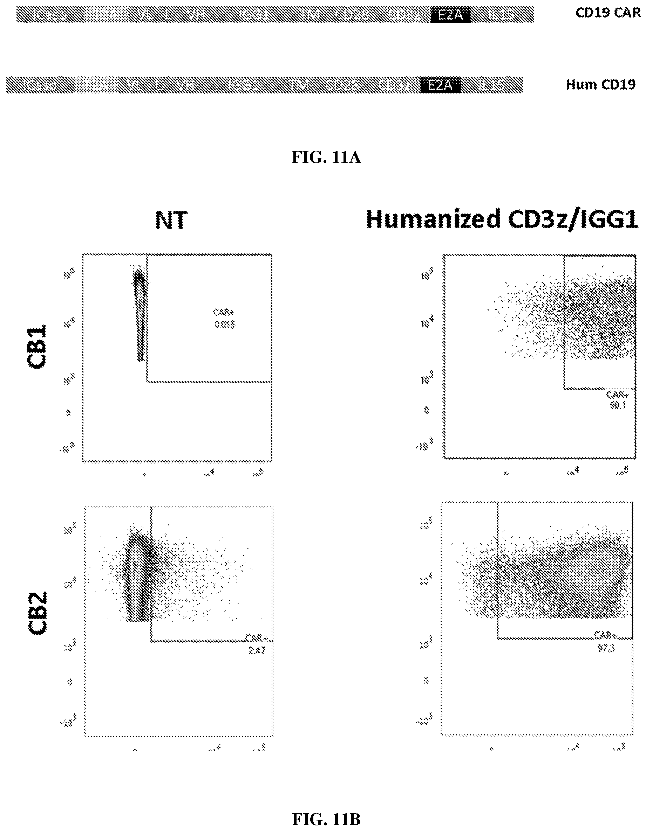

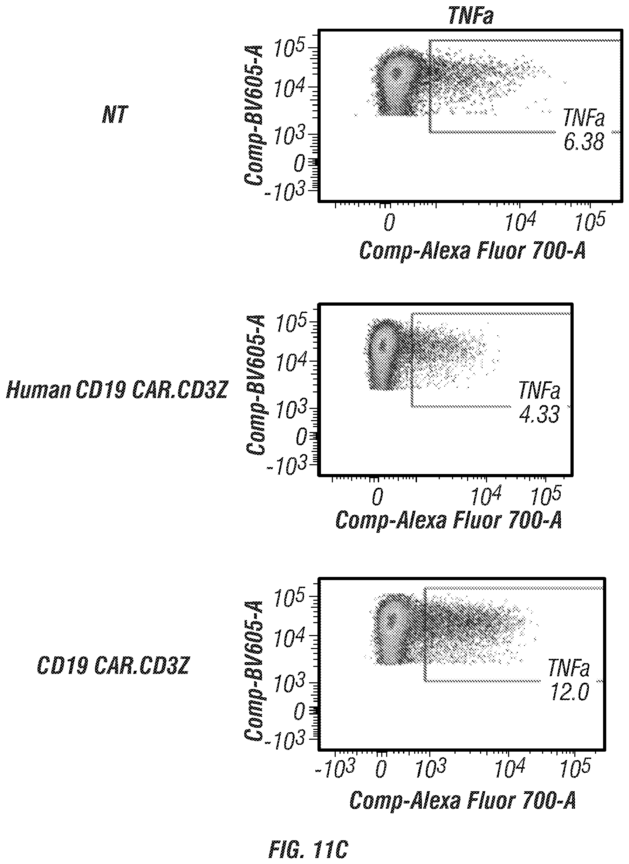

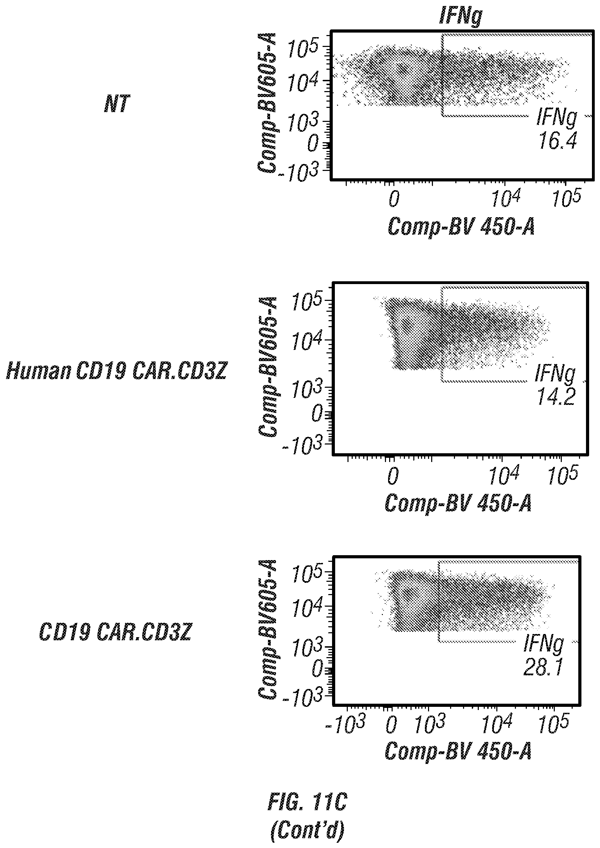

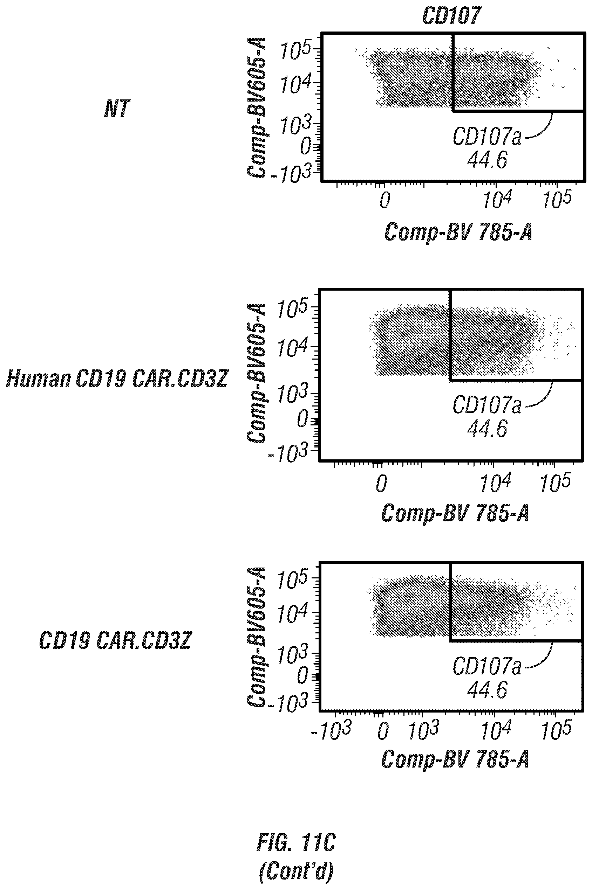

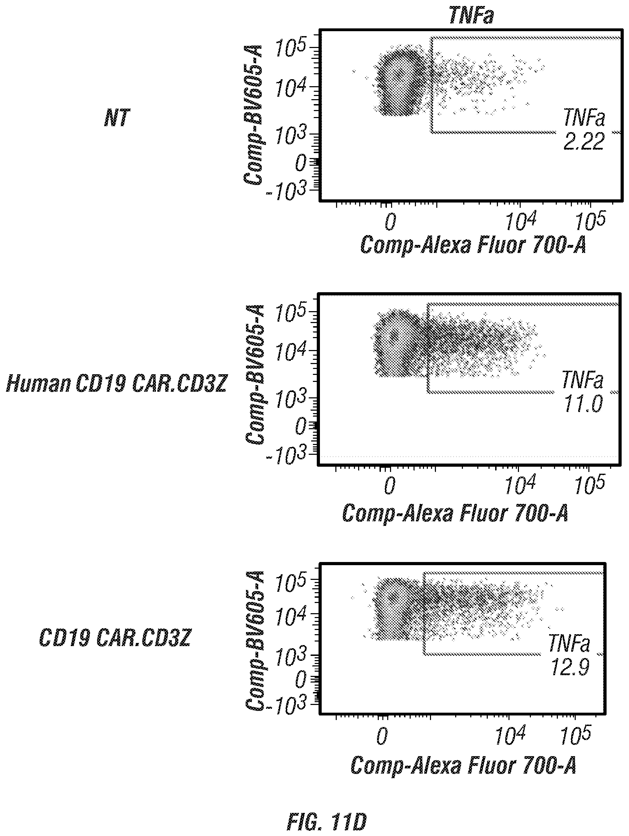

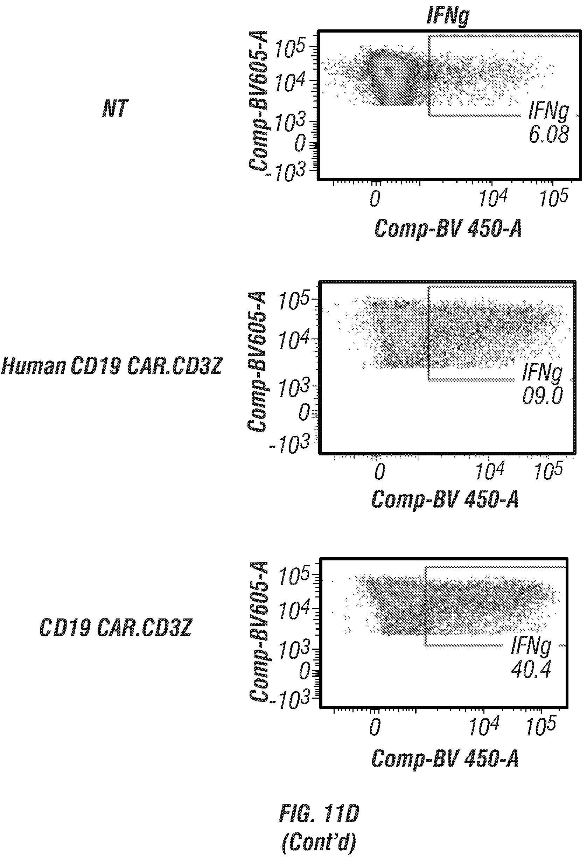

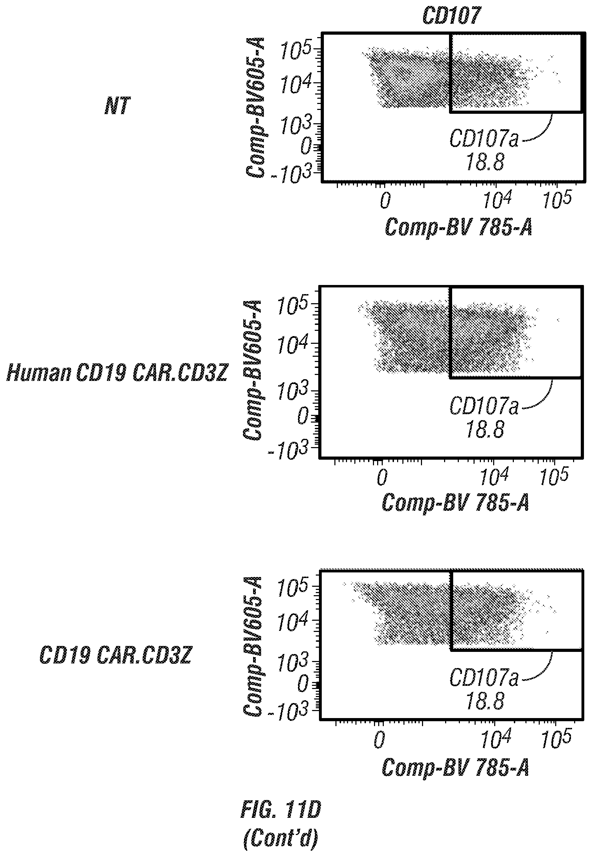

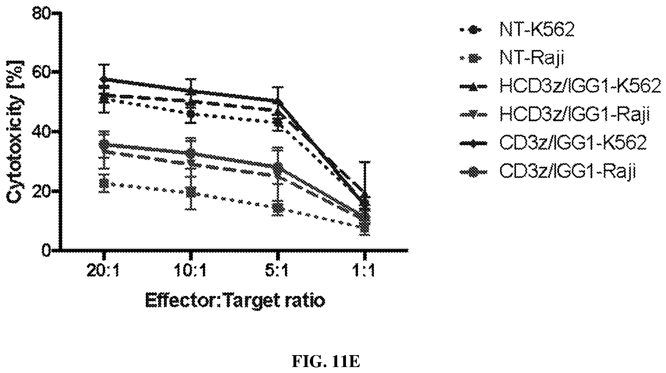

**FIGS. 11A-11E****: (A)** Schematic of CD19-CAR and hCD19-CAR constructs. **(B)** Transduction efficiency of CAR. **(C)** Cytotoxicity of different CAR constructs against K562 cells. **(D)** Cytotoxicity of different CAR constructs against Raji cells. **(E)** Cytotoxicity assay for cells with control CD3ζ/IgG1 CAR or humanized CD3ζ/IgG1 CAR against K562 or Raji cells at indicated effector:target ratios.

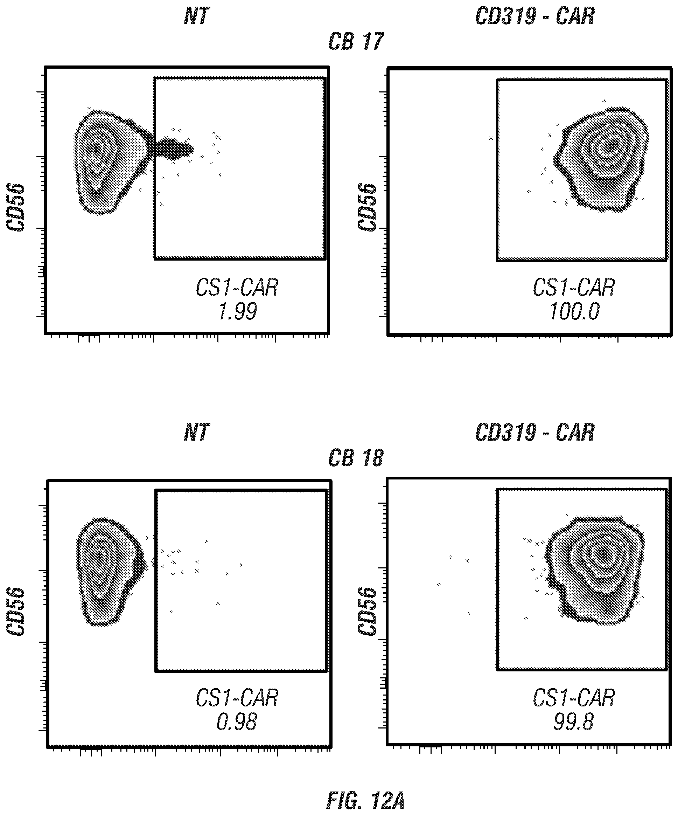

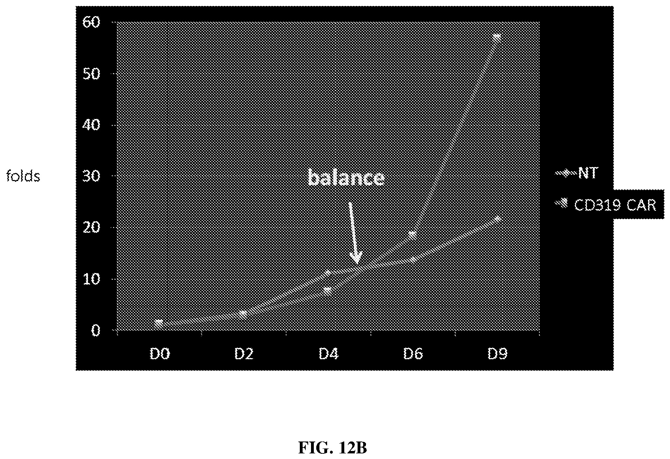

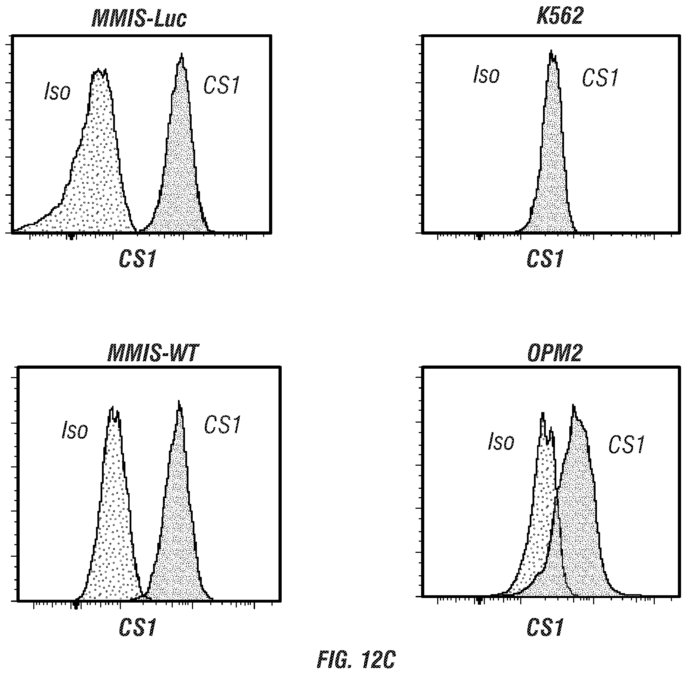

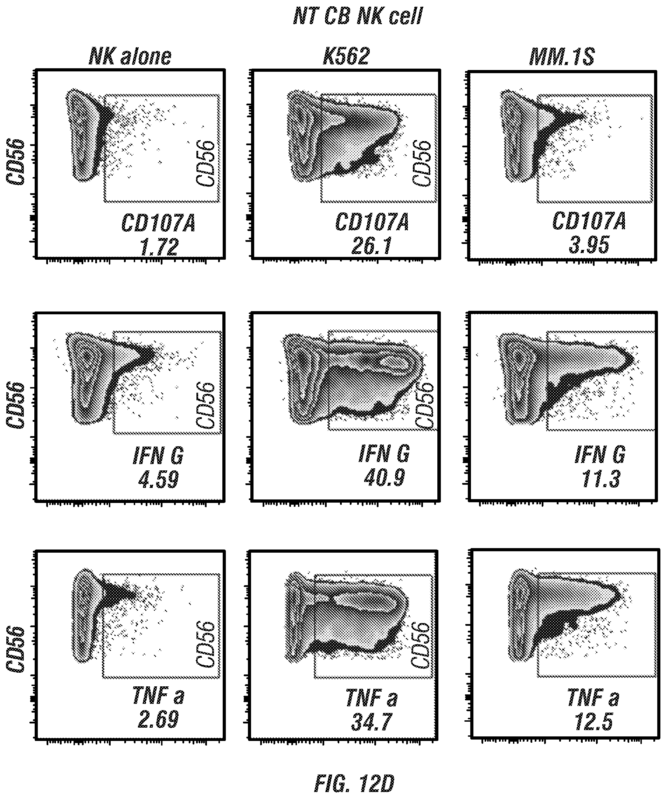

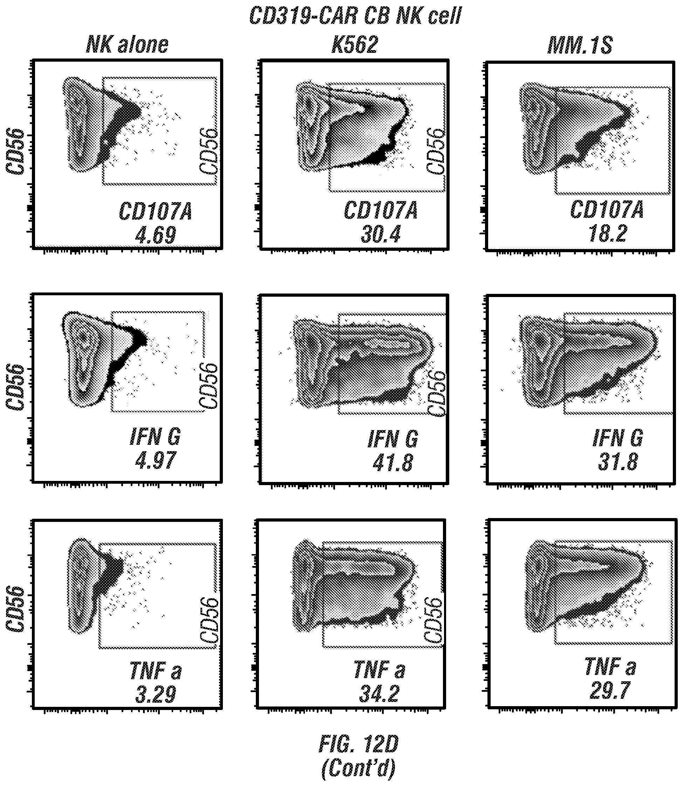

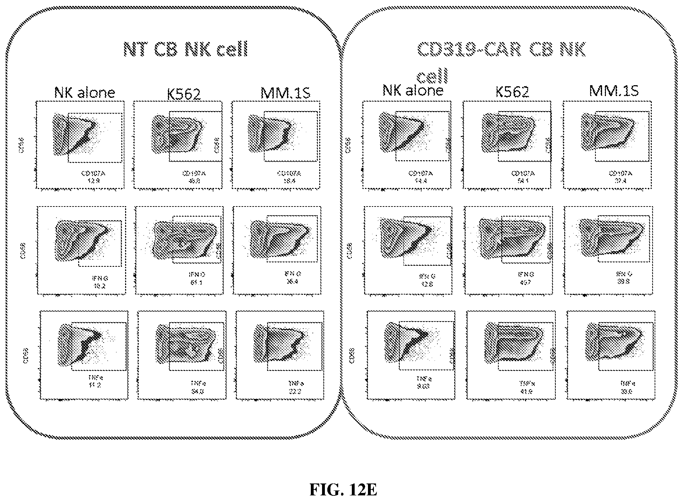

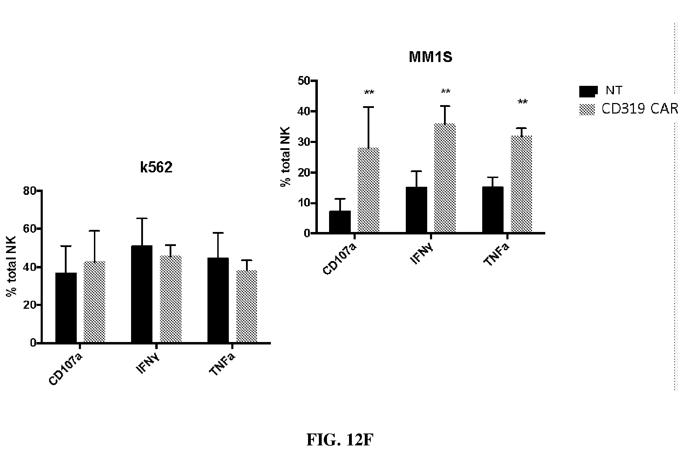

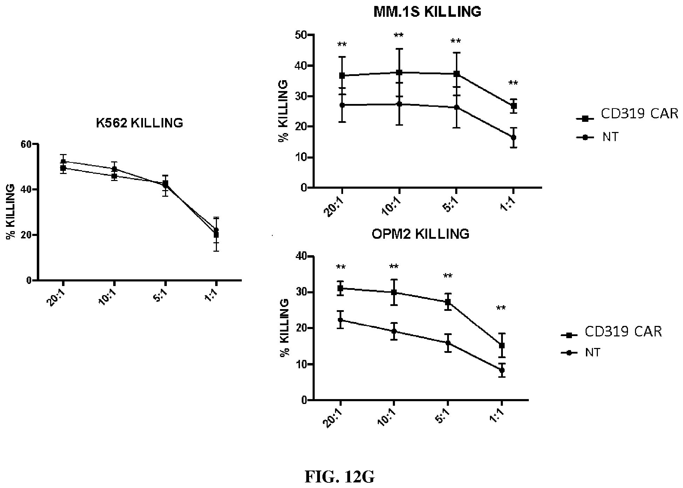

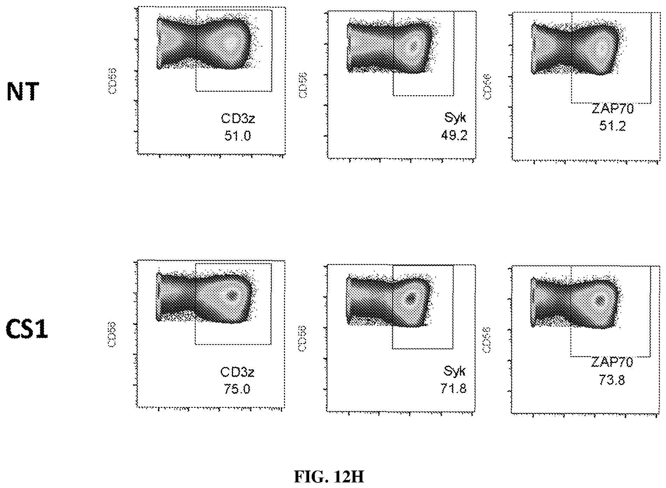

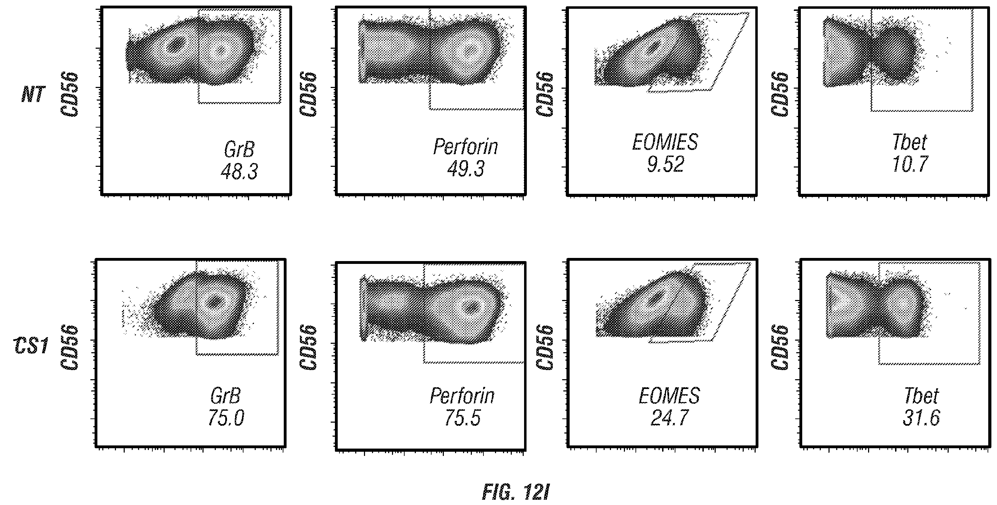

**FIGS. 12A-12I****: (A)** CD319-CAR NK transduction. **(B)** Growth curve of NK cells. **(C)** CD319 expression in cell lines. **(D-F)** CD319-CAR NK cytotoxicity assay. **(G)** Cytotoxicity assay with CD319-CAR NK cells against indicated cell types including K562, MM.1S, and OPM2. **(H-I)** CD319-CAR NK cells have an effector phenotype.

### DESCRIPTION OF ILLUSTRATIVE EMBODIMENTS

Chimeric antigen receptor (CAR) based cellular immunotherapy has shown significant efficacies in cancer treatment, in particular for treatment of leukemia and lymphoma. Nevertheless, currently available CARs can comprise various design defects. Recent clinical results using CARs to reprogram immune cells for targeted cancer immunotherapy have shown potent anti-tumor cytotoxicity. Since most CAR single chain variable fragment (scFv) modules are derivatives of murine antibodies, human anti-mouse immune-reactivity compromises the efficacy of CARs thus derived.

Thus, in some embodiments, the present disclosure provides a platform technology to engineer humanized CARs (hCARs) against antigens. The antigens may include CD19 (hCD19CAR), CD123 (hCD123CAR), mesothelin (hMesoCAR), ROR1 (hROR1CAR), CD5 (hCD5CAR), CLL-1 (hCLL-1CAR), and/or CD99 (hCD99CAR) as well as other antigens that may be targeted for treatment of a disease, such as cancer or autoimmune disorders. Accordingly, in certain embodiments, the present disclosure further provides humanized CARs and methods of their use in the treatment of cancer.

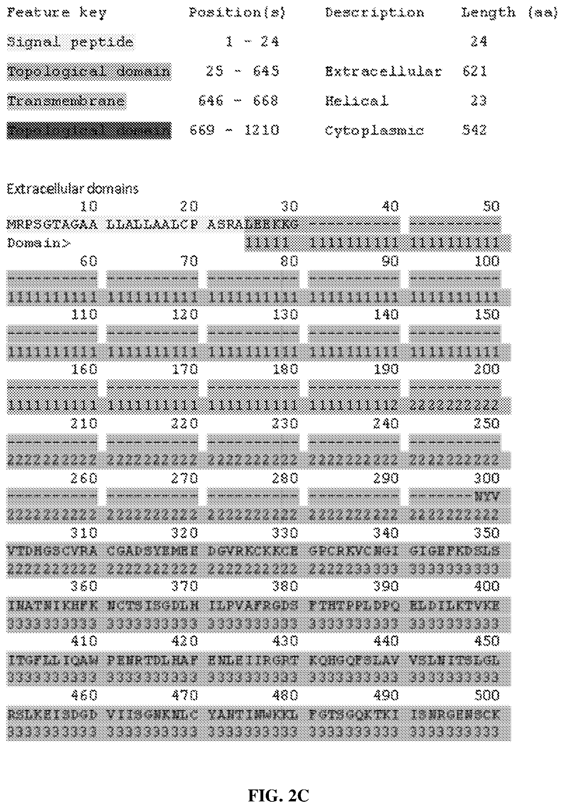

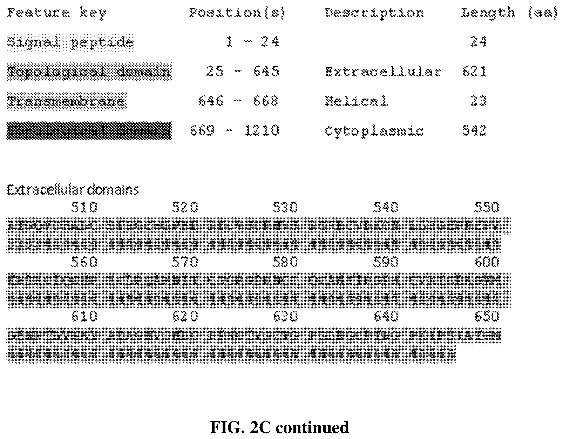

In some embodiments, the present disclosure provides a platform for the humanization of CARs, specifically the scFv of the CAR. The process follows 5 modular components as described in Example 1. These can include sequence analysis, 3D structure modeling, human framework grafting, humanization scoring, and functional analysis. Next, IgG is focused on for the humanization process, making use of extensive sequence variations in the allotypes (*i.e.,* versions of the same isotype) to fine tune for human framework selection (FIG. 2).

In the first step, the antibody sequences for human heavy and light chains can be curated in a local database from sources such as GenBank. The variable domains may be aligned using IgBLAST (a version of BLAST) to delineate allotypes and sort the sequences according to homologies. CDR regions can then be identified and framework sequences are cataloged (FIG. 1A). 3D structure modeling may then be performed, such as using a protein structure homology-modeler (*e.g.*, SWISS-MODEL).

In some embodiments, immune cells are engineered to express CARs with the humanized scFvs provided herein. The immune cells may be NK cells and/or T cells, such as derived from peripheral blood, umbilical cord blood, or marrow-derived infiltrating lymphocytes. The immune cells with a scFv derived from the present humanization platform can mitigate the possibility of human anti-mouse antibody (HAMA) production and associated immune-mediated rejection of genetically modified immune cell therapy products.

Thus, further embodiments provide methods of treating diseases or disorders by administering immune cells which express CARs with humanized scFvs. These methods can be used to lower immune rejection as well as longer persistence of infused cell therapy products. Exemplary subjects that may be treated include cancer patients with CD19⁺ CLL, ALL and NHL; CD123-positive malignancies including AML, CML. MDS and BPDCN; Mesothelin-positive cancers including cancers of the cervix, endometrium, and the head and neck; ROR1-positive malignancies including CLL (e.g, without prior CAR therapy or those who relapse following CD19-CAR with CD19-negative disease),breast, ovary and pancreatic cancers; CD5-positive malignancies including ALL and NHL as well as thymic cancer.

In further embodiments, the present disclosure provides CARs comprising truncated EGFRvIII wherein the receptor does not comprise the cytoplasmic endodomain or a functional EGF binding domain, referred to herein as Ev3. EGFRvIII is an EGFR variant that is found only in tumor cells, such as glioblastoma multiforme and lung cancer, and not in normal EGFR expressing cells such as epithelial cells. EGFRvIII has a somatic mutation resulting in deletion of exons 2-7 (801 base pair in-frame deletion, 267 amino acid missing compared to wild-type). The deletion of EGFRvIII also covers most of domains 1 and 2 resulting in a truncated domain 1 and truncated domain 2. Further, EGFRvIII has a specific epitope as a result of the fusion between exon 1 and exon 8 creating an extra glycine residue (amino acids 6-273 are replaced by a glycine residue; *i.e.,* LEEKKGNYVVTD), which forms a tumor-specific antigen, such as for glioblastoma multiforme.

The truncated EGFRvIII provided herein, referred to as Ev3 (or tEv3), does not comprise an endodomain or functional EGF binding domain. Specifically, the Ev3 extracellular domain may be used as the CAR stalk, excluding the transmembrane and intracellular signaling domain, for a) signal transduction, b) cellular marker for assigning CAR positivity and, c) antigenic marker for cell ablation via antibody binding (*e.g.*, with Cetuximab). In particular aspects, Ev3 comprises truncated domain 1 (L1), truncated domain 2 (CR1), domain 3 (L2), and domain 4 (CR2) of EGFR (as depicted in FIG. 2B). An exemplary sequence of Ev3 is depicted in FIG. 2C. The present Ev3 may comprise a sequence at least 90%, 95%, 96%, 97%, 98%, or 99% identical to the sequence in FIG. 2C. The cetuximab binding site (in domain 3) is preserved in both Ev3 and wild-type EGFR extracellular domains. In addition to the cetuximab (and/or panitumumab) binding site, the Ev3 epitope presents additional binding sites for other antibodies (*e.g.*, MR1, mAb 806, and DH8.3)

The CARs provided herein can use Ev3 as an integral signal transduction hinge which can double as an antigenic safety switch to covalently link the scFv *(i.e.,* the antigen recognition domain) with the signaling domains *(i.e.,* CD28 and CD3ζ). The Ev3 also allows for detection of CAR immune cells *in vitro* and *in vivo.* In addition, the Ev3 hinge ensures retention of genetic loads (CARs).

A further aspect of the Ev3 hinge that is provided herein is its ability to be targeted by antibodies, such as MR1, which as specific to EGFRvIII and will not cross-react with domain III and IV of EGFR which is present in other tissues and cells, such as epithelial cells. Cetuximab and panitumumab are clinically available antibodies and FDA-approved which obviates the need to acquire regulatory approval for idiotypic antibodies for each CAR with different antigenic specificities, such as CD19, CD123, CD5, ROR1, CD33, CD99, and mesothelin. The ability to target Ev3-CARs can be used to detect CAR-positive cells and/or controlled cellular ablation. Cellular ablation may be desired in the event of adverse events, such as cytokine release syndrome, neurotoxicity, and/or on-target or off tumor activity. As Ev3 is not normally expressed in immune cells, such as T or NK cells, enforced expression of Ev3 will specifically mark genetically-modified immune cells. Finally, Ev3 does not comprise a signaling endodomain as the cytoplasmic domain is removed) or an EGF binding domain, CARs comprising Ev3 will not respond or crosstalk with EGF signaling pathways (*e.g*., AR, ARF4, CAV1, CAV3, CBL, CBLB, CBLC, CD44, CDC25A, CRK, CTNNB1, DCN, EGF, GRB14, Grb2, JAK2, MUC1, NCK1, NCK2, PKCα, PLCG1, PLSCR1, PTPN1, PTPN11, PTPN6, PTPRK, SH2D3A, SH3KBP1, SHC1, SOS1, Src, STAT1, STAT3, STAT3, STAT5A, UBC, WAS) either outside or inside the cell. Accordingly, in particular aspects, none of these gene products are expected to interfere with the Ev3 hinge of Ev3-CARS. In certain aspects, the Ev3 may have one, two, three, or more modifications.

In additional aspects, the function, efficacy, and cellular persistence of Ev3-CAR may be further enhanced by the incorporation of the DNAX-activation protein 12 (DAP12) signaling module, an important element in NK cells activation, in Ev3-DAP12-CARs.

Further embodiments provide methods of introducing the CARs provided herein to immune cells, such as T cell and NK cells. The CAR-T or CAR-NK cells may be administered to a subject, such as for the treatment of cancer. In particular, the cancers that may be treated by the CAR-T or CAR-NK cells include leukemia and lymphoma, such as chronic lymphocytic leukemia (CLL), acute lymphocytic leukemia (ALL), and non-hodgkin lymphoma (NHL).

### I. Definitions

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more. The terms "about", "substantially" and "approximately" mean, in general, the stated value plus or minus 5%.

The term "exogenous," when used in relation to a protein, gene, nucleic acid, or polynucleotide in a cell or organism refers to a protein, gene, nucleic acid, or polynucleotide that has been introduced into the cell or organism by artificial or natural means; or in relation to a cell, the term refers to a cell that was isolated and subsequently introduced to other cells or to an organism by artificial or natural means. An exogenous nucleic acid may be from a different organism or cell, or it may be one or more additional copies of a nucleic acid that occurs naturally within the organism or cell. An exogenous cell may be from a different organism, or it may be from the same organism. By way of a non-limiting example, an exogenous nucleic acid is one that is in a chromosomal location different from where it would be in natural cells, or is otherwise flanked by a different nucleic acid sequence than that found in nature.

By "expression construct" or "expression cassette" is meant a nucleic acid molecule that is capable of directing transcription. An expression construct includes, at a minimum, one or more transcriptional control elements (such as promoters, enhancers or a structure functionally equivalent thereof) that direct gene expression in one or more desired cell types, tissues or organs. Additional elements, such as a transcription termination signal, may also be included.

A "vector" or "construct" (sometimes referred to as a gene delivery system or gene transfer "vehicle") refers to a macromolecule or complex of molecules comprising a polynucleotide to be delivered to a host cell, either *in vitro* or *in vivo.*

A "plasmid," a common type of a vector, is an extra-chromosomal DNA molecule separate from the chromosomal DNA that is capable of replicating independently of the chromosomal DNA. In certain cases, it is circular and double-stranded.

As used herein, the term "patient" or "subject" refers to a living mammalian organism, such as a human, monkey, cow, sheep, goat, dog, cat, mouse, rat, guinea pig, or transgenic species thereof. In certain embodiments, the patient or subject is a primate. Non-limiting examples of human patients are adults, juveniles, infants and fetuses.

An "immune disorder," "immune-related disorder," or "immune-mediated disorder" refers to a disorder in which the immune response plays a key role in the development or progression of the disease. Immune-mediated disorders include autoimmune disorders, allograft rejection, graft versus host disease and inflammatory and allergic conditions.

An "immune response" is a response of a cell of the immune system, such as a B cell, or a T cell, or innate immune cell to a stimulus. In one embodiment, the response is specific for a particular antigen (an "antigen-specific response").

The terms "tumor-associated antigen," "tumor antigen" and "cancer cell antigen" are used interchangeably herein. In each case, the terms refer to proteins, glycoproteins or carbohydrates that are specifically or preferentially expressed by cancer cells.

An "epitope" is the site on an antigen recognized by an antibody as determined by the specificity of the amino acid sequence. Two antibodies are said to bind to the same epitope if each competitively inhibits (blocks) binding of the other to the antigen as measured in a competitive binding assay. Alternatively, two antibodies have the same epitope if most amino acid mutations in the antigen that reduce or eliminate binding of one antibody reduce or eliminate binding of the other. Two antibodies are said to have overlapping epitopes if each partially inhibits binding of the other to the antigen, and/or if some amino acid mutations that reduce or eliminate binding of one antibody reduce or eliminate binding of the other.

"Treating" or treatment of a disease or condition refers to executing a protocol, which may include administering one or more drugs to a patient, in an effort to alleviate signs or symptoms of the disease. Desirable effects of treatment include decreasing the rate of disease progression, ameliorating or palliating the disease state, and remission or improved prognosis. Alleviation can occur prior to signs or symptoms of the disease or condition appearing, as well as after their appearance. Thus, "treating" or "treatment" may include "preventing" or "prevention" of disease or undesirable condition. In addition, "treating" or "treatment" does not require complete alleviation of signs or symptoms, does not require a cure, and specifically includes protocols that have only a marginal effect on the patient.

The term "effective," as that term is used in the specification and/or claims, means adequate to accomplish a desired, expected, or intended result. "Effective amount," "Therapeutically effective amount" or "pharmaceutically effective amount" when used in the context of treating a patient or subject with a compound means that amount of the compound which, when administered to a subject or patient for treating or preventing a disease, is an amount sufficient to effect such treatment or prevention of the disease.

"Treatment" or "treating" includes (1) inhibiting a disease in a subject or patient experiencing or displaying the pathology or symptomatology of the disease (e.g., arresting further development of the pathology and/or symptomatology), (2) ameliorating a disease in a subject or patient that is experiencing or displaying the pathology or symptomatology of the disease (e.g., reversing the pathology and/or symptomatology), and/or (3) effecting any measurable decrease in a disease or symptom thereof in a subject or patient that is experiencing or displaying the pathology or symptomatology of the disease.

"Prevention" or "preventing" includes: (1) inhibiting the onset of a disease in a subject or patient which may be at risk and/or predisposed to the disease but does not yet experience or display any or all of the pathology or symptomatology of the disease, and/or (2) slowing the onset of the pathology or symptomatology of a disease in a subject or patient which may be at risk and/or predisposed to the disease but does not yet experience or display any or all of the pathology or symptomatology of the disease.

As used herein, the term "framework region(s)" refers to regions of the variable region of an antibody which act as a scaffold for the CDRs. Thus, the framework regions may comprise the non-CDR sequences of the variable light chain and variable heavy chain. The CDRs of a variable region may be determined by methods known in the art, such as by using the Kabat numbering system as described in Sela-Culang *et al.,* 2013; incorporated herein by reference in its entirety. The system described by Kabat (CITE) not only provides an unambiguous residue numbering system applicable to any variable region of an antibody, but also provides precise residue boundaries defining the three CDRs.

As generally used herein "pharmaceutically acceptable" refers to those compounds, materials, compositions, and/or dosage forms which are, within the scope of sound medical judgment, suitable for use in contact with the tissues, organs, and/or bodily fluids of human beings and animals without excessive toxicity, irritation, allergic response, or other problems or complications commensurate with a reasonable benefit/risk ratio.

"Pharmaceutically acceptable salts" means salts of compounds disclosed herein which are pharmaceutically acceptable, as defined above, and which possess the desired pharmacological activity. Such salts include acid addition salts formed with inorganic acids such as hydrochloric acid, hydrobromic acid, sulfuric acid, nitric acid, phosphoric acid, and the like; or with organic acids such as 1,2-ethanedisulfonic acid, 2-hydroxyethanesulfonic acid, 2-naphthalenesulfonic acid, 3-phenylpropionic acid, 4,4'-methylenebis(3-hydroxy-2-ene-1-carboxylic acid), 4-methylbicyclo[2.2.2]oct-2-ene-1-carboxylic acid, acetic acid, aliphatic mono- and dicarboxylic acids, aliphatic sulfuric acids, aromatic sulfuric acids, benzenesulfonic acid, benzoic acid, camphorsulfonic acid, carbonic acid, cinnamic acid, citric acid, cyclopentanepropionic acid, ethanesulfonic acid, fumaric acid, glucoheptonic acid, gluconic acid, glutamic acid, glycolic acid, heptanoic acid, hexanoic acid, hydroxynaphthoic acid, lactic acid, laurylsulfuric acid, maleic acid, malic acid, malonic acid, mandelic acid, methanesulfonic acid, muconic acid, o-(4-hydroxybenzoyl)benzoic acid, oxalic acid, p-chlorobenzenesulfonic acid, phenyl-substituted alkanoic acids, propionic acid, p-toluenesulfonic acid, pyruvic acid, salicylic acid, stearic acid, succinic acid, tartaric acid, tertiarybutylacetic acid, trimethylacetic acid, and the like. Pharmaceutically acceptable salts also include base addition salts which may be formed when acidic protons present are capable of reacting with inorganic or organic bases. Acceptable inorganic bases include sodium hydroxide, sodium carbonate, potassium hydroxide, aluminum hydroxide and calcium hydroxide. Acceptable organic bases include ethanolamine, diethanolamine, triethanolamine, tromethamine, N-methylglucamine and the like. It should be recognized that the particular anion or cation forming a part of any salt of this invention is not critical, so long as the salt, as a whole, is pharmacologically acceptable. Additional examples of pharmaceutically acceptable salts and their methods of preparation and use are presented in Handbook of Pharmaceutical Salts: Properties, and Use (P. H. Stahl & C. G. Wermuth eds., Verlag Helvetica Chimica Acta, 2002).

A "pharmaceutically acceptable carrier," "drug carrier," or simply "carrier" is a pharmaceutically acceptable substance formulated along with the active ingredient medication that is involved in carrying, delivering and/or transporting a chemical agent. Drug carriers may be used to improve the delivery and the effectiveness of drugs, including for example, controlled-release technology to modulate drug bioavailability, decrease drug metabolism, and/or reduce drug toxicity. Some drug carriers may increase the effectiveness of drug delivery to the specific target sites. Examples of carriers include: liposomes, microspheres (e.g., made of poly(lactic-co-glycolic) acid), albumin microspheres, synthetic polymers, nanofibers, protein-DNA complexes, protein conjugates, erythrocytes, virosomes, and dendrimers.

The term "chimeric antigen receptors (CARs)," as used herein, may refer to artificial T cell receptors, chimeric T cell receptors, or chimeric immunoreceptors, for example, and encompass engineered receptors that graft an artificial specificity onto a particular immune effector cell. CARs may be employed to impart the specificity of a monoclonal antibody onto a T cell, thereby allowing a large number of specific T cells to be generated, for example, for use in adoptive cell therapy. In specific embodiments, CARs direct specificity of the cell to a tumor associated antigen, for example. In some embodiments, CARs comprise an intracellular activation domain, a transmembrane domain, and an extracellular domain comprising a tumor associated antigen binding region. In particular aspects, CARs comprise fusions of single-chain variable fragments (scFv) derived from monoclonal antibodies, fused to CD3-zeta a transmembrane domain and endodomain. The specificity of other CAR designs may be derived from ligands of receptors (*e.g.*, peptides) or from pattern-recognition receptors, such as Dectins. In certain cases, the spacing of the antigen-recognition domain can be modified to reduce activation-induced cell death. In certain cases, CARs comprise domains for additional co-stimulatory signaling, such as CD3ζ, FcR, CD27, CD28, CD137, DAP10, and/or OX40. In some cases, molecules can be co-expressed with the CAR, including co-stimulatory molecules, reporter genes for imaging *(e.g.,* for positron emission tomography), gene products that conditionally ablate the T cells upon addition of a pro-drug, homing receptors, chemokines, chemokine receptors, cytokines, and cytokine receptors.

The term "antigen presenting cells (APCs)" refers to a class of cells capable of presenting one or more antigens in the form of peptide-MHC complex recognizable by specific effector cells of the immune system, and thereby inducing an effective cellular immune response against the antigen or antigens being presented. APCs can be intact whole cells such as macrophages, B-cells, endothelial cells, activated T-cells, and dendritic cells; or other molecules, naturally occurring or synthetic, such as purified MHC Class I molecules complexed to β2-microglobulin. While many types of cells may be capable of presenting antigens on their cell surface for T-cell recognition, only dendritic cells have the capacity to present antigens in an efficient amount to activate naive T-cells for cytotoxic T-lymphocyte (CTL) responses.

The term "culturing" refers to the *in vitro* maintenance, differentiation, and/or propagation of cells in suitable media. By "enriched" is meant a composition comprising cells present in a greater percentage of total cells than is found in the tissues where they are present in an organism.

An "anti-cancer" agent is capable of negatively affecting a cancer cell/tumor in a subject, for example, by promoting killing of cancer cells, inducing apoptosis in cancer cells, reducing the growth rate of cancer cells, reducing the incidence or number of metastases, reducing tumor size, inhibiting tumor growth, reducing the blood supply to a tumor or cancer cells, promoting an immune response against cancer cells or a tumor, preventing or inhibiting the progression of cancer, or increasing the lifespan of a subject with cancer.

The term "suicide gene" as used herein is defined as a gene which may be used to selectively target cells for killing.

A polynucleotide or polynucleotide region (or a polypeptide or polypeptide region) has a certain percentage (for example, 80%, 85%, 90%, or 95%) of "sequence identity" or "homology" to another sequence means that, when aligned, that percentage of bases (or amino acids) are the same in comparing the two sequences. This alignment and the percent homology or sequence identity can be determined using software programs known in the art, for example those described in CURRENT PROTOCOLS IN MOLECULAR BIOLOGY (F. M. Ausubel et al., eds., 1987) Supplement 30, section 7.7.18, Table 7.7.1. Preferably, default parameters are used for alignment. A preferred alignment program is BLAST, using default parameters. In particular, preferred programs are BLASTN and BLASTP, using the following default parameters: Genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+SwissProtein+SPupdate+PIR.

The term "truncated" as used herein refers to a fragment of a protein or domain of a protein which is shorter than the full length protein or domain of a protein. The truncation may be by removal of 1 or more amino acids, such as 5, 10, 15, 20, 25, 30 or more amino acids of the full length sequence.

### II. Immune Cells

Certain embodiments of the present disclosure concern immune cells which express a CAR, such as with Ev3 as the hinge of the CAR and/or a humanized scFv as the antigen binding domain of the cAR. The immune cells may be T cells *(e.g.,* regulatory T cells, CD4⁺ T cells, CD8⁺ T cells, or gamma-delta T cells), NK cells, invariant NK cells, NKT cells, stem cells (*e.g.*, mesenchymal stem cells (MSCs) or induced pluripotent stem (iPSC) cells). In some embodiments, the cells are monocytes or granulocytes, e.g., myeloid cells, macrophages, neutrophils, dendritic cells, mast cells, eosinophils, and/or basophils. Also provided herein are methods of producing and engineering the immune cells as well as methods of using and administering the cells for adoptive cell therapy, in which case the cells may be autologous or allogeneic. Thus, the immune cells may be used as immunotherapy, such as to target cancer cells.

The immune cells may be isolated from subjects, particularly human subjects. The immune cells can be obtained from a subject of interest, such as a subject suspected of having a particular disease or condition, a subject suspected of having a predisposition to a particular disease or condition, or a subject who is undergoing therapy for a particular disease or condition. Immune cells can be collected from any location in which they reside in the subject including, but not limited to, blood, cord blood, spleen, thymus, lymph nodes, and bone marrow. The isolated immune cells may be used directly, or they can be stored for a period of time, such as by freezing.

The immune cells may be enriched/purified from any tissue where they reside including, but not limited to, blood (including blood collected by blood banks or cord blood banks), spleen, bone marrow, tissues removed and/or exposed during surgical procedures, and tissues obtained via biopsy procedures. Tissues/organs from which the immune cells are enriched, isolated, and/or purified may be isolated from both living and non-living subjects, wherein the non-living subjects are organ donors. In particular embodiments, the immune cells are isolated from blood, such as peripheral blood or cord blood. In some aspects, immune cells isolated from cord blood have enhanced immunomodulation capacity, such as measured by CD4- or CD8-positive T cell suppression. In specific aspects, the immune cells are isolated from pooled blood, particularly pooled cord blood, for enhanced immunomodulation capacity. The pooled blood may be from 2 or more sources, such as 3, 4, 5, 6, 7, 8, 9, 10 or more sources *(e.g.,* donor subjects).

The population of immune cells can be obtained from a subject in need of therapy or suffering from a disease associated with reduced immune cell activity. Thus, the cells will be autologous to the subject in need of therapy. Alternatively, the population of immune cells can be obtained from a donor, preferably a histocompatibility matched donor. The immune cell population can be harvested from the peripheral blood, cord blood, bone marrow, spleen, or any other organ/tissue in which immune cells reside in said subject or donor. The immune cells can be isolated from a pool of subjects and/or donors, such as from pooled cord blood.

When the population of immune cells is obtained from a donor distinct from the subject, the donor is preferably allogeneic, provided the cells obtained are subject-compatible in that they can be introduced into the subject. Allogeneic donor cells are may or may not be human-leukocyte-antigen (HLA)-compatible.

### A. T Cells

**In** some embodiments, the immune cells are T cells. Several basic approaches for the derivation, activation and expansion of functional anti-tumor effector cells have been described in the last two decades. These include: autologous cells, such as tumor-infiltrating lymphocytes (TILs); T cells activated *ex-vivo* using autologous DCs, lymphocytes, artificial antigen-presenting cells (APCs) or beads coated with T cell ligands and activating antibodies, or cells isolated by virtue of capturing target cell membrane; allogeneic cells naturally expressing anti-host tumor T cell receptor (TCR); and non-tumor-specific autologous or allogeneic cells genetically reprogrammed or "redirected" to express tumor-reactive TCR or chimeric TCR molecules displaying antibody-like tumor recognition capacity known as "T-bodies". These approaches have given rise to numerous protocols for T cell preparation and immunization which can be used in the methods described herein.

In some embodiments, the T cells are derived from the blood, bone marrow, lymph, umbilical cord, or lymphoid organs. In some aspects, the cells are human cells. The cells typically are primary cells, such as those isolated directly from a subject and/or isolated from a subject and frozen. In some embodiments, the cells include one or more subsets of T cells or other cell types, such as whole T cell populations, CD4⁺ cells, CD8⁺ cells, and subpopulations thereof, such as those defined by function, activation state, maturity, potential for differentiation, expansion, recirculation, localization, and/or persistence capacities, antigen- specificity, type of antigen receptor, presence in a particular organ or compartment, marker or cytokine secretion profile, and/or degree of differentiation. With reference to the subject to be treated, the cells may be allogeneic and/or autologous. In some aspects, such as for off-the-shelf technologies, the cells are pluripotent and/or multipotent, such as stem cells, such as induced pluripotent stem cells (iPSCs). In some embodiments, the methods include isolating cells from the subject, preparing, processing, culturing, and/or engineering them, as described herein, and re-introducing them into the same patient, before or after cryopreservation.

Among the sub-types and subpopulations of T cells (*e.g.,* CD4⁺ and/or CD8⁺ T cells) are naive T (T_{N}) cells, effector T cells (T_{EFF}), memory T cells and sub-types thereof, such as stem cell memory T (TSC_{M}), central memory T (TC_{M}), effector memory T (T_{EM}), or terminally differentiated effector memory T cells, tumor-infiltrating lymphocytes (TIL), immature T cells, mature T cells, helper T cells, cytotoxic T cells, mucosa-associated invariant T (MAIT) cells, naturally occurring and adaptive regulatory T (Treg) cells, helper T cells, such as TH1 cells, TH2 cells, TH3 cells, TH17 cells, TH9 cells, TH22 cells, follicular helper T cells, alpha/beta T cells, and delta/gamma T cells.

In some embodiments, one or more of the T cell populations is enriched for or depleted of cells that are positive for a specific marker, such as surface markers, or that are negative for a specific marker. In some cases, such markers are those that are absent or expressed at relatively low levels on certain populations of T cells *(e.g.,* non-memory cells) but are present or expressed at relatively higher levels on certain other populations of T cells (*e.g.*, memory cells).

In some embodiments, T cells are separated from a PBMC sample by negative selection of markers expressed on non-T cells, such as B cells, monocytes, or other white blood cells, such as CD14. In some aspects, a CD4⁺ or CD8⁺ selection step is used to separate CD4⁺ helper and CD8⁺ cytotoxic T cells. Such CD4⁺ and CD8⁺ populations can be further sorted into sub-populations by positive or negative selection for markers expressed or expressed to a relatively higher degree on one or more naive, memory, and/or effector T cell subpopulations.

In some embodiments, CD8⁺ T cells are further enriched for or depleted of naive, central memory, effector memory, and/or central memory stem cells, such as by positive or negative selection based on surface antigens associated with the respective subpopulation. In some embodiments, enrichment for central memory T (T_{CM}) cells is carried out to increase efficacy, such as to improve long-term survival, expansion, and/or engraftment following administration, which in some aspects is particularly robust in such sub-populations.

In some embodiments, the T cells are autologous T cells. In this method, tumor samples are obtained from patients and a single cell suspension is obtained. The single cell suspension can be obtained in any suitable manner, e.g., mechanically (disaggregating the tumor using, *e.g.*, a gentleMACS^{™} Dissociator, Miltenyi Biotec, Auburn, Calif.) or enzymatically (*e.g.*, collagenase or DNase). Single-cell suspensions of tumor enzymatic digests are cultured in interleukin-2 (IL-2).

The cultured T cells can be pooled and rapidly expanded. Rapid expansion provides an increase in the number of antigen-specific T-cells of at least about 50-fold (*e.g.,* 50-, 60-, 70-, 80-, 90-, or 100-fold, or greater) over a period of about 10 to about 14 days. More preferably, rapid expansion provides an increase of at least about 200-fold *(e.g.,* 200-, 300-, 400-, 500-, 600-, 700-, 800-, 900-, or greater) over a period of about 10 to about 14 days.

Expansion can be accomplished by any of a number of methods as are known in the art. For example, T cells can be rapidly expanded using non-specific T-cell receptor stimulation in the presence of feeder lymphocytes and either interleukin-2 (IL-2) or interleukin-15 (IL-15), with IL-2 being preferred. The non-specific T-cell receptor stimulus can include around 30 ng/ml of OKT3, a mouse monoclonal anti-CD3 antibody (available from Ortho-McNeil^{®}, Raritan, N.J.). Alternatively, T cells can be rapidly expanded by stimulation of peripheral blood mononuclear cells (PBMC) *in vitro* with one or more antigens (including antigenic portions thereof, such as epitope(s), or a cell) of the cancer, which can be optionally expressed from a vector, such as an human leukocyte antigen A2 (HLA-A2) binding peptide, in the presence of a T-cell growth factor, such as 300 IU/ml IL-2 or IL-15, with IL-2 being preferred. The *in vitro-*induced T-cells are rapidly expanded by re-stimulation with the same antigen(s) of the cancer pulsed onto HLA-A2-expressing antigen-presenting cells. Alternatively, the T-cells can be re-stimulated with irradiated, autologous lymphocytes or with irradiated HLA-A2⁺ allogeneic lymphocytes and IL-2, for example.

The autologous T cells can be modified to express a T cell growth factor that promotes the growth and activation of the autologous T cells. Suitable T cell growth factors include, for example, interleukin (IL)-2, IL-7, IL-15, and IL-12. Suitable methods of modification are known in the art. See, for instance, Sambrook et al., Molecular Cloning: A Laboratory Manual, 3rd ed., Cold Spring Harbor Press, Cold Spring Harbor, N.Y. 2001; and Ausubel et al., Current Protocols in Molecular Biology, Greene Publishing Associates and John Wiley & Sons, NY, 1994. In particular aspects, modified autologous T cells express the T cell growth factor at high levels. T cell growth factor coding sequences, such as that of IL-12, are readily available in the art, as are promoters, the operable linkage of which to a T cell growth factor coding sequence promote high-level expression.

### B. NK Cells

**In** some embodiments, the immune cells are natural killer (NK) cells. NK cells are a subpopulation of lymphocytes that have spontaneous cytotoxicity against a variety of tumor cells, virus-infected cells, and some normal cells in the bone marrow and thymus. NK cells differentiate and mature in the bone marrow, lymph nodes, spleen, tonsils, and thymus. NK cells can be detected by specific surface markers, such as CD16, CD56, and CD8 in humans. NK cells do not express T cell antigen receptors, the pan T marker CD3, or surface immunoglobulin B cell receptors.

**In** certain embodiments, NK cells are derived from human peripheral blood mononuclear cells (PBMC), unstimulated leukapheresis products (PBSC), human embryonic stem cells (hESCs), induced pluripotent stem cells (iPSCs), bone marrow, or umbilical cord blood by methods well known in the art. Particularly, umbilical CB is used to derive NK cells. In certain aspects, the NK cells are isolated and expanded by the previously described method of *ex vivo* expansion of NK cells (Spanholtz *et al.,* 2011; Shah *et al.,* 2013). In this method, CB mononuclear cells are isolated by ficoll density gradient centrifugation and cultured in a bioreactor with IL-2 and artificial antigen presenting cells (aAPCs). After 7 days, the cell culture is depleted of any cells expressing CD3 and re-cultured for an additional 7 days. The cells are again CD3-depleted and characterized to determine the percentage of CD56⁺/CD3⁻ cells or NK cells. In other methods, umbilical CB is used to derive NK cells by the isolation of CD34⁺ cells and differentiation into CD56⁺/CD3⁻ cells by culturing in medium contain SCF, IL-7, IL-15, and IL-2.

### C. Stem Cells

**In** some embodiments, the immune cells of the present disclosure may be stem cells, such as induced pluripotent stem cells (PSCs), mesenchymal stem cells (MSCs), or hematopoietic stem cells (HSCs).

The pluripotent stem cells used herein may be induced pluripotent stem (iPS) cells, commonly abbreviated iPS cells or iPSCs. With the exception of germ cells, any cell can be used as a starting point for iPSCs. For example, cell types could be keratinocytes, fibroblasts, hematopoietic cells, mesenchymal cells, liver cells, or stomach cells. There is no limitation on the degree of cell differentiation or the age of an animal from which cells are collected; even undifferentiated progenitor cells (including somatic stem cells) and finally differentiated mature cells can be used as sources of somatic cells in the methods disclosed herein.

Somatic cells can be reprogrammed to produce iPS cells using methods known to one of skill in the art. Generally, nuclear reprogramming factors are used to produce pluripotent stem cells from a somatic cell. In some embodiments, at least three, or at least four, of Klf4, c-Myc, Oct3/4, Sox2, Nanog, and Lin28 are utilized. In other embodiments, Oct3/4, Sox2, c-Myc and Klf4 are utilized or Oct3/4, Sox2, Nanog, and Lin28.

Once derived, iPSCs can be cultured in a medium sufficient to maintain pluripotency. In certain embodiments, undefined conditions may be used; for example, pluripotent cells may be cultured on fibroblast feeder cells or a medium that has been exposed to fibroblast feeder cells in order to maintain the stem cells in an undifferentiated state. In some embodiments, the cell is cultured in the co-presence of mouse embryonic fibroblasts treated with radiation or an antibiotic to terminate the cell division, as feeder cells. Alternately, pluripotent cells may be cultured and maintained in an essentially undifferentiated state using a defined, feeder-independent culture system, such as a TESR^{™} medium or E8^{™}/Essential 8^{™} medium.

### III. Enhanced Chimeric Antigen Receptors

### A. Truncated EGFRvIII (Ev3)

In certain embodiments, the present disclosure provides enhanced CARs which comprise Ev3 as the hinge or stalk region of the CAR by covalently linking the antigen binding region to the intracellular signaling domain(s). The antigen binding region may comprise a single-chain variable fragment (scFv) derived from an anti-antigen binding domain derived from an antibody, such as a mouse anti-human antibody. The scFv can target any potential antigen. The scFv may be humanized, such as in the framework region of the scFv. In particular aspects, the accessibility to the detection or ablation antibodies (e.g., via ADCC) is maintained.

The Ev3 may comprise or have at least 80% *(e.g.,* 81%, 82%, 83%, 84%, 85%, 86%, 87%, 88%, 89%, 90%, 91%, 92%, 93%, 94%, 95%, 96%, 97%, 98%, 99%, or 100%) sequence identity to SEQ ID NO:2 LEEKK-GNYVVTDHGSCVRACGADSYEMEEDGVRKCKKCEGPCRK-VCNGIGIGEFKDSLSINATNIKHFKNCTSISGDLHILPVAFRGDSFTHTPPLDPQELDIL KTVKEITGFLLIQAWPENRTDLHAFENLEIIRGRTKQHGQFSLAVVSLNITSLGLRSLK EISDGDVIISGNKNLCYANTINWKKLFGTSGQKTKIISNRGENSCKATGQ-VCHALCSPEGCWGPEPRDCVSCRNVSRGRECVDKCNLLEGEPREFVENSECIQCHPE CLPQAMNITCTGRGPDNCIQCAHYIDGPHCVKTCPAGVMGENNTLVWKYADAGHV CHLCHPNCTYGCTGPGLEGCPTNGPKIPS (comprising partial Domain 1-partial Domain 2-Domain 3-Domain 4). In certain aspects, the Ev3 may have one, two, three, or more modifications.

In one embodiment, there is provided a CD19Ev3-CAR comprising from N-terminal to C-terminal a scFv derived from a mouse anti-human CD19 monoclonal antibody, a Ev3 hinge including the transmembrane domain, a CD28 endodomain, and a CD3ζ endodomain. In another embodiment, the CD19Ev3-CAR comprises a humanized CD19 (hCD19) referred to as hCD19Ev3-CAR. The CAR may further comprise DAP12 endodomain, such as between the Ev3 transmembrane domain and the CD3ζ endodomain, such a CD19Ev3DAP12-CAR or hCD19Ev3DAP12-CAR.

In one embodiment, there is provided a CD123Ev3-CAR comprising from N-terminal to C-terminal a scFv derived from a mouse anti-human CD123 monoclonal antibody, a Ev3 hinge including the transmembrane domain, a CD28 endodomain, and a CD3ζ endodomain. In another embodiment, the CD123Ev3-CAR comprises a humanized CD123 (hCD123) referred to as hCD123Ev3-CAR. The CAR may further comprise DAP12 endodomain, such as between the Ev3 transmembrane domain and the CD3ζ endodomain, such a CD123Ev3DAP12-CAR or hCD123Ev3DAP12-CAR.

In one embodiment, there is provided a MesoEv3-CAR comprising from N-terminal to C-terminal a scFv derived from a mouse anti-human mesothelin monoclonal antibody, a Ev3 hinge including the transmembrane domain, a CD28 endodomain, and a CD3ζ endodomain. In another embodiment, the MesoEv3-CAR comprises a humanized Meso (hMeso) referred to as hMesoEv3-CAR. The CAR may further comprise DAP12 endodomain, such as between the Ev3 transmembrane domain and the CD3ζ endodomain, such a MesoEv3DAP12-CAR or hMesoEv3DAP12-CAR.

In one embodiment, there is provided a ROR1-Ev3-CAR comprising from N-terminal to C-terminal a scFv derived from a mouse anti-human ROR1 monoclonal antibody, a Ev3 hinge including the transmembrane domain, a CD28 endodomain, and a CD3ζ endodomain. In another embodiment, the ROR1-Ev3-CAR comprises a humanized ROR1 (hROR1) referred to as hROR1Ev3-CAR. The CAR may further comprise DAP12 endodomain, such as between the Ev3 transmembrane domain and the CD3ζ endodomain, such a ROR1-Ev3DAP12-CAR or hROR1-Ev3DAP12-CAR.

In one embodiment, there is provided a CD5Ev3-CAR comprising from N-terminal to C-terminal a scFv derived from a mouse anti-human CD5 monoclonal antibody, a Ev3 hinge including the transmembrane domain, a CD28 endodomain, and a CD3ζ endodomain. In another embodiment, the CD5Ev3-CAR comprises a humanized CD5 (hCD5) referred to as hCD5Ev3-CAR. The CAR may further comprise DAP12 endodomain, such as between the Ev3 transmembrane domain and the CD3ζ endodomain, such a CD5Ev3DAP12-CAR or hCD5Ev3DAP12-CAR.

In one embodiment, there is provided a CD99Ev3-CAR comprising from N-terminal to C-terminal a scFv derived from a mouse anti-human CD99 monoclonal antibody, a Ev3 hinge including the transmembrane domain, a CD28 endodomain, and a CD3ζ endodomain. In another embodiment, the CD5Ev3-CAR comprises a humanized CD99 (hCD99) referred to as hCD99Ev3-CAR. The CAR may further comprise DAP12 endodomain, such as between the Ev3 transmembrane domain and the CD3ζ endodomain, such a CD99Ev3DAP12-CAR or hCD99Ev3DAP12-CAR.

EGFRvIII is the result of a mutation of exons 2-7 of the EGFR gene which renders the mutant receptor incapable of binding any known ligand as the receptor does not have a complete ligand binding domain. The truncated EGFRvIII, Ev3, of the present disclosure further comprises a deletion of the intracellular domain or endodomain of EGFRvIII. Thus, Ev3 does not comprise a functional EGF binding domain or the intracellular signaling domain of EGFRvIII.

The Ev3 hinge may comprise a nucleotide sequence of SEQ ID NO:1 and/or an amino acid sequence of SEQ ID NO:2. In particular embodiments, the Ev3 hinge has at least 80, 85, 90, 91, 92, 93, 94, 95, 96, 97, 98, 99, or 100% sequence identity with SEQ ID NO:1 or SEQ ID NO:2. The Ev3 hinge region may comprise a fragment of SEQ ID NO:2 or may comprise additional amino acids on the N-terminal or C-terminal end of the receptor fragment. An exemplary schematic of Ev3 is depicted in FIG. 2.

The inclusion of the truncated EGFRvIII in the hinge region of a CAR allows for retention of genetic cargo load. The Ev3 can also be used a safety switch and/or for detection of the CAR *in vitro* or *in vivo.* The integral Ev4 hinge can be recognized by antibodies, such as clinically accessible monoclonal antibodies (e.g., cetuximab), for detection or cellular ablation. In addition, the Ev3 does not comprise a ligand binding domain; thus, there is no endogenous function or crosstalk with other pathways. The Ev3 is also plays a role in the CAR signal transduction and is tumor specific which can minimize side-effects of CAR therapies.

### B. Antigens

The CARs provided herein may have any antigenic specificity useful in the treatment of a disease or disorder. The CARs provided herein may comprise more than one antigen, such as two antigens. Exemplary antigens include, but are not limited to, CD19, CD123, mesothelin, CD5, CD47, CLL-1, CD33, CD99, CLL-1, U5snRNP200, CD200, CS1, BAFF-R, ROR-1, CD99, Mesothelin, or BCMA.

The humanization platform provided herein may be used for the development of a humanized CAR for any given antigen. Exemplary antigens with humanized CARs include, but are not limited to, CD19, CD123, mesothelin, CD5, CD47, CLL-1, CD33, CD99, U5snRNP200, CD200, CS1, BAFF-R, ROR-1, or BCMA.

In one embodiment, the encoded light chain variable region comprises one, two, three or all four framework regions of the human PH0879 germline sequence. The encoded heavy chain variable region of hCD19 may comprise one, two, three or all four framework regions of the human ACD43442 germline sequence.

In one embodiment, the encoded antigen binding domain is a scFv comprising a light chain and a heavy chain of an amino acid sequence of SEQ ID NO:6, 12, 18, 24, 30, 58 or 61. In one embodiment, the antigen binding domain *(e.g.,* an scFv) comprises: a light chain variable region comprising an amino acid sequence having at least one, two or three modifications *(e.g.,* substitutions) but not more than 10, 20 or 30 modifications *(e.g.,* substitutions) of an amino acid sequence of a light chain variable region provided herein, or a sequence with 90-99% identity with an amino acid sequence of SEQ ID NOs:7, 13, 19, 25, 31, 59 or 63; and/or a heavy chain variable region comprising an amino acid sequence having at least one, two or three modifications (*e.g.*, substitutions) but not more than 10, 20 or 30 modifications (*e.g*., substitutions) of an amino acid sequence of a heavy chain variable region of SEQ ID NOs:8, 14, 20, 26, 32, 60 or 64; or a sequence with 90-99% identity to an amino acid sequence of SEQ ID NOs:6, 12, 18, 24, 30, 58 or 61.

In some embodiments, a humanized scFv for CD19 is provided herein. The humanized CD19 (hCD19) scFv may have a variable light chain sequence of SEQ ID NO:7 (DIQMTQSPSSLSASVGDRVTITC**RASQDISKYLN**WYQQKPGKVPKLLIY**HTSRLHS** GVPDRFSGSGSGTDFTLTISSLQPEDVATYYC**QQGNTLPYT**FGQGTKVEIKR) and a variable heavy chain of SEQ ID NO:8 (EVQLVESGGGLVQPGGSLRLSCAAS**GVSLPDYGVS**WVRQAPGKGLEWIG**VIWGSE TTYYNSALKS**KFIISRDNAKNSLYLQMNSLRAEDTAVYYCAR**HYYYGGSYAMDY** WGQGTLVTVSS). In other aspects, the hCD19 scFv may comprise a V_{L} with at least 80%, 85%, 90%, 91%, 921%, 93%, 94%, 95%, 96%, 97%, 98%, or 99% sequence identity to SEQ ID NO:7 and/or a V_{H} with at least 80%, 85%, 90%, 91%, 921%, 93%, 94%, 95%, 96%, 97%, 98%, or 99% sequence identity to SEQ ID NO:8. The hCD19 scFv may comprise an amino acid sequence with at least 80%, 85%, 90%, 91%, 921%, 93%, 94%, 95%, 96%, 97%, 98%, 99%, or 100% sequence identity to SEQ ID NO:6. The hCD19 scFv may be encoded by a polynucleotide with at least 80%, 85%, 90%, 91%, 921%, 93%, 94%, 95%, 96%, 97%, 98%, 99%, or 100% sequence identity to SEQ ID NO:3. The V_{L} of the hCD19 scFv may be encoded by a polynucleotide with at least 80%, 85%, 90%, 91%, 921%, 93%, 94%, 95%, 96%, 97%, 98%, 99%, or 100% sequence identity to SEQ ID NO:4. The V_{H} of the hCD19 scFv may be encoded by a polynucleotide with at least 80%, 85%, 90%, 91%, 921%, 93%, 94%, 95%, 96%, 97%, 98%, 99%, or 100% sequence identity to SEQ ID NO:5.

In some embodiments, a humanized scFv for CD123 is provided herein. The humanized CD123 (hCD123) scFv may have a variable light chain sequence of SEQ ID NO:13 and a variable heavy chain of SEQ ID NO:14. In other aspects, the hCD123 scFv may comprise a V_{L} with at least 80%, 85%, 90%, 91%, 92%, 93%, 94%, 95%, 96%, 97%, 98%, or 99% sequence identity to SEQ ID NO:13 and/or a V_{H} with at least 80%, 85%, 90%, 91%, 92%, 93%, 94%, 95%, 96%, 97%, 98%, or 99% sequence identity to SEQ ID NO:14. The hCD123 scFv may comprise an amino acid sequence with at least 80%, 85%, 90%, 91%, 92%, 93%, 94%, 95%, 96%, 97%, 98%, 99%, or 100% sequence identity to SEQ ID NO:12. The hCD123 scFv may be encoded by a polynucleotide with at least 80%, 85%, 90%, 91%, 92%, 93%, 94%, 95%, 96%, 97%, 98%, 99%, or 100% sequence identity to SEQ ID NO:9. The V_{L} of the hCD123 scFv may be encoded by a polynucleotide with at least 80%, 85%, 90%, 91%, 92%, 93%, 94%, 95%, 96%, 97%, 98%, 99%, or 100% sequence identity to SEQ ID NO:10. The V_{H} of the hCD123 scFv may be encoded by a polynucleotide with at least 80%, 85%, 90%, 91%, 92%, 93%, 94%, 95%, 96%, 97%, 98%, 99%, or 100% sequence identity to SEQ ID NO:11.

In some embodiments, a humanized scFv for mesothelin is provided herein. The humanized mesothelin (hMeso) scFv may have a variable light chain sequence of SEQ ID NO:19 and a variable heavy chain of SEQ ID NO:20. In other aspects, the hMeso scFv may comprise a V_{L} with at least 80%, 85%, 90%, 91%, 921%, 93%, 94%, 95%, 96%, 97%, 98%, or 99% sequence identity to SEQ ID NO:19 and/or a V_{H} with at least 80%, 85%, 90%, 91%, 921%, 93%, 94%, 95%, 96%, 97%, 98%, or 99% sequence identity to SEQ ID NO:20. The hMeso scFv may comprise an amino acid sequence with at least 80%, 85%, 90%, 91%, 92%, 93%, 94%, 95%, 96%, 97%, 98%, 99%, or 100% sequence identity to SEQ ID NO:18. The hMeso scFv may be encoded by a polynucleotide with at least 80%, 85%, 90%, 91%, 92%, 93%, 94%, 95%, 96%, 97%, 98%, 99%, or 100% sequence identity to SEQ ID NO:15. The V_{L} of the hMeso scFv may be encoded by a polynucleotide with at least 80%, 85%, 90%, 91%, 92%, 93%, 94%, 95%, 96%, 97%, 98%, 99%, or 100% sequence identity to SEQ ID NO:16. The V_{H} of the hMeso scFv may be encoded by a polynucleotide with at least 80%, 85%, 90%, 91%, 92%, 93%, 94%, 95%, 96%, 97%, 98%, 99%, or 100% sequence identity to SEQ ID NO:17.

In some embodiments, a humanized scFv for ROR1 is provided herein. The humanized ROR1 (hROR1) scFv may have a variable light chain sequence of SEQ ID NO:25 and a variable heavy chain of SEQ ID NO:26. In other aspects, the hROR1 scFv may comprise a V_{L} with at least 80%, 85%, 90%, 91%, 921%, 93%, 94%, 95%, 96%, 97%, 98%, or 99% sequence identity to SEQ ID NO:25 and/or a V_{H} with at least 80%, 85%, 90%, 91%, 92%, 93%, 94%, 95%, 96%, 97%, 98%, or 99% sequence identity to SEQ ID NO:26. The hROR1 scFv may comprise an amino acid sequence with at least 80%, 85%, 90%, 91%, 92%, 93%, 94%, 95%, 96%, 97%, 98%, 99%, or 100% sequence identity to SEQ ID NO:24. The hROR1 scFv may be encoded by a polynucleotide with at least 80%, 85%, 90%, 91%, 92%, 93%, 94%, 95%, 96%, 97%, 98%, 99%, or 100% sequence identity to SEQ ID NO:21. The V_{L} of the hROR1 scFv may be encoded by a polynucleotide with at least 80%, 85%, 90%, 91%, 92%, 93%, 94%, 95%, 96%, 97%, 98%, 99%, or 100% sequence identity to SEQ ID NO:22. The V_{H} of the **hROR1** scFv may be encoded by a polynucleotide with at least 80%, 85%, 90%, 91%, 92%, 93%, 94%, 95%, 96%, 97%, 98%, 99%, or 100% sequence identity to SEQ ID NO:23.

In some embodiments, a humanized scFv for CD5 is provided herein. The humanized CD5 (hCD5) scFv may have a variable light chain sequence of SEQ ID NO:31 and a variable heavy chain of SEQ ID NO:32. In other aspects, the hCD5 scFv may comprise a V_{L} with at least 80%, 85%, 90%, 91%, 921%, 93%, 94%, 95%, 96%, 97%, 98%, or 99% sequence identity to SEQ ID NO:31 and/or a V_{H} with at least 80%, 85%, 90%, 91%, 92%, 93%, 94%, 95%, 96%, 97%, 98%, or 99% sequence identity to SEQ ID NO:32. The hCD5 scFv may comprise an amino acid sequence with at least 80%, 85%, 90%, 91%, 92%, 93%, 94%, 95%, 96%, 97%, 98%, 99%, or 100% sequence identity to SEQ ID NO:30. The hCD5 scFv may be encoded by a polynucleotide with at least 80%, 85%, 90%, 91%, 92%, 93%, 94%, 95%, 96%, 97%, 98%, 99%, or 100% sequence identity to SEQ ID NO:27. The V_{L} of the hCD5 scFv may be encoded by a polynucleotide with at least 80%, 85%, 90%, 91%, 92%, 93%, 94%, 95%, 96%, 97%, 98%, 99%, or 100% sequence identity to SEQ ID NO:28. The V_{H} of the hCD5 scFv may be encoded by a polynucleotide with at least 80%, 85%, 90%, 91%, 92%, 93%, 94%, 95%, 96%, 97%, 98%, 99%, or 100% sequence identity to SEQ ID NO:29.

In some embodiments, a humanized scFv for CD99 is provided herein. The humanized CD99 (hCD99) scFv may have a variable light chain sequence of SEQ ID NO:63 and a variable heavy chain of SEQ ID NO:64. In other aspects, the hCD99 scFv may comprise a V_{L} with at least 80%, 85%, 90%, 91%, 921%, 93%, 94%, 95%, 96%, 97%, 98%, or 99% sequence identity to SEQ ID NO:63 and/or a V_{H} with at least 80%, 85%, 90%, 91%, 92%, 93%, 94%, 95%, 96%, 97%, 98%, or 99% sequence identity to SEQ ID NO:64. The hCD99 scFv may comprise an amino acid sequence with at least 80%, 85%, 90%, 91%, 92%, 93%, 94%, 95%, 96%, 97%, 98%, 99%, or 100% sequence identity to SEQ ID NO:61. The hCD99 scFv may be encoded by a polynucleotide with at least 80%, 85%, 90%, 91%, 92%, 93%, 94%, 95%, 96%, 97%, 98%, 99%, or 100% sequence identity to SEQ ID NO:61.

In some embodiments, a humanized scFv for CD99 is provided herein. The humanized CD99 (hCD99) scFv may have a variable light chain sequence of SEQ ID NO:59 and a variable heavy chain of SEQ ID NO:60. In other aspects, the hCD99 scFv may comprise a V_{L} with at least 80%, 85%, 90%, 91%, 921%, 93%, 94%, 95%, 96%, 97%, 98%, or 99% sequence identity to SEQ ID NO:59 and/or a V_{H} with at least 80%, 85%, 90%, 91%, 92%, 93%, 94%, 95%, 96%, 97%, 98%, or 99% sequence identity to SEQ ID NO:60. The hCD99 scFv may comprise an amino acid sequence with at least 80%, 85%, 90%, 91%, 92%, 93%, 94%, 95%, 96%, 97%, 98%, 99%, or 100% sequence identity to SEQ ID NO:58. The hCD99 scFv may be encoded by a polynucleotide with at least 80%, 85%, 90%, 91%, 92%, 93%, 94%, 95%, 96%, 97%, 98%, 99%, or 100% sequence identity to SEQ ID NO:57.

Among the antigens targeted by the genetically engineered antigen receptors are those expressed in the context of a disease, condition, or cell type to be targeted via the adoptive cell therapy. Among the diseases and conditions are proliferative, neoplastic, and malignant diseases and disorders, including cancers and tumors, including hematologic cancers, cancers of the immune system, such as lymphomas, leukemias, and/or myelomas, such as B, T, and myeloid leukemias, lymphomas, and multiple myelomas. In some embodiments, the antigen is selectively expressed or overexpressed on cells of the disease or condition, e.g., the tumor or pathogenic cells, as compared to normal or non-targeted cells or tissues, antigens associated with autoimmune or alloimmune disorders, or pathogen-specific antigens. In other embodiments, the antigen is expressed on normal cells and/or is expressed on the engineered cells.

Any suitable antigen may find use in the present method. Exemplary antigens include, but are not limited to, antigenic molecules from infectious agents, auto-/self-antigens, tumor-/cancer-associated antigens, and tumor neoantigens. In particular aspects, the antigens include NY-ESO, EGFRvIII, Muc-1, Her2, CA-125, WT-1, Mage-A3, Mage-A4, Mage-A10, TRAIL/DR4, and CEA.

Tumor-associated antigens may be derived from prostate, breast, colorectal, lung, pancreatic, renal, mesothelioma, ovarian, or melanoma cancers. Exemplary tumor-associated antigens or tumor cell-derived antigens include MAGE 1, 3, and MAGE 4; PRAME; BAGE; RAGE, Lage (also known as NY ESO 1); SAGE; and HAGE or GAGE. These non-limiting examples of tumor antigens are expressed in a wide range of tumor types such as melanoma, lung carcinoma, sarcoma, and bladder carcinoma. Prostate cancer tumor-associated antigens include, for example, prostate specific membrane antigen (PSMA), prostate-specific antigen (PSA), prostatic acid phosphates, NKX3.1, and six-transmembrane epithelial antigen of the prostate (STEAP).

Other tumor associated antigens include Plu-1, HASH-1, HasH-2, Cripto and Criptin. Additionally, a tumor antigen may be a self peptide hormone, such as whole length gonadotrophin hormone releasing hormone, a short 10 amino acid long peptide, useful in the treatment of many cancers.

Tumor antigens include tumor antigens derived from cancers that are characterized by tumor-associated antigen expression, such as HER-2/neu expression. Tumor-associated antigens of interest include lineage-specific tumor antigens such as the melanocyte-melanoma lineage antigens MART-1/Melan-A, gp100, gp75, mda-7, tyrosinase and tyrosinase-related protein. Illustrative tumor-associated antigens include, but are not limited to, tumor antigens derived from or comprising any one or more of, p53, Ras, c-Myc, cytoplasmic serine/threonine kinases (e.g., A-Raf, B-Raf, and C-Raf, cyclin-dependent kinases), MAGE-A1, MAGE-A2, MAGE-A3, MAGE-A4, MAGE-A6, MAGE-A10, MAGE-A12, MART-1, BAGE, DAM-6, -10, GAGE-1, -2, -8, GAGE-3, -4, -5, -6, -7B, NA88-A, MART-1, MC1R, Gp100, PSA, PSM, Tyrosinase, TRP-1, TRP-2, ART-4, CAMEL, CEA, Cyp-B, hTERT, hTRT, iCE, MUC1, MUC2, Phosphoinositide 3-kinases (PI3Ks), TRK receptors, PRAME, P15, RU1, RU2, SART-1, SART-3, Wilms' tumor antigen (WT1), AFP, -catenin/m, Caspase-8/m, CEA, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, BCR-ABL, interferon regulatory factor 4 (IRF4), ETV6/AML, LDLR/FUT, Pml/RAR, Tumor-associated calcium signal transducer 1 (TACSTD1) TACSTD2, receptor tyrosine kinases (e.g., Epidermal Growth Factor receptor (EGFR) (in particular, EGFRvIII), platelet derived growth factor receptor (PDGFR), vascular endothelial growth factor receptor (VEGFR), cytoplasmic tyrosine kinases (e.g., src-family, syk-ZAP70 family), integrin-linked kinase (ILK), signal transducers and activators of transcription STAT3, STATS, and STATE, hypoxia inducible factors *(e.g.,* HIF-1 and HIF-2), Nuclear Factor-Kappa B (NF-B), Notch receptors *(e.g.,* Notch1-4), c-Met, mammalian targets of rapamycin (mTOR), WNT, extracellular signal-regulated kinases (ERKs), and their regulatory subunits, PMSA, PR-3, MDM2, Mesothelin, renal cell carcinoma-5T4, SM22-alpha, carbonic anhydrases I (CAI) and IX (CAIX) (also known as G250), STEAD, TEL/AML1, GD2, proteinase3, hTERT, sarcoma translocation breakpoints, EphA2, ML-IAP, EpCAM, ERG (TMPRSS2 ETS fusion gene), NA17, PAX3, ALK, androgen receptor, cyclin B1, polysialic acid, MYCN, RhoC, GD3, fucosyl GM1, mesothelian, PSCA, sLe, PLAC1, GM3, BORIS, Tn, GLoboH, NY-BR-1, RGsS, SART3, STn, PAX5, OY-TES1, sperm protein 17, LCK, HMWMAA, AKAP-4, SSX2, XAGE 1, B7H3, legumain, TIE2, Page4, MAD-CT-1, FAP, MAD-CT-2, fos related antigen 1, CBX2, CLDN6, SPANX, TPTE, ACTL8, ANKRD30A, CDKN2A, MAD2L1, CTAG1B, SUNC1, LRRN1 and idiotype.

Antigens may include epitopic regions or epitopic peptides derived from genes mutated in tumor cells or from genes transcribed at different levels in tumor cells compared to normal cells, such as telomerase enzyme, survivin, mesothelin, mutated ras, bcr/abl rearrangement, Her2/neu, mutated or wild-type p53, cytochrome P450 1B1, and abnormally expressed intron sequences such as N-acetylglucosaminyltransferase-V; clonal rearrangements of immunoglobulin genes generating unique idiotypes in myeloma and B-cell lymphomas; tumor antigens that include epitopic regions or epitopic peptides derived from oncoviral processes, such as human papilloma virus proteins E6 and E7; Epstein bar virus protein LMP2; nonmutated oncofetal proteins with a tumor-selective expression, such as carcinoembryonic antigen and alpha-fetoprotein.

In other embodiments, an antigen is obtained or derived from a pathogenic microorganism or from an opportunistic pathogenic microorganism (also called herein an infectious disease microorganism), such as a virus, fungus, parasite, and bacterium. In certain embodiments, antigens derived from such a microorganism include full-length proteins.

Illustrative pathogenic organisms whose antigens are contemplated for use in the method described herein include human immunodeficiency virus (HIV), herpes simplex virus (HSV), respiratory syncytial virus (RSV), cytomegalovirus (CMV), Epstein-Barr virus (EBV), Influenza A, B, and C, vesicular stomatitis virus (VSV), vesicular stomatitis virus (VSV), polyomavirus *(e.g.,* BK virus and JC virus), adenovirus, *Staphylococcus* species including Methicillin-resistant *Staphylococcus aureus* (MRSA), and *Streptococcus* species including *Streptococcus pneumoniae.* As would be understood by the skilled person, proteins derived from these and other pathogenic microorganisms for use as antigen as described herein and nucleotide sequences encoding the proteins may be identified in publications and in public databases such as GENBANK^{®}, Swiss-Prot^{®}, and TrEMBL^{®}.

Antigens derived from human immunodeficiency virus (HIV) include any of the HIV virion structural proteins *(e.g.,* gp120, gp41, p17, p24), protease, reverse transcriptase, or HIV proteins encoded by tat, rev, nef, vif, vpr and vpu.

Antigens derived from herpes simplex virus (e.g., HSV 1 and HSV2) include, but are not limited to, proteins expressed from HSV late genes. The late group of genes predominantly encodes proteins that form the virion particle. Such proteins include the five proteins from (UL) which form the viral capsid: UL6, UL18, UL35, UL38 and the major capsid protein UL19, UL45, and UL27, each of which may be used as an antigen as described herein. Other illustrative HSV proteins contemplated for use as antigens herein include the ICP27 (H1, H2), glycoprotein B (gB) and glycoprotein D (gD) proteins. The HSV genome comprises at least 74 genes, each encoding a protein that could potentially be used as an antigen.

Antigens derived from cytomegalovirus (CMV) include CMV structural proteins, viral antigens expressed during the immediate early and early phases of virus replication, glycoproteins I and III, capsid protein, coat protein, lower matrix protein pp65 (ppUL83), p52 (ppUL44), IE1 and 1E2 (UL123 and UL122), protein products from the cluster of genes from UL128-UL150 (Rykman, *et al.,* 2006), envelope glycoprotein B (gB), gH, gN, and pp150. As would be understood by the skilled person, CMV proteins for use as antigens described herein may be identified in public databases such as GenBank^{®}, Swiss-Prot^{®}, and TrEMBL^{®}.

Antigens derived from Epstein-Ban virus (EBV) that are contemplated for use in certain embodiments include EBV lytic proteins gp350 and gp110, EBV proteins produced during latent cycle infection including Epstein-Ban nuclear antigen (EBNA)-1, EBNA-2, EBNA-3A, EBNA-3B, EBNA-3C, EBNA-leader protein (EBNA-LP) and latent membrane proteins (LMP)-1, LMP-2A and LMP-2B.

Antigens derived from respiratory syncytial virus (RSV) that are contemplated for use herein include any of the eleven proteins encoded by the RSV genome, or antigenic fragments thereof: NS 1, NS2, N (nucleocapsid protein), M (Matrix protein) SH, G and F (viral coat proteins), M2 (second matrix protein), M2-1 (elongation factor), M2-2 (transcription regulation), RNA polymerase, and phosphoprotein P.

Antigens derived from Vesicular stomatitis virus (VSV) that are contemplated for use include any one of the five major proteins encoded by the VSV genome, and antigenic fragments thereof: large protein (L), glycoprotein (G), nucleoprotein (N), phosphoprotein (P), and matrix protein (M).

Antigens derived from an influenza virus that are contemplated for use in certain embodiments include hemagglutinin (HA), neuraminidase (NA), nucleoprotein (NP), matrix proteins M1 and M2, NS1, NS2 (NEP), PA, PB1, PB1-F2, and PB2.

Exemplary viral antigens also include, but are not limited to, adenovirus polypeptides, alphavirus polypeptides, calicivirus polypeptides *(e.g.,* a calicivirus capsid antigen), coronavirus polypeptides, distemper virus polypeptides, Ebola virus polypeptides, enterovirus polypeptides, flavivirus polypeptides, hepatitis virus (AE) polypeptides (a hepatitis B core or surface antigen, a hepatitis C virus E1 or E2 glycoproteins, core, or non-structural proteins), herpesvirus polypeptides (including a herpes simplex virus or varicella zoster virus glycoprotein), infectious peritonitis virus polypeptides, leukemia virus polypeptides, Marburg virus polypeptides, orthomyxovirus polypeptides, papilloma virus polypeptides, parainfluenza virus polypeptides *(e.g.,* the hemagglutinin and neuraminidase polypeptides), paramyxovirus polypeptides, parvovirus polypeptides, pestivirus polypeptides, picorna virus polypeptides *(e.g.,* a poliovirus capsid polypeptide), pox virus polypeptides *(e.g.,* a vaccinia virus polypeptide), rabies virus polypeptides *(e.g.,* a rabies virus glycoprotein G), reovirus polypeptides, retrovirus polypeptides, and rotavirus polypeptides.

In certain embodiments, the antigen may be bacterial antigens. In certain embodiments, a bacterial antigen of interest may be a secreted polypeptide. In other certain embodiments, bacterial antigens include antigens that have a portion or portions of the polypeptide exposed on the outer cell surface of the bacteria.

Antigens derived from *Staphylococcus* species including Methicillin-resistant *Staphylococcus aureus* (MRSA) that are contemplated for use include virulence regulators, such as the Agr system, Sar and Sae, the Arl system, Sar homologues (Rot, MgrA, SarS, SarR, SarT, SarU, SarV, SarX, SarZ and TcaR), the Srr system and TRAP. Other *Staphylococcus* proteins that may serve as antigens include Clp proteins, HtrA, MsrR, aconitase, CcpA, SvrA, Msa, CfvA and CfvB (see, *e.g.,* Staphylococcus: Molecular Genetics, 2008 Caister Academic Press, Ed. Jodi Lindsay). The genomes for two species of *Staphylococcus aureus* (N315 and Mu50) have been sequenced and are publicly available, for example at PATRIC (PATRIC: The VBI PathoSystems Resource Integration Center, Snyder et al., 2007). As would be understood by the skilled person, *Staphylococcus* proteins for use as antigens may also be identified in other public databases such as GenBank^{®}, Swiss-Prot^{®}, and TrEMBL^{®}.

Antigens derived from *Streptococcus pneumoniae* that are contemplated for use in certain embodiments described herein include pneumolysin, PspA, choline-binding protein A (CbpA), NanA, NanB, SpnHL, PavA, LytA, Pht, and pilin proteins (RrgA; RrgB; RrgC). Antigenic proteins of *Streptococcus pneumoniae* are also known in the art and may be used as an antigen in some embodiments. The complete genome sequence of a virulent strain of *Streptococcus pneumoniae* has been sequenced and, as would be understood by the skilled person, *S. pneumoniae* proteins for use herein may also be identified in other public databases such as GenBank^{®}, Swiss-Prot^{®}, and TrEMBL^{®}. Proteins of particular interest for antigens according to the present disclosure include virulence factors and proteins predicted to be exposed at the surface of the pneumococci.

Examples of bacterial antigens that may be used as antigens include, but are not limited to, *Actinomyces* polypeptides, *Bacillus* polypeptides, *Bacteroides* polypeptides, *Bordetella* polypeptides, *Bartonella* polypeptides, *Borrelia* polypeptides *(e.g., B. burgdorferi* OspA), *Brucella* polypeptides, *Campylobacter* polypeptides, Capnocytophaga polypeptides, *Chlamydia* polypeptides, *Corynebacterium* polypeptides, *Coxiella* polypeptides, *Dermatophilus* polypeptides, *Enterococcus* polypeptides, *Ehrlichia* polypeptides, *Escherichia* polypeptides, *Francisella* polypeptides, *Fusobacterium* polypeptides, *Haemobartonella* polypeptides, *Haemophilus* polypeptides *(e.g., H. influenzae* type b outer membrane protein), *Helicobacter* polypeptides, *Klebsiella* polypeptides, L-form bacteria polypeptides, *Leptospira* polypeptides, *Listeria* polypeptides, *Mycobacteria* polypeptides, *Mycoplasma* polypeptides, *Neisseria* polypeptides, *Neorickettsia* polypeptides, *Nocardia* polypeptides, *Pasteurella* polypeptides, *Peptococcus* polypeptides, *Peptostreptococcus* polypeptides, *Pneumococcus* polypeptides (i.e., *S. pneumoniae* polypeptides) (see description herein), *Proteus* polypeptides, *Pseudomonas* polypeptides, *Rickettsia* polypeptides, *Rochalimaea* polypeptides, *Salmonella* polypeptides, *Shigella* polypeptides, *Staphylococcus* polypeptides, group A *streptococcus* polypeptides *(e.g., S. pyogenes* M proteins), group B *streptococcus (S. agalactiae)* polypeptides, *Treponema* polypeptides, and *Yersinia* polypeptides *(e.g., Y pestis* F1 and V antigens).

Examples of fungal antigens include, but are not limited to, *Absidia* polypeptides, *Acremonium* polypeptides, *Alternaria* polypeptides, *Aspergillus* polypeptides, *Basidiobolus* polypeptides, *Bipolaris* polypeptides, *Blastomyces* polypeptides, *Candida* polypeptides, *Coccidioides* polypeptides, *Conidiobolus* polypeptides, *Cryptococcus* polypeptides, *Curvalaria* polypeptides, *Epidermophyton* polypeptides, *Exophiala* polypeptides, *Geotrichum* polypeptides, *Histoplasma* polypeptides, *Madurella* polypeptides, *Malassezia* polypeptides, *Microsporum* polypeptides, *Moniliella* polypeptides, *Mortierella* polypeptides, *Mucor* polypeptides, *Paecilomyces* polypeptides, *Penicillium* polypeptides, *Phialemonium* polypeptides, *Phialophora* polypeptides, *Prototheca* polypeptides, *Pseudallescheria* polypeptides, *Pseudomicrodochium* polypeptides, *Pythium* polypeptides, *Rhinosporidium* polypeptides, *Rhizopus* polypeptides, *Scolecobasidium* polypeptides, *Sporothrix* polypeptides, *Stemphylium* polypeptides, *Trichophyton* polypeptides, *Trichosporon* polypeptides, and *Xylohypha* polypeptides.

Examples of protozoan parasite antigens include, but are not limited to, *Babesia* polypeptides, *Balantidium* polypeptides, *Besnoitia* polypeptides, *Cryptosporidium* polypeptides, *Eimeria* polypeptides, *Encephalitozoon* polypeptides, *Entamoeba* polypeptides, *Giardia* polypeptides, *Hammondia* polypeptides, *Hepatozoon* polypeptides, *Isospora* polypeptides, *Leishmania* polypeptides, *Microsporidia* polypeptides, *Neospora* polypeptides, *Nosema* polypeptides, *Pentatrichomonas* polypeptides, *Plasmodium* polypeptides. Examples of helminth parasite antigens include, but are not limited to, *Acanthocheilonema* polypeptides, *Aelurostrongylus* polypeptides, *Ancylostoma* polypeptides, *Angiostrongylus* polypeptides, *Ascaris* polypeptides, *Brugia* polypeptides, *Bunostomum* polypeptides, *Capillaria* polypeptides, *Chabertia* polypeptides, *Cooperia* polypeptides, *Crenosoma* polypeptides, *Dictyocaulus* polypeptides, *Dioctophyme* polypeptides, *Dipetalonema* polypeptides, *Diphyllobothrium* polypeptides, *Diplydium* polypeptides, *Dirofilaria* polypeptides, *Dracunculus* polypeptides, *Enterobius* polypeptides, *Filaroides* polypeptides, *Haemonchus* polypeptides, *Lagochilascaris* polypeptides, *Loa* polypeptides, *Mansonella* polypeptides, *Muellerius* polypeptides, *Nanophyetus* polypeptides, *Necator* polypeptides, *Nematodirus* polypeptides, *Oesophagostomum* polypeptides, *Onchocerca* polypeptides, *Opisthorchis* polypeptides, *Ostertagia* polypeptides, *Parafilaria* polypeptides, *Paragonimus* polypeptides, *Parascaris* polypeptides, *Physaloptera* polypeptides, *Protostrongylus* polypeptides, *Setaria* polypeptides, *Spirocerca* polypeptides *Spirometra* polypeptides, *Stephanofilaria* polypeptides, *Strongyloides* polypeptides, *Strongylus* polypeptides, *Thelazia* polypeptides, *Toxascaris* polypeptides, *Toxocara* polypeptides, *Trichinella* polypeptides, *Trichostrongylus* polypeptides, *Trichuris* polypeptides, *Uncinaria* polypeptides, and *Wuchereria* polypeptides. *(e.g., P. falciparum* circumsporozoite (PfCSP)), sporozoite surface protein 2 (PfSSP2), carboxyl terminus of liver state antigen 1 (PfLSA1 c-term), and exported protein 1 (PfExp-1), *Pneumocystis* polypeptides, *Sarcocystis* polypeptides, *Schistosoma* polypeptides, *Theileria* polypeptides, *Toxoplasma* polypeptides, and *Trypanosoma* polypeptides.

Examples of ectoparasite antigens include, but are not limited to, polypeptides (including antigens as well as allergens) from fleas; ticks, including hard ticks and soft ticks; flies, such as midges, mosquitoes, sand flies, black flies, horse flies, horn flies, deer flies, tsetse flies, stable flies, myiasis-causing flies and biting gnats; ants; spiders, lice; mites; and true bugs, such as bed bugs and kissing bugs.

### C. Chimeric Antigen Receptors

**In** some embodiments, the CAR contains an extracellular antigen-recognition domain that specifically binds to an antigen. In some embodiments, the antigen is a protein expressed on the surface of cells. In some embodiments, the CAR is a TCR-like CAR and the antigen is a processed peptide antigen, such as a peptide antigen of an intracellular protein, which, like a TCR, is recognized on the cell surface in the context of a major histocompatibility complex (MHC) molecule.

In some embodiments, the chimeric antigen receptor comprises: a) an intracellular signaling domain, b) a hinge and transmembrane domain, such as a Ev3 hinge, and c) an extracellular domain comprising an antigen binding region.

In some embodiments, the engineered antigen receptors include chimeric antigen receptors (CARs), including activating or stimulatory CARs, costimulatory CARs (see WO2014/055668), and/or inhibitory CARs (iCARs, see Fedorov et al., 2013). The CARs generally include an extracellular antigen (or ligand) binding domain linked to one or more intracellular signaling components, in some aspects via linkers and/or transmembrane domain(s). Such molecules typically mimic or approximate a signal through a natural antigen receptor, a signal through such a receptor in combination with a costimulatory receptor, and/or a signal through a costimulatory receptor alone.

Certain embodiments of the present disclosure concern the use of nucleic acids, including nucleic acids encoding an antigen-specific CAR polypeptide, including a CAR that has been humanized to reduce immunogenicity (hCAR), comprising an intracellular signaling domain, a transmembrane domain, and an extracellular domain comprising one or more signaling motifs. In certain embodiments, the CAR may recognize an epitope comprising the shared space between one or more antigens. In certain embodiments, the binding region can comprise complementary determining regions of a monoclonal antibody, variable regions of a monoclonal antibody, and/or antigen binding fragments thereof. In another embodiment, that specificity is derived from a peptide (e.g., cytokine) that binds to a receptor.

It is contemplated that the human CAR nucleic acids may be human genes used to enhance cellular immunotherapy for human patients. In a specific embodiment, the invention includes a full-length CAR cDNA or coding region. The antigen binding regions or domain can comprise a fragment of the V_{H} and V_{L} chains of a single-chain variable fragment (scFv) derived from a particular human monoclonal antibody, such as those described in U.S. Patent 7,109,304, incorporated herein by reference. The fragment can also be any number of different antigen binding domains of a human antigen-specific antibody. In a more specific embodiment, the fragment is an antigen-specific scFv encoded by a sequence that is optimized for human codon usage for expression in human cells.

The arrangement could be multimeric, such as a diabody or multimers. The multimers are most likely formed by cross pairing of the variable portion of the light and heavy chains into a diabody. The hinge portion of the construct can have multiple alternatives from being totally deleted, to having the first cysteine maintained, to a proline rather than a serine substitution, to being truncated up to the first cysteine. The Fc portion can be deleted. Any protein that is stable and/or dimerizes can serve this purpose. One could use just one of the Fc domains, e.g., either the CH2 or CH3 domain from human immunoglobulin. One could also use the hinge, CH2 and CH3 region of a human immunoglobulin that has been modified to improve dimerization. One could also use just the hinge portion of an immunoglobulin. One could also use portions of CD8alpha.

In some embodiments, the CAR nucleic acid comprises a sequence encoding other costimulatory receptors, such as a transmembrane domain and a modified CD28 intracellular signaling domain. Other costimulatory receptors include, but are not limited to one or more of CD28, CD27, OX-40 (CD134), DAP10, DAP12, and 4-1BB (CD137). In addition to a primary signal initiated by CD3ζ, an additional signal provided by a human costimulatory receptor inserted in a human CAR is important for full activation of NK cells and could help improve *in vivo* persistence and the therapeutic success of the adoptive immunotherapy. The CAR may express one or more cytokines, such as IL-15. The inclusion of one or more cytokines may promote *in vivo* persistence. In some aspects, the CAR comprises the antigen binding domain, such as humanized scFv, CD3ζ, DAP12, and IL-15, optionally in combination with the Ev3 hinge.

In some embodiments, CAR is constructed with a specificity for a particular antigen (or marker or ligand), such as an antigen expressed in a particular cell type to be targeted by adoptive therapy, *e.g.,* a cancer marker, and/or an antigen intended to induce a dampening response, such as an antigen expressed on a normal or non-diseased cell type. Thus, the CAR typically includes in its extracellular portion one or more antigen binding molecules, such as one or more antigen-binding fragment, domain, or portion, or one or more antibody variable domains, and/or antibody molecules. In some embodiments, the CAR includes an antigen-binding portion or portions of an antibody molecule, such as a single-chain antibody fragment (scFv) derived from the variable heavy (V_{H}) and variable light (V_{L}) chains of a monoclonal antibody (mAb).

In certain embodiments of the chimeric antigen receptor, the antigen-specific portion of the receptor (which may be referred to as an extracellular domain comprising an antigen binding region) comprises a tumor associated antigen or a pathogen-specific antigen binding domain. Antigens include carbohydrate antigens recognized by pattern-recognition receptors, such as Dectin-1. A tumor associated antigen may be of any kind so long as it is expressed on the cell surface of tumor cells. Exemplary embodiments of tumor associated antigens include CD19, CD319 (CS1), CD20, carcinoembryonic antigen, alphafetoprotein, CA-125, MUC-1, CD56, EGFR, c-Met, AKT, Her2, Her3, epithelial tumor antigen, melanoma-associated antigen, mutated p53, mutated ras, and so forth. In certain embodiments, the CAR may be co-expressed with a cytokine to improve persistence when there is a low amount of tumor-associated antigen. For example, CAR may be co-expressed with IL-15.

The sequence of the open reading frame encoding the chimeric receptor can be obtained from a genomic DNA source, a cDNA source, or can be synthesized *(e.g., via* PCR), or combinations thereof. Depending upon the size of the genomic DNA and the number of introns, it may be desirable to use cDNA or a combination thereof as it is found that introns stabilize the mRNA. Also, it may be further advantageous to use endogenous or exogenous non-coding regions to stabilize the mRNA.

It is contemplated that the chimeric construct can be introduced into immune cells as naked DNA or in a suitable vector. Methods of stably transfecting cells by electroporation using naked DNA are known in the art. Naked DNA generally refers to the DNA encoding a chimeric receptor contained in a plasmid expression vector in proper orientation for expression.

Alternatively, a viral vector *(e.g.,* a retroviral vector, adenoviral vector, adeno-associated viral vector, or lentiviral vector) can be used to introduce the chimeric construct into immune cells. Suitable vectors for use in accordance with the method of the present disclosure are non-replicating in the immune cells. A large number of vectors are known that are based on viruses, where the copy number of the virus maintained in the cell is low enough to maintain the viability of the cell, such as, for example, vectors based on HIV, SV40, EBV, HSV, or BPV.

In some aspects, the antigen-specific binding, or recognition component is linked to one or more transmembrane and intracellular signaling domains. In some embodiments, the CAR includes a transmembrane domain fused to the extracellular domain of the CAR. In one embodiment, the transmembrane domain that naturally is associated with one of the domains in the CAR is used. In some instances, the transmembrane domain is selected or modified by amino acid substitution to avoid binding of such domains to the transmembrane domains of the same or different surface membrane proteins to minimize interactions with other members of the receptor complex.

The transmembrane domain in some embodiments is derived either from a natural or from a synthetic source. Where the source is natural, the domain in some aspects is derived from any membrane-bound or transmembrane protein. Transmembrane regions include those derived from (*i.e.* comprise at least the transmembrane region(s) of) the alpha, beta or zeta chain of the T- cell receptor, CD28, CD3 zeta, CD3 epsilon, CD3 gamma, CD3 delta, CD45, CD4, CD5, CD8, CD9, CD 16, CD22, CD33, CD37, CD64, CD80, CD86, CD 134, CD137, CD154, ICOS/CD278, GITR/CD357, NKG2D, and DAP molecules. Alternatively the transmembrane domain in some embodiments is synthetic. In some aspects, the synthetic transmembrane domain comprises predominantly hydrophobic residues such as leucine and valine. In some aspects, a triplet of phenylalanine, tryptophan and valine will be found at each end of a synthetic transmembrane domain.

In certain embodiments, the platform technologies disclosed herein to genetically modify immune cells, such as NK cells, comprise (i) non-viral gene transfer using an electroporation device *(e.g.,* a nucleofector), (ii) CARs that signal through endodomains *(e.g.,* CD28/CD3-ζ, CD137/CD3-ζ, or other combinations), (iii) CARs with variable lengths of extracellular domains connecting the antigen-recognition domain to the cell surface, and, in some cases, (iv) artificial antigen presenting cells (aAPC) derived from K562 to be able to robustly and numerically expand CAR⁺ immune cells (Singh *et al.,* 2008; Singh *et al.,* 2011).

In some embodiments, the CAR may be retrovirally tranduced in the cells. The retroviral vector may be the pSFG4 vector. The retroviral transfer vector pSFG4 comprises a backbone based on pUC19 plasmid (large fragment [2.63kb] in between HindIII and EcoRI restriction enzyme sites) carrying viral components from Moloney Murine Leukemia Virus (MoMLV) including 5' LTR, psi packaging sequence, and 3' LTR. LTRs are long terminal repeats found on either side of a retroviral provirus, and in the case of transfer vector, brackets the genetic cargo of interest (in our case mainly CARs and associated genetic components). The psi packaging sequence, which is a target site for packaging by nucleocapsid, is also incorporated in cis, sandwiched between the 5' LTR and the CAR coding sequence. Thus, the basic structure of the pSFG4 transfer vector can be outlined as such: pUC19 sequence - 5' LTR - psi packaging sequence - genetic cargo of interest - 3' LTR - pUC19 sequence.

### D. Antigen-Presenting Cells

Antigen-presenting cells, which include macrophages, B lymphocytes, and dendritic cells, are distinguished by their expression of a particular MHC molecule. APCs internalize antigen and re-express a part of that antigen, together with the MHC molecule on their outer cell membrane. The major histocompatibility complex (MHC) is a large genetic complex with multiple loci. The MHC loci encode two major classes of MHC membrane molecules, referred to as class I and class II MHCs. T helper lymphocytes generally recognize antigen associated with MHC class II molecules, and T cytotoxic lymphocytes recognize antigen associated with MHC class I molecules. In humans the MHC is referred to as the HLA complex and in mice the H-2 complex.

aAPC systems may comprise at least one exogenous assisting molecule. Any suitable number and combination of assisting molecules may be employed. The assisting molecule may be selected from assisting molecules such as co-stimulatory molecules and adhesion molecules. Exemplary co-stimulatory molecules include CD86, CD64 (FcyRI), 41BB ligand, and IL-21. Adhesion molecules may include carbohydrate-binding glycoproteins such as selectins, transmembrane binding glycoproteins such as integrins, calcium-dependent proteins such as cadherins, and single-pass transmembrane immunoglobulin (Ig) superfamily proteins, such as intercellular adhesion molecules (ICAMs), which promote, for example, cell-to-cell or cell-to-matrix contact. Exemplary adhesion molecules include LFA-3 and ICAMs, such as ICAM-1. Techniques, methods, and reagents useful for selection, cloning, preparation, and expression of exemplary assisting molecules, including co-stimulatory molecules and adhesion molecules.

### IV. Methods of Use

In some embodiments, the present disclosure provides methods for immunotherapy comprising administering an effective amount of the immune cells expressing CARs *(e.g.,* CARs with Ev3 hinge and/or humanized scFv) of the present disclosure. In one embodiments, a medical disease or disorder is treated by transfer of an immune cell population that elicits an immune response. In certain embodiments of the present disclosure, cancer or infection is treated by transfer of an immune cell population that elicits an immune response. Provided herein are methods for treating or delaying progression of cancer in an individual comprising administering to the individual an effective amount an antigen-specific cell therapy. The present methods may be applied for the treatment of immune disorders, solid cancers, hematologic cancers, and viral infections.

In particular embodiments, methods are provided for treating cancer patients by administering immune cells, such as NK cells and/or T cells, expressing CARs provided herein with an antigen-binding domain specific for the antigen expressed by said cancer. For example, a subject with chronic lymphocytic leukemia (CLL), B cell acute lymphoblastic leukemia (ALL), or non-Hodgkin lymphoma (NHL) may be treated with CD19-Ev3-CAR immune cells to ablate CD19-positive cells such as cancer stem cells. In other aspects, the subject may have acute myeloid leukemia (AML), chronic myeloid leukemia (CML), myelodysplastic syndromes (MDS), or plasmacytoid dendritic cell leukemia (PDCL) and be treated with CD123-Ev3-CAR immune cells. In additional aspects, the subject may have T-cell leukemia and lymphoma and be treated with CD5-Ev3-CAR immune cells. Subjects with CLL, breast cancer, pancreatic cancer or lung cancer may be treated with ROR1-Ev3-CAR immune cells. Patients with cervical, endometrial or ovarian cancer may be treated with Meso-Ev3-CAR immune cells.

Subjects with multiple myeloma (MM) or other CD319-expressig cancers may be treated with CD319-CAR immune cells. In some aspects, patient with amyloidosis are treated by ablating CD319-positive cells, such as cancer stem cells. In some aspects, patients with monoclonal gammopathy of unknown significance (MGUS) or smoldering myeloma are treated with CD319-CAR immune cells. Subjects with CD319-positive cells in autoimmune disorders, such as rheumatoid arthritis, SLE, or autoimmune hemolytic anemia may be treated by immune cells of the present embodiments. In particular aspects, a CD319-expressing cancer is treated by administering immune cells expressing Ev3-CAR with CD319 scFv, CD3ζ, DAP12, and IL-15.

In some embodiments, cells comprising the Ev3 CARs provided herein may be ablated and/or detected by anti-EGFR antibodies. Exemplary anti-EGFR antibodies include, but are not limited to, cetuximab (Erbitux) and panitumumab (Vectibix) as well as biosimilars. Both of these antibodies have been approved by the FDA for treatment of colorectal cancer. In some embodiments, the detection of cells expressing Ev3-CARs comprises the administration of an anti-EGFR antibody with a detectable tag. In other aspects, the ablation of cells expressing Ev3-CARs comprises the administration of an anti-EGFR antibody which induces ADCC. In some aspects, the anti-EGFR antibody may be fused to a pro-drug or cytotoxic agent, such as a toxin.

Tumors for which the present treatment methods are useful include any malignant cell type, such as those found in a solid tumor or a hematological tumor. Exemplary solid tumors can include, but are not limited to, a tumor of an organ selected from the group consisting of pancreas, colon, cecum, stomach, brain, head, neck, ovary, kidney, larynx, sarcoma, lung, bladder, melanoma, prostate, and breast. Exemplary hematological tumors include tumors of the bone marrow, T or B cell malignancies, leukemias, lymphomas, blastomas, myelomas, and the like. Further examples of cancers that may be treated using the methods provided herein include, but are not limited to, lung cancer (including small-cell lung cancer, non-small cell lung cancer, adenocarcinoma of the lung, and squamous carcinoma of the lung), cancer of the peritoneum, gastric or stomach cancer (including gastrointestinal cancer and gastrointestinal stromal cancer), pancreatic cancer, cervical cancer, ovarian cancer, liver cancer, bladder cancer, breast cancer, colon cancer, colorectal cancer, endometrial or uterine carcinoma, salivary gland carcinoma, kidney or renal cancer, prostate cancer, vulval cancer, thyroid cancer, various types of head and neck cancer, and melanoma.

The cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; lentigo malignant melanoma; acral lentiginous melanomas; nodular melanomas; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphomas; B-cell lymphoma; low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; Waldenstrom's macroglobulinemia; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; hairy cell leukemia; chronic lymphocytic leukemia (CLL); acute lymphoblastic leukemia (ALL); acute myeloid leukemia (AML); and chronic myeloblastic leukemia.

Particular embodiments concern methods of treatment of leukemia. Leukemia is a cancer of the blood or bone marrow and is characterized by an abnormal proliferation (production by multiplication) of blood cells, usually white blood cells (leukocytes). It is part of the broad group of diseases called hematological neoplasms. Leukemia is a broad term covering a spectrum of diseases. Leukemia is clinically and pathologically split into its acute and chronic forms.

In certain embodiments of the present disclosure, immune cells are delivered to an individual in need thereof, such as an individual that has cancer or an infection. The cells then enhance the individual's immune system to attack the respective cancer or pathogenic cells. In some cases, the individual is provided with one or more doses of the immune cells. In cases where the individual is provided with two or more doses of the immune cells, the duration between the administrations should be sufficient to allow time for propagation in the individual, and in specific embodiments the duration between doses is 1, 2, 3, 4, 5, 6, 7, or more days.

Certain embodiments of the present disclosure provide methods for treating or preventing an immune-mediated disorder. In one embodiment, the subject has an autoimmune disease. Non-limiting examples of autoimmune diseases include: alopecia areata, ankylosing spondylitis, antiphospholipid syndrome, autoimmune Addison's disease, autoimmune diseases of the adrenal gland, autoimmune hemolytic anemia, autoimmune hepatitis, autoimmune oophoritis and orchitis, autoimmune thrombocytopenia, Behcet's disease, bullous pemphigoid, cardiomyopathy, celiac spate-dermatitis, chronic fatigue immune dysfunction syndrome (CFIDS), chronic inflammatory demyelinating polyneuropathy, Churg-Strauss syndrome, cicatrical pemphigoid, CREST syndrome, cold agglutinin disease, Crohn's disease, discoid lupus, essential mixed cryoglobulinemia, fibromyalgia-fibromyositis, glomerulonephritis, Graves' disease, Guillain-Barre, Hashimoto's thyroiditis, idiopathic pulmonary fibrosis, idiopathic thrombocytopenia purpura (ITP), IgA neuropathy, juvenile arthritis, lichen planus, lupus erthematosus, Meniere's disease, mixed connective tissue disease, multiple sclerosis, type 1 or immune-mediated diabetes mellitus, myasthenia gravis, nephrotic syndrome (such as minimal change disease, focal glomerulosclerosis, or mebranous nephropathy), pemphigus vulgaris, pernicious anemia, polyarteritis nodosa, polychondritis, polyglandular syndromes, polymyalgia rheumatica, polymyositis and dermatomyositis, primary agammaglobulinemia, primary biliary cirrhosis, psoriasis, psoriatic arthritis, Raynaud's phenomenon, Reiter's syndrome, Rheumatoid arthritis, sarcoidosis, scleroderma, Sjogren's syndrome, stiff-man syndrome, systemic lupus erythematosus, lupus erythematosus, ulcerative colitis, uveitis, vasculitides (such as polyarteritis nodosa, takayasu arteritis, temporal arteritis/giant cell arteritis, or dermatitis herpetiformis vasculitis), vitiligo, and Wegener's granulomatosis. Thus, some examples of an autoimmune disease that can be treated using the methods disclosed herein include, but are not limited to, multiple sclerosis, rheumatoid arthritis, systemic lupus erythematosis, type I diabetes mellitus, Crohn's disease; ulcerative colitis, myasthenia gravis, glomerulonephritis, ankylosing spondylitis, vasculitis, or psoriasis. The subject can also have an allergic disorder such as Asthma.

In yet another embodiment, the subject is the recipient of a transplanted organ or stem cells and immune cells are used to prevent and/or treat rejection. In particular embodiments, the subject has or is at risk of developing graft versus host disease. GVHD is a possible complication of any transplant that uses or contains stem cells from either a related or an unrelated donor. There are two kinds of GVHD, acute and chronic. Acute GVHD appears within the first three months following transplantation. Signs of acute GVHD include a reddish skin rash on the hands and feet that may spread and become more severe, with peeling or blistering skin. Acute GVHD can also affect the stomach and intestines, in which case cramping, nausea, and diarrhea are present. Yellowing of the skin and eyes (jaundice) indicates that acute GVHD has affected the liver. Chronic GVHD is ranked based on its severity: stage/grade 1 is mild; stage/grade 4 is severe. Chronic GVHD develops three months or later following transplantation. The symptoms of chronic GVHD are similar to those of acute GVHD, but in addition, chronic GVHD may also affect the mucous glands in the eyes, salivary glands in the mouth, and glands that lubricate the stomach lining and intestines. Any of the populations of immune cells disclosed herein can be utilized. Examples of a transplanted organ include a solid organ transplant, such as kidney, liver, skin, pancreas, lung and/or heart, or a cellular transplant such as islets, hepatocytes, myoblasts, bone marrow, or hematopoietic or other stem cells. The transplant can be a composite transplant, such as tissues of the face. Immune cells can be administered prior to transplantation, concurrently with transplantation, or following transplantation. In some embodiments, the immune cells are administered prior to the transplant, such as at least 1 hour, at least 12 hours, at least 1 day, at least 2 days, at least 3 days, at least 4 days, at least 5 days, at least 6 days, at least 1 week, at least 2 weeks, at least 3 weeks, at least 4 weeks, or at least 1 month prior to the transplant. In one specific, non-limiting example, administration of the therapeutically effective amount of immune cells occurs 3-5 days prior to transplantation.

In some embodiments, the subject can be administered nonmyeloablative lymphodepleting chemotherapy prior to the immune cell therapy. The nonmyeloablative lymphodepleting chemotherapy can be any suitable such therapy, which can be administered by any suitable route. The nonmyeloablative lymphodepleting chemotherapy can comprise, for example, the administration of cyclophosphamide and fludarabine, particularly if the cancer is melanoma, which can be metastatic. An exemplary route of administering cyclophosphamide and fludarabine is intravenously. Likewise, any suitable dose of cyclophosphamide and fludarabine can be administered. In particular aspects, around 60 mg/kg of cyclophosphamide is administered for two days after which around 25 mg/m² fludarabine is administered for five days.

In certain embodiments, a growth factor that promotes the growth and activation of the immune cells is administered to the subject either concomitantly with the immune cells or subsequently to the immune cells. The immune cell growth factor can be any suitable growth factor that promotes the growth and activation of the immune cells. Examples of suitable immune cell growth factors include interleukin (IL)-2, IL-7, IL-15, and IL-12, which can be used alone or in various combinations, such as IL-2 and IL-7, IL-2 and IL-15, IL-7 and IL-15, IL-2, IL-7 and IL-15, IL-12 and IL-7, IL-12 and IL-15, or IL-12 and IL2.

Therapeutically effective amounts of immune cells can be administered by a number of routes, including parenteral administration, for example, intravenous, intraperitoneal, intramuscular, intrasternal, or intraarticular injection, or infusion.

The therapeutically effective amount of immune cells for use in adoptive cell therapy is that amount that achieves a desired effect in a subject being treated. For instance, this can be the amount of immune cells necessary to inhibit advancement, or to cause regression of an autoimmune or alloimmune disease, or which is capable of relieving symptoms caused by an autoimmune disease, such as pain and inflammation. It can be the amount necessary to relieve symptoms associated with inflammation, such as pain, edema and elevated temperature. It can also be the amount necessary to diminish or prevent rejection of a transplanted organ.

The immune cell population can be administered in treatment regimens consistent with the disease, for example a single or a few doses over one to several days to ameliorate a disease state or periodic doses over an extended time to inhibit disease progression and prevent disease recurrence. The precise dose to be employed in the formulation will also depend on the route of administration, and the seriousness of the disease or disorder, and should be decided according to the judgment of the practitioner and each patient's circumstances. The therapeutically effective amount of immune cells will be dependent on the subject being treated, the severity and type of the affliction, and the manner of administration. In some embodiments, doses that could be used in the treatment of human subjects range from at least 3.8×10⁴, at least 3.8×10⁵, at least 3.8×10⁶, at least 3.8×10⁷, at least 3.8×10⁸, at least 3.8×10⁹, or at least 3.8×10¹⁰ immune cells/m². In a certain embodiment, the dose used in the treatment of human subjects ranges from about 3.8×10⁹ to about 3.8×10¹⁰ immune cells/m². In additional embodiments, a therapeutically effective amount of immune cells can vary from about 5×10⁶ cells per kg body weight to about 7.5×10⁸ cells per kg body weight, such as about 2×10⁷ cells to about 5×10⁸ cells per kg body weight, or about 5×10⁷ cells to about 2×10⁸ cells per kg body weight. The exact amount of immune cells is readily determined by one of skill in the art based on the age, weight, sex, and physiological condition of the subject. Effective doses can be extrapolated from dose-response curves derived from in vitro or animal model test systems.

The immune cells may be administered in combination with one or more other therapeutic agents for the treatment of the immune-mediated disorder. Combination therapies can include, but are not limited to, one or more anti-microbial agents (for example, antibiotics, anti-viral agents and anti-fungal agents), anti-tumor agents (for example, fluorouracil, methotrexate, paclitaxel, fludarabine, etoposide, doxorubicin, or vincristine), immune-depleting agents (for example, fludarabine, etoposide, doxorubicin, or vincristine), immunosuppressive agents (for example, azathioprine, or glucocorticoids, such as dexamethasone or prednisone), anti-inflammatory agents (for example, glucocorticoids such as hydrocortisone, dexamethasone or prednisone, or non-steroidal anti-inflammatory agents such as acetylsalicylic acid, ibuprofen or naproxen sodium), cytokines (for example, interleukin-10 or transforming growth factor-beta), hormones (for example, estrogen), or a vaccine. In addition, immunosuppressive or tolerogenic agents including but not limited to calcineurin inhibitors *(e.g.,* cyclosporin and tacrolimus); mTOR inhibitors *(e.g.,* Rapamycin); mycophenolate mofetil, antibodies (*e*.*g*., recognizing CD3, CD4, CD40, CD154, CD45, IVIG, or B cells); chemotherapeutic agents (*e*.*g*., Methotrexate, Treosulfan, Busulfan); irradiation; or chemokines, interleukins or their inhibitors (*e.g*., BAFF, IL-2, anti-IL-2R, IL-4, JAK kinase inhibitors) can be administered. Such additional pharmaceutical agents can be administered before, during, or after administration of the immune cells, depending on the desired effect. This administration of the cells and the agent can be by the same route or by different routes, and either at the same site or at a different site.

### B. Pharmaceutical Compositions

Also provided herein are pharmaceutical compositions and formulations comprising immune cells *(e.g.,* T cells or NK cells) and a pharmaceutically acceptable carrier.

Pharmaceutical compositions and formulations as described herein can be prepared by mixing the active ingredients (such as an antibody or a polypeptide) having the desired degree of purity with one or more optional pharmaceutically acceptable carriers (Remington's Pharmaceutical Sciences 22nd edition, 2012), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e*.*g*. Zn- protein complexes); and/or non-ionic surfactants such as polyethylene glycol (PEG).

### C. Combination Therapies

In certain embodiments, the compositions and methods of the present embodiments involve an immune cell population in combination with at least one additional therapy. The additional therapy may be radiation therapy, surgery (*e*.*g*., lumpectomy and a mastectomy), chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy.

In some embodiments, the additional therapy is the administration of small molecule enzymatic inhibitor or anti-metastatic agent. In some embodiments, the additional therapy is the administration of side- effect limiting agents *(e.g.,* agents intended to lessen the occurrence and/or severity of side effects of treatment, such as anti-nausea agents, *etc*.). In some embodiments, the additional therapy is radiation therapy. In some embodiments, the additional therapy is surgery. In some embodiments, the additional therapy is a combination of radiation therapy and surgery. In some embodiments, the additional therapy is gamma irradiation. In some embodiments, the additional therapy is therapy targeting PBK/AKT/mTOR pathway, HSP90 inhibitor, tubulin inhibitor, apoptosis inhibitor, and/or chemopreventative agent. The additional therapy may be one or more of the chemotherapeutic agents known in the art.

An immune cell therapy may be administered before, during, after, or in various combinations relative to an additional cancer therapy, such as immune checkpoint therapy. The administrations may be in intervals ranging from concurrently to minutes to days to weeks. In embodiments where the immune cell therapy is provided to a patient separately from an additional therapeutic agent, one would generally ensure that a significant period of time did not expire between the time of each delivery, such that the two compounds would still be able to exert an advantageously combined effect on the patient. In such instances, it is contemplated that one may provide a patient with the antibody therapy and the anti-cancer therapy within about 12 to 24 or 72 h of each other and, more particularly, within about 6-12 h of each other. In some situations it may be desirable to extend the time period for treatment significantly where several days (2, 3, 4, 5, 6, or 7) to several weeks (1, 2, 3, 4, 5, 6, 7, or 8) lapse between respective administrations.

Various combinations may be employed. For the example below a CAR immune cell therapy is "A" and an anti-cancer therapy is "B":

| | | | | | | | |
|---|---|---|---|---|---|---|---|
| A/B/A | B/A/B | B/B/A | A/A/B | A/B/B | B/A/A | A/B/B/B | B/A/B/B |
| B/B/B/A | B/B/A/B | | A/A/B/B | A/B/A/B | A/B/B/A | B/B/A/A | |
| B/A/B/A | B/A/A/B | | A/A/A/B | B/A/A/A | A/B/A/A | A/A/B/A | |

Administration of any compound or therapy of the present embodiments to a patient will follow general protocols for the administration of such compounds, taking into account the toxicity, if any, of the agents. Therefore, in some embodiments there is a step of monitoring toxicity that is attributable to combination therapy.

### 1. Chemotherapy

A wide variety of chemotherapeutic agents may be used in accordance with the present embodiments. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis.

Examples of chemotherapeutic agents include alkylating agents, such as thiotepa and cyclosphosphamide; alkyl sulfonates, such as busulfan, improsulfan, and piposulfan; aziridines, such as benzodopa, carboquone, meturedopa, and uredopa; ethylenimines and methylamelamines, including altretamine, triethylenemelamine, trietylenephosphoramide, triethiylenethiophosphoramide, and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards, such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas, such as carmustine, chlorozotocin, fotemustine, lomustine, nimustine, and ranimnustine; antibiotics, such as the enediyne antibiotics (*e*.*g*., calicheamicin, especially calicheamicin gammalI and calicheamicin omegaI1); dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, such as mitomycin C, mycophenolic acid, nogalarnycin, olivomycins, peplomycin, potfiromycin, puromycin, quelamycin, rodorubicin, streptonigrin, streptozocin, tubercidin, ubenimex, zinostatin, and zorubicin; anti-metabolites, such as methotrexate and 5-fluorouracil (5-FU); folic acid analogues, such as denopterin, pteropterin, and trimetrexate; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, and floxuridine; androgens, such as calusterone, dromostanolone propionate, epitiostanol, mepitiostane, and testolactone; anti-adrenals, such as mitotane and trilostane; folic acid replenisher, such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids, such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSKpolysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, *e*.*g*., paclitaxel and docetaxel gemcitabine; 6-thioguanine; mercaptopurine; platinum coordination complexes, such as cisplatin, oxaliplatin, and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan *(e.g.,* CPT-11); topoisomerase inhibitor RFS 2000; difluorometlhylomithine (DMFO); retinoids, such as retinoic acid; capecitabine; carboplatin, procarbazine,plicomycin, gemcitabien, navelbine, farnesyl-protein tansferase inhibitors, transplatinum, and pharmaceutically acceptable salts, acids, or derivatives of any of the above.

### 2. Radiotherapy

Other factors that cause DNA damage and have been used extensively include what are commonly known as γ-rays, X-rays, and/or the directed delivery of radioisotopes to tumor cells. Other forms of DNA damaging factors are also contemplated, such as microwaves, proton beam irradiation, and UV-irradiation. It is most likely that all of these factors affect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

### 3. Immunotherapy

The skilled artisan will understand that additional immunotherapies may be used in combination or in conjunction with methods of the embodiments. In the context of cancer treatment, immunotherapeutics, generally, rely on the use of immune effector cells and molecules to target and destroy cancer cells. Rituximab (RITUXAN^{®}) is such an example. The immune effector may be, for example, an antibody specific for some marker on the surface of a tumor cell. The antibody alone may serve as an effector of therapy or it may recruit other cells to actually affect cell killing. The antibody also may be conjugated to a drug or toxin (chemotherapeutic, radionuclide, ricin A chain, cholera toxin, pertussis toxin, etc.) and serve as a targeting agent. Alternatively, the effector may be a lymphocyte carrying a surface molecule that interacts, either directly or indirectly, with a tumor cell target. Various effector cells include cytotoxic T cells and NK cells

Antibody-drug conjugates (ADCs) comprise monoclonal antibodies (MAbs) that are covalently linked to cell-killing drugs and may be used in combination therapies. This approach combines the high specificity of MAbs against their antigen targets with highly potent cytotoxic drugs, resulting in "armed" MAbs that deliver the payload (drug) to tumor cells with enriched levels of the antigen. Targeted delivery of the drug also minimizes its exposure in normal tissues, resulting in decreased toxicity and improved therapeutic index. Exemplary ADC drugs inlcude ADCETRIS^{®} (brentuximab vedotin) and KADCYLA^{®} (trastuzumab emtansine or T-DM1).

In one aspect of immunotherapy, the tumor cell must bear some marker that is amenable to targeting, *i.e.,* is not present on the majority of other cells. Many tumor markers exist and any of these may be suitable for targeting in the context of the present embodiments. Common tumor markers include CD20, carcinoembryonic antigen, tyrosinase (p97), gp68, TAG-72, HMFG, Sialyl Lewis Antigen, MucA, MucB, PLAP, laminin receptor, erb B, and p155. An alternative aspect of immunotherapy is to combine anticancer effects with immune stimulatory effects. Immune stimulating molecules also exist including: cytokines, such as IL-2, IL-4, IL-12, GM-CSF, gamma-IFN, chemokines, such as MIP-1, MCP-1, IL-8, and growth factors, such as FLT3 ligand.

Examples of immunotherapies include immune adjuvants, *e*.*g*., Mycobacterium bovis, Plasmodium falciparum, dinitrochlorobenzene, and aromatic compounds); cytokine therapy, *e.g.,* interferons α, β, and γ, IL-1, GM-CSF, and TNF; gene therapy, *e.g.,* TNF, IL-1, IL-2, and p53; and monoclonal antibodies, *e.g.,* anti-CD20, anti-ganglioside GM2, and anti-p185. It is contemplated that one or more anti-cancer therapies may be employed with the antibody therapies described herein.

In some embodiments, the immunotherapy may be an immune checkpoint inhibitor. Immune checkpoints either turn up a signal (*e*.*g*., co-stimulatory molecules) or turn down a signal. Inhibitory immune checkpoints that may be targeted by immune checkpoint blockade include adenosine A2A receptor (A2AR), B7-H3 (also known as CD276), B and T lymphocyte attenuator (BTLA), cytotoxic T-lymphocyte-associated protein 4 (CTLA-4, also known as CD152), indoleamine 2,3-dioxygenase (IDO), killer-cell immunoglobulin (KIR), lymphocyte activation gene-3 (LAG3), programmed death 1 (PD-1), T-cell immunoglobulin domain and mucin domain 3 (TIM-3) and V-domain Ig suppressor of T cell activation (VISTA). In particular, the immune checkpoint inhibitors target the PD-1 axis and/or CTLA-4.

The immune checkpoint inhibitors may be drugs such as small molecules, recombinant forms of ligand or receptors, or, in particular, are antibodies, such as human antibodies. Known inhibitors of the immune checkpoint proteins or analogs thereof may be used, in particular chimerized, humanized or human forms of antibodies may be used. As the skilled person will know, alternative and/or equivalent names may be in use for certain antibodies mentioned in the present disclosure. Such alternative and/or equivalent names are interchangeable in the context of the present disclosure. For example it is known that lambrolizumab is also known under the alternative and equivalent names MK-3475 and pembrolizumab.

In some embodiments, the PD-1 binding antagonist is a molecule that inhibits the binding of PD-1 to its ligand binding partners. In a specific aspect, the PD-1 ligand binding partners are PDL1 and/or PDL2. In another embodiment, a PDL1 binding antagonist is a molecule that inhibits the binding of PDL1 to its binding partners. In a specific aspect, PDL1 binding partners are PD-1 and/or B7-1. In another embodiment, the PDL2 binding antagonist is a molecule that inhibits the binding of PDL2 to its binding partners. In a specific aspect, a PDL2 binding partner is PD-1. The antagonist may be an antibody, an antigen binding fragment thereof, an immunoadhesin, a fusion protein, or oligopeptide.

In some embodiments, the PD-1 binding antagonist is an anti-PD-1 antibody *(e.g.,* a human antibody, a humanized antibody, or a chimeric antibody). In some embodiments, the anti-PD-1 antibody is selected from the group consisting of nivolumab, pembrolizumab, and CT-011. In some embodiments, the PD-1 binding antagonist is an immunoadhesin (*e*.*g*., an immunoadhesin comprising an extracellular or PD-1 binding portion of PDL1 or PDL2 fused to a constant region (*e.g.,* an Fc region of an immunoglobulin sequence). In some embodiments, the PD-1 binding antagonist is AMP- 224. Nivolumab, also known as MDX-1106-04, MDX-1106, ONO-4538, BMS-936558, and OPDIVO^{®}, is an anti-PD-1 antibody that may be used. Pembrolizumab, also known as MK-3475, Merck 3475, lambrolizumab, KEYTRUDA^{®}, and SCH-900475, is an exemplary anti-PD-1 antibody. CT-011, also known as hBAT or hBAT-1, is also an anti-PD-1 antibody. AMP-224, also known as B7-DCIg, is a PDL2-Fc fusion soluble receptor.

Another immune checkpoint that can be targeted in the methods provided herein is the cytotoxic T-lymphocyte-associated protein 4 (CTLA-4), also known as CD152. The complete cDNA sequence of human CTLA-4 has the Genbank accession number L15006. CTLA-4 is found on the surface of T cells and acts as an "off" switch when bound to CD80 or CD86 on the surface of antigen-presenting cells. CTLA4 is a member of the immunoglobulin superfamily that is expressed on the surface of Helper T cells and transmits an inhibitory signal to T cells. CTLA4 is similar to the T-cell co-stimulatory protein, CD28, and both molecules bind to CD80 and CD86, also called B7-1 and B7-2 respectively, on antigen-presenting cells. CTLA4 transmits an inhibitory signal to T cells, whereas CD28 transmits a stimulatory signal. Intracellular CTLA4 is also found in regulatory T cells and may be important to their function. T cell activation through the T cell receptor and CD28 leads to increased expression of CTLA-4, an inhibitory receptor for B7 molecules.

In some embodiments, the immune checkpoint inhibitor is an anti-CTLA-4 antibody (*e.g*., a human antibody, a humanized antibody, or a chimeric antibody), an antigen binding fragment thereof, an immunoadhesin, a fusion protein, or oligopeptide.

Anti-human-CTLA-4 antibodies (or VH and/or VL domains derived therefrom) suitable for use in the present methods can be generated using methods well known in the art. Alternatively, art recognized anti-CTLA-4 antibodies can be used. An exemplary anti-CTLA-4 antibody is ipilimumab (also known as 10D1, MDX- 010, MDX- 101, and Yervoy^{®}) or antigen binding fragments and variants thereof. In other embodiments, the antibody comprises the heavy and light chain CDRs or VRs of ipilimumab. Accordingly, in one embodiment, the antibody comprises the CDR1, CDR2, and CDR3 domains of the VH region of ipilimumab, and the CDR1, CDR2 and CDR3 domains of the VL region of ipilimumab. In another embodiment, the antibody competes for binding with and/or binds to the same epitope on CTLA-4 as the above- mentioned antibodies. In another embodiment, the antibody has at least about 90% variable region amino acid sequence identity with the above-mentioned antibodies (*e.g.,* at least about 90%, 95%, or 99% variable region identity with ipilimumab).

### 4. Surgery

Approximately 60% of persons with cancer will undergo surgery of some type, which includes preventative, diagnostic or staging, curative, and palliative surgery. Curative surgery includes resection in which all or part of cancerous tissue is physically removed, excised, and/or destroyed and may be used in conjunction with other therapies, such as the treatment of the present embodiments, chemotherapy, radiotherapy, hormonal therapy, gene therapy, immunotherapy, and/or alternative therapies. Tumor resection refers to physical removal of at least part of a tumor. In addition to tumor resection, treatment by surgery includes laser surgery, cryosurgery, electrosurgery, and microscopically-controlled surgery (Mohs' surgery).

Upon excision of part or all of cancerous cells, tissue, or tumor, a cavity may be formed in the body. Treatment may be accomplished by perfusion, direct injection, or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### 5. Other Agents

It is contemplated that other agents may be used in combination with certain aspects of the present embodiments to improve the therapeutic efficacy of treatment. These additional agents include agents that affect the upregulation of cell surface receptors and GAP junctions, cytostatic and differentiation agents, inhibitors of cell adhesion, agents that increase the sensitivity of the hyperproliferative cells to apoptotic inducers, or other biological agents. Increases in intercellular signaling by elevating the number of GAP junctions would increase the anti-hyperproliferative effects on the neighboring hyperproliferative cell population. In other embodiments, cytostatic or differentiation agents can be used in combination with certain aspects of the present embodiments to improve the anti-hyperproliferative efficacy of the treatments. Inhibitors of cell adhesion are contemplated to improve the efficacy of the present embodiments. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with certain aspects of the present embodiments to improve the treatment efficacy.

### V. Articles of Manufacture or Kits

An article of manufacture or a kit is provided comprising immune cells is also provided herein. The article of manufacture or kit can further comprise a package insert comprising instructions for using the immune cells to treat or delay progression of cancer in an individual or to enhance immune function of an individual having cancer. Any of the antigen-specific immune cells described herein may be included in the article of manufacture or kits. Suitable containers include, for example, bottles, vials, bags and syringes. The container may be formed from a variety of materials such as glass, plastic (such as polyvinyl chloride or polyolefin), or metal alloy (such as stainless steel or hastelloy). In some embodiments, the container holds the formulation and the label on, or associated with, the container may indicate directions for use. The article of manufacture or kit may further include other materials desirable from a commercial and user standpoint, including other buffers, diluents, filters, needles, syringes, and package inserts with instructions for use. In some embodiments, the article of manufacture further includes one or more of another agent (*e.g.,* a chemotherapeutic agent, and anti-neoplastic agent). Suitable containers for the one or more agent include, for example, bottles, vials, bags and syringes.

### VI. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Development of Truncated EGFRvIII (Ev3) CAR

An Ev3-CAR was designed in which the Ev3 stalk serves as an integrative structure to transduce signals from antigen binding, such as by scFv, as well as an "off" switch, meaning it can be recognized by clinically accessible antibodies such as cetuximab to ablate CAR-positive immune cells (FIG. 1A). The Ev3-CAR may further comprise DAP12 signaling domain and is designated Ev3-DAP12-CAR. A schematic depicting Ev3 as compared to EGFR which comprise the endodomain and the EGF binding domain is shown in FIG. 2.

Retroviral vectors encoding the Ev3-CAR or Ev3-DAP12-CAR with various antigen binding domains were generated as depicted in FIGS. 3A-3D. The antigen-binding domain was both the scFv derived from the mouse anti-human antibody as well as a humanized scFv. The vectors comprise CD28 and CD3ζ signaling domains and, optionally, DAP12. The vectors also comprise a suicide gene, inducible caspase 9.

The CD19-specific CARs including CAR19.Ev3.CD28.CD3ζ, CAR19.Ev3.DAP12.CD3ζ or humanized CAR19.Ev3.CD3ζ were transduced into NK cells (FIG. 4A). The CAR-NK cells were tested for their efficacy at killing CD19⁺ Raji or K562 cells (FIG. 4B). The CAR19-Ev3-CD28-DAP12-CAR NK cells were observed to have the highest cytoxicity. Thus, the use of Ev3 in the hinge region of the CAR in combination with DAP12 endodomain produces CARs that are highly cytotoxic against target cells.

### Example 2 - Development of Humanized CARs

A platform was developed to generate humanized scFvs for use in producing humanized CARs. Due to structural constraints, some residues contribute more to antigen binding, thus 3-D modeling was used to identify potentially critical interactions to generate the humanized CARs. The stepwise process detailed below may be repeated iteratively to improve on previous cycles.

The CAR humanization workflow provided herein comprises 5 basic process modules as detailed below and depicted in FIG. 5A.

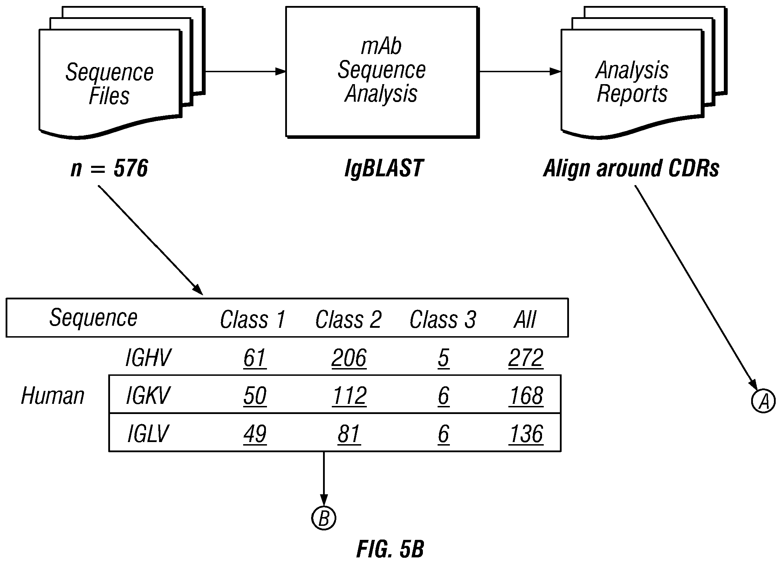

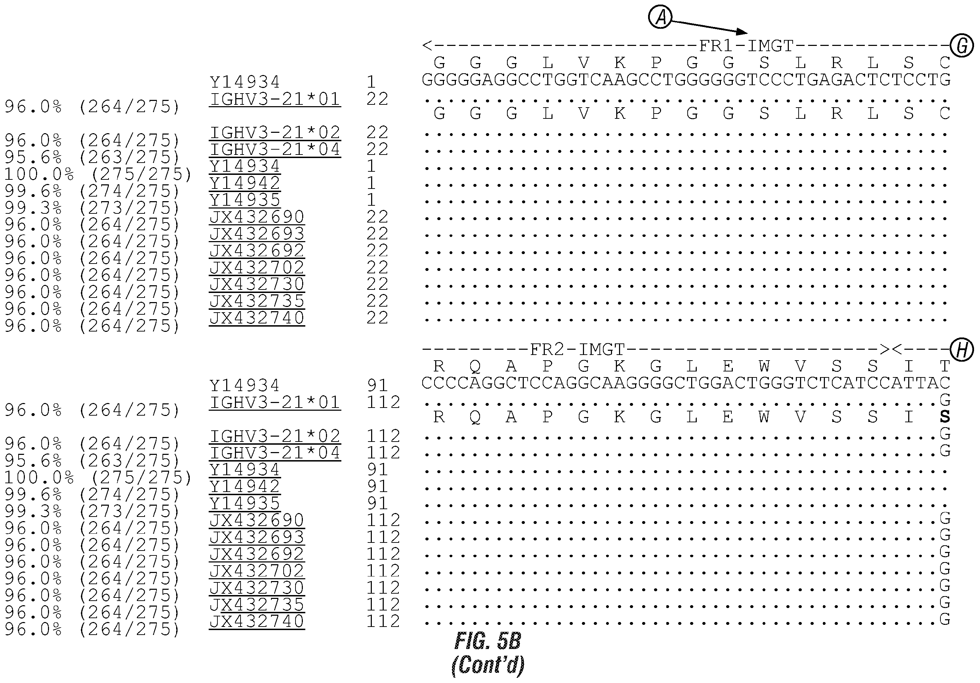

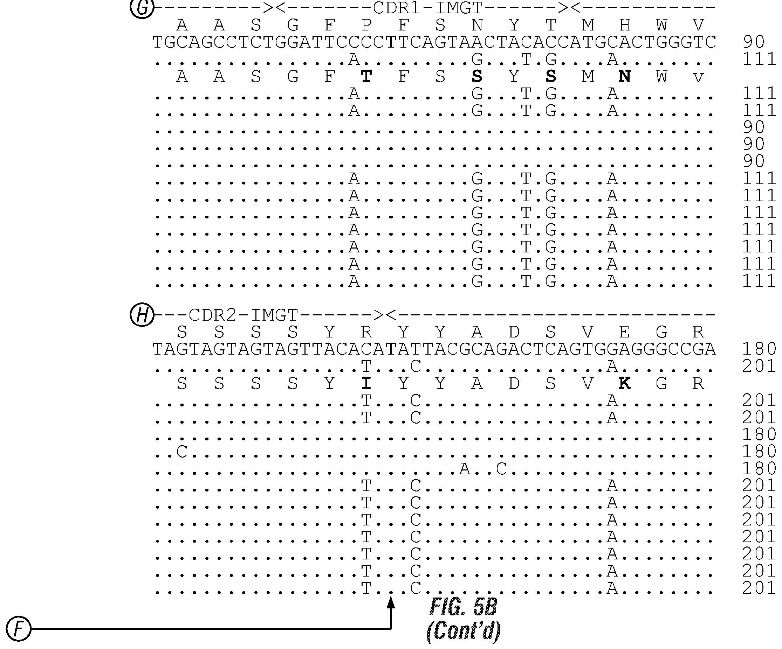

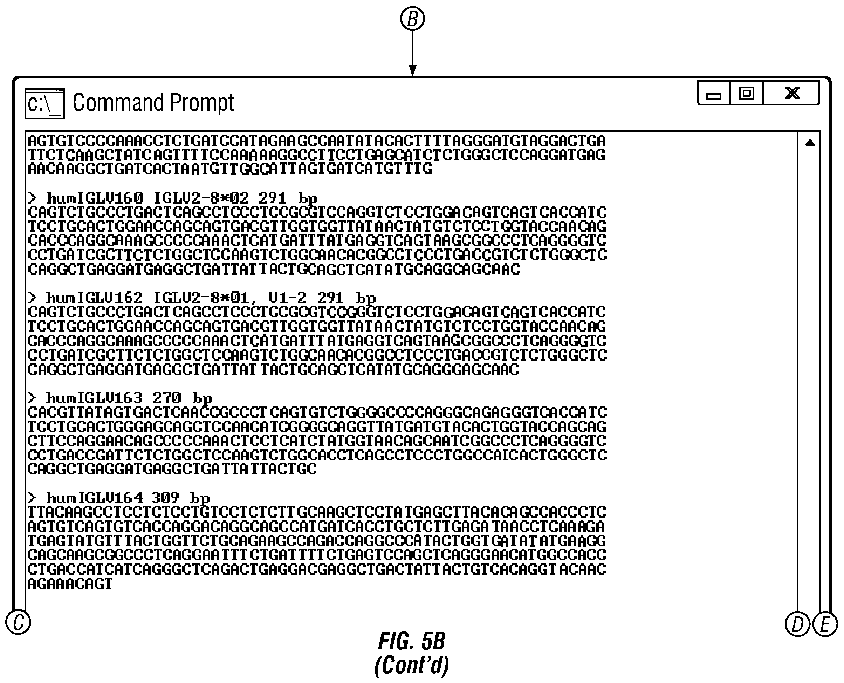

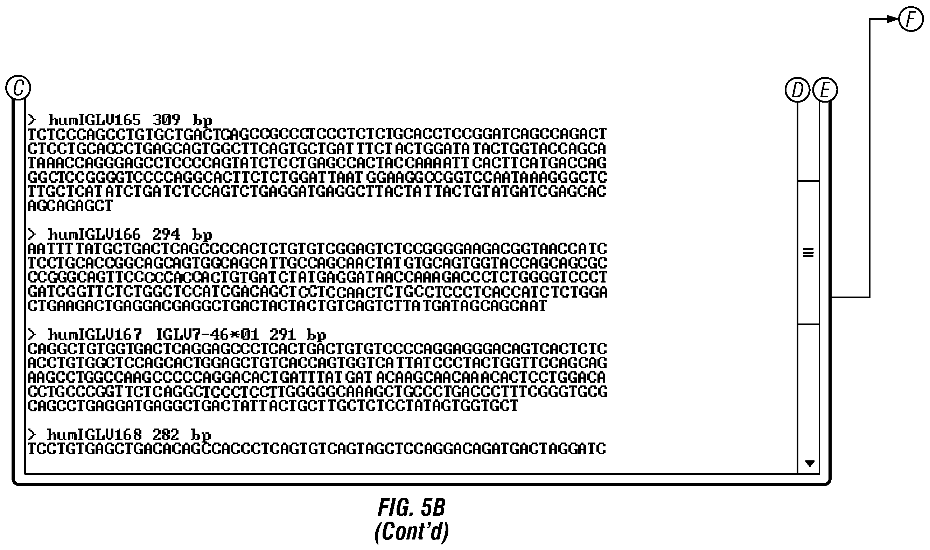

Module I comprises monoclonal antibody (mAb) sequence analysis. The BLAST program was harnessed in search of immunoglobulin (IG) sequences for the best match against the starting queries, such as the murine mAb sequences (FIG. 5B). BLAST can analyze nucleotide and amino acid sequences, either in series (one-by-one) or in parallel (in batches), as well as search against germline gene databases including curated sequence databases simultaneously thus maximizing the chance of getting the best matching variable (V gene. For reference sequences, databases of germline variable (V), diversity (D) and joining (J) genes were used to delineate IG V domain framework regions (FR1-4s) and complementarity determining regions (CDRs). For each antibody, the variable domains were analyzed for the light and heavy chains, each are encoded by multiple genes, including the gene V, D and J genes. The focus of this study was mainly on the V genes to compare variations in the context of defined regions, finding the boundaries for each FR and CDR regions and comparing with other IG sequences in a database.

Due to individual variations within the genomic sequences (*i.e.,* estimated at 1 single nucleotide polymorphism per 1,000 bp DNA sequence), the germline IG sequences comprise many thousands of allotypes (*i.e*., alleles or versions of a gene) (FIG. 6). Databases of human allotypes were generated and exploited for CAR humanization.

As an example, a humanized domain was derived using a particular human allotype by CDR-grafting to transform an anti-human CD19-binding domain VL and VH sequences from a mouse antibody (clone FMC63) (FIG. 7A). The CDR regions are underlined in FIG. 7A.

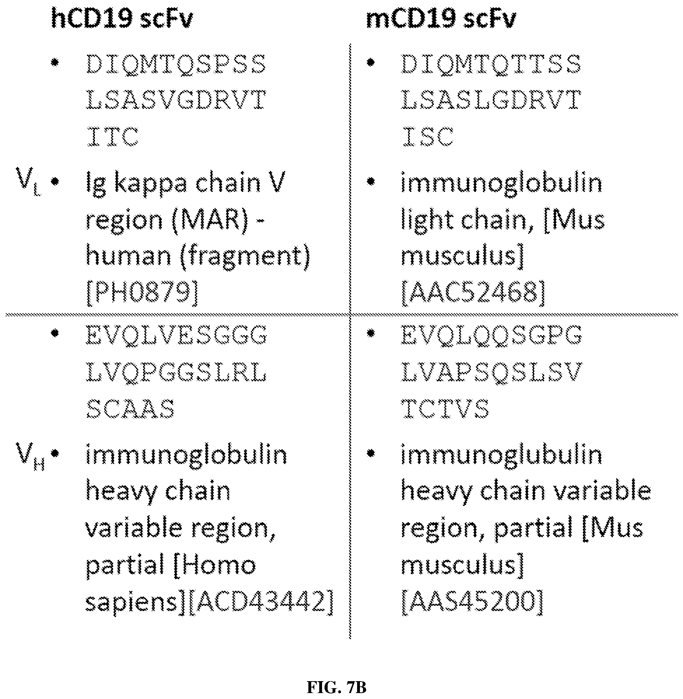

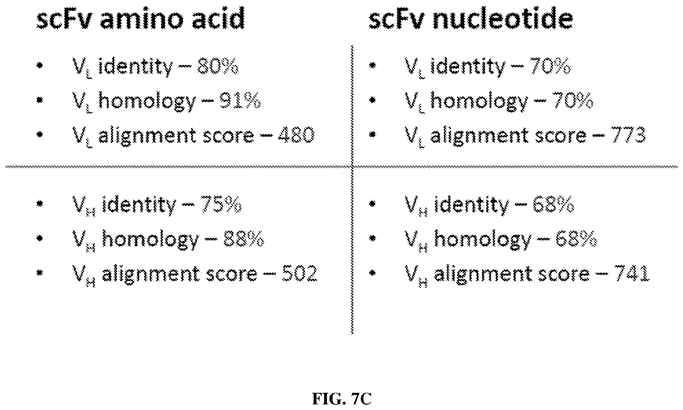

The sources of the framework sequences were confirmed by BLAST (Altshul *et al.,* 1997) analyses shown in FIG. 7B. Additionally, the sequence homologies and identities were readily quantified and are summarized in FIG. 7C. As a comparison, the sequences that were created de novo were compared to previously available sequences and substantial and readily distinguishable differences were detected at the amino acid and nucleotide levels.

Module II comprised 3D structure modeling. To identify critical residues for antigen binding, 3D structures were built to evaluate the constructs. SWISS-MODEL is a protein structure homology-modeler that was used to make 3-dimensional models of humanized mAbs. Structure files were extracted from SAbDab, a structural antibody database, as templates for homology modeling. As an example of characterizing the 3D conformation of CDR regions of VL and VH domains, homology modeling was employed using SWISS-MODEL (Arnold *et al.,* 2006; Benkert *et al.,* 2011) algorithms with the following primary amino acid sequences:

hCD19 V_{L} (SEQ ID NO:9): DIQMTQSPSSLSASVGDRVTITCRASQDIS
KYLNWYQQKPGKVPKLLIYHTSRLHSGVPDRFSGSGSGTDFTLTISSLQP
EDVATYYCQQGNTLPYTFGQGTKVEIKR

hCD19 V_H (SEQ ID NO:8): EVQLVESGGGLVQPGGSLRL
SCAASGVSLPDYGVSWVRQAPGKGLEWIGVIWGSETTYYNSALKSKFIISRD
NAKNSLYL QMNSLRAEDTAVYYCARHYYYGGSYAMDYWGQGTLVTVSS

The SWISS-MODEL template library (SMTL version 2017-06-28, PDB release 2017-06-23) was searched with BLAST (Altschul *et al.,* 1997) and HHBlits (Remmert *et al.,* 2012) for evolutionary related structures matching the target sequences hCD19 V_{L} and hCD19 V_{H}.

Overall, 13,535 templates were found for hCD19 V_{L}, with 2fgw.1.A showing the best fit (FIG. 8A) and used for further analysis. The target sequence (hCD19 V_{L}) was searched with BLAST against the primary amino acid sequence contained in the SMTL. A total of 761 templates were found. An initial HHblits profile had been built using the procedure outlined by Remmert *et al.,* followed by 1 iteration of HHblits against NR20. The profile thus obtained was then searched against all profiles of the SMTL. A total of 12,946 templates were found.

In all, 14,631 templates were found for hCD19 V_{H}, with 4uv7.1.B showing the best fit (FIG. 8A) and used for further analysis. The target sequence (hCD19 V_{L}) was searched with BLAST against the primary amino acid sequence contained in the SMTL. A total of 755 templates were found. An initial HHblits profile had been built using the procedure outlined by Remmert *et al.,* followed by 1 iteration of HHblits against NR20. The profile thus obtained was then searched against all profiles of the SMTL. A total of 13,964 templates were found.

*Template Selection:* For each identified template, the template's quality had been predicted from features of the target-template alignment. The templates with the highest quality were then been selected for model building.

*Model Building*: Models were built based on the target-template alignment using ProMod3. Coordinates which were conserved between the target and the template were copied from the template to the model. Insertions and deletions were remodeled using a fragment library. Side chains were then rebuilt. Finally, the geometry of the resulting model was regularized by using a force field. In case loop modeling with ProMod3 failed, an alternative model was built with PROMOD-II (Geux *et al.,* 1997).

*Model Quality Estimation:* The global and per-residue model quality had been assessed using the QMEAN scoring function (Benkert *et al.,* 2011). For improved performance, weights of the individual QMEAN terms had been trained specifically for SWISS-MODEL.

The 3-dimensional models of hCD19 V_{L} and hCD19 V_{H} showed that the CDR residues exposed the water-phase and were able to form paratopic surfaces consistently with their antigen binding properties.

Module III comprised human framework grafting. As part of the synthetic CAR building process, a robust and reliable CDR grafting technique was included in the platform technology. The antigen binding sequences (CDRs) were identified and spliced using custom analysis algorithms (a combination of traditional Kabat/IMGT and novel methods) from antibody variable domains into selective human framework sequences (carefully curated custom databases based homology and position of key residues) to create a panel of full length humanized scFvs for robust expression.

Fully humanized scFvs are an essential step in the process of incorporating the antigen-binding domains of mAbs into CARs for clinical application while reducing the possibility of human anti-mouse reactivity ensuring persistence and viability of CAR-modified therapeutic cells. The present CAR humanization platform's efficacy was shown by the de novo creation of a panel of at least 5 high quality, full-length, humanized CARs for expression characterization, functional verification, and pre-clinical testing.

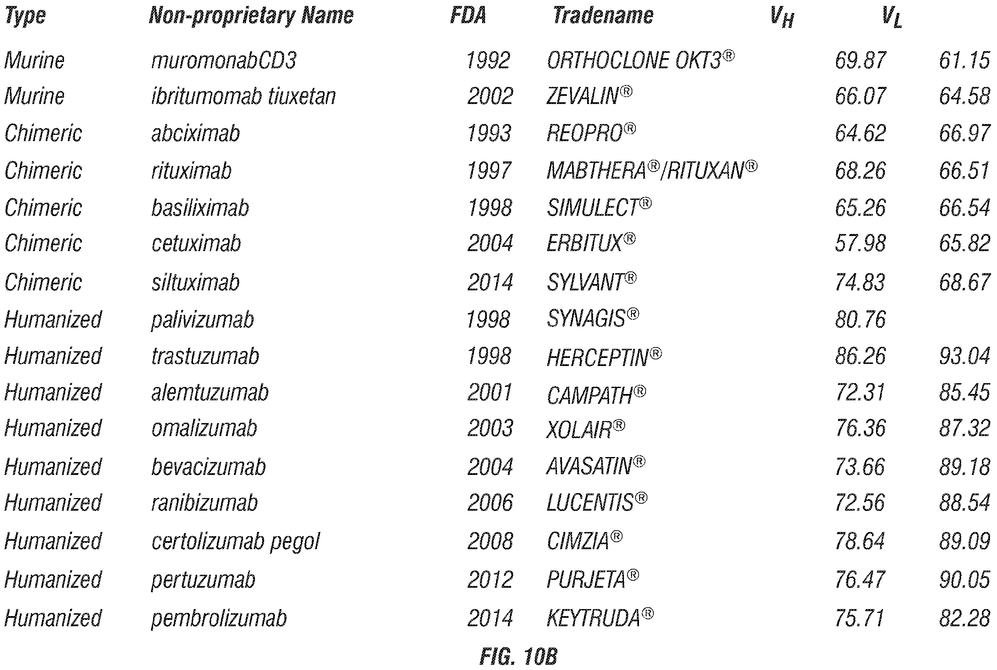

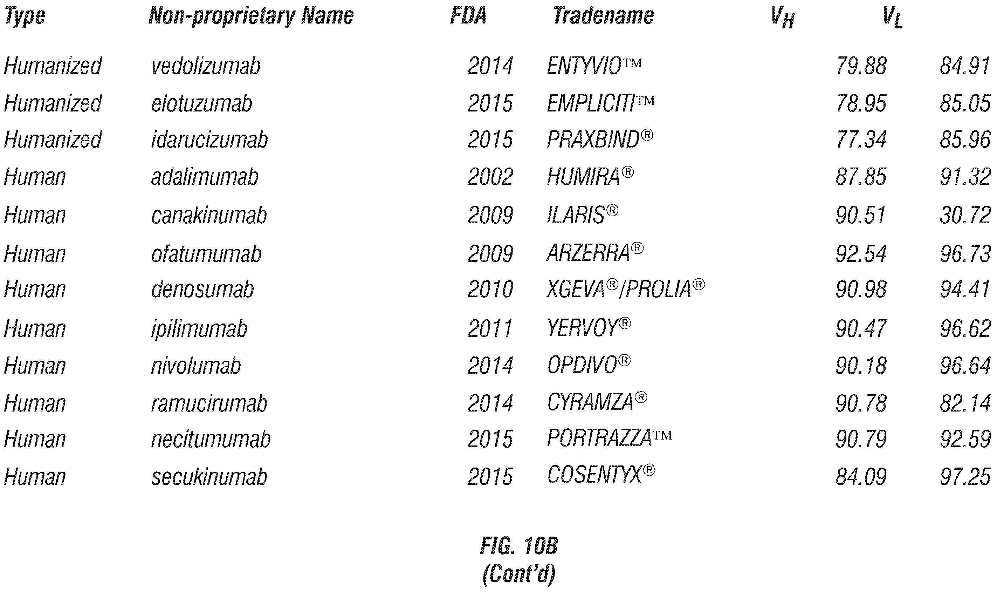

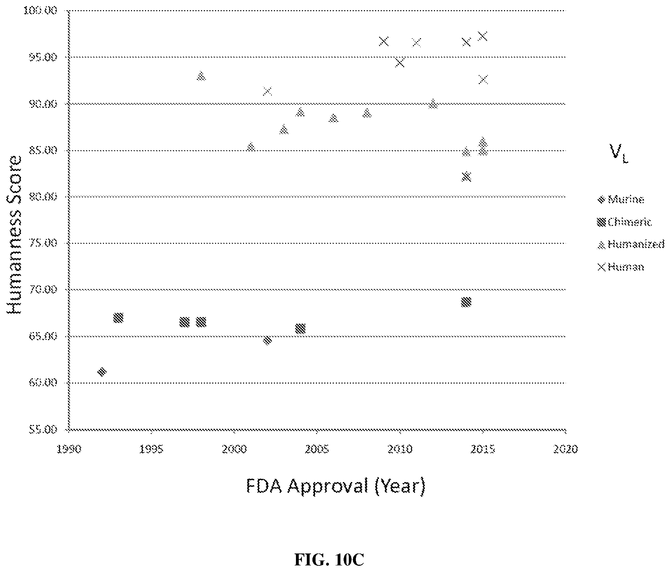

Module IV comprises humanization scoring. Scoring metrics were used to quantify and objectively evaluate the synthetic constructs as part of the CAR humanization process. The T20¹ scoring algorithm was applied to a series of commercially available and FDA-approved monoclonal antibodies (mAbs) as listed in FIG. 10B to test its utility. As expected, the V_{L} and V_{H} (FIG. 10C) sequences score higher with increasing degrees of humanization. Interestingly, V_{L} sequences trend higher in T20 scores as compared to paired V_{H} sequences.

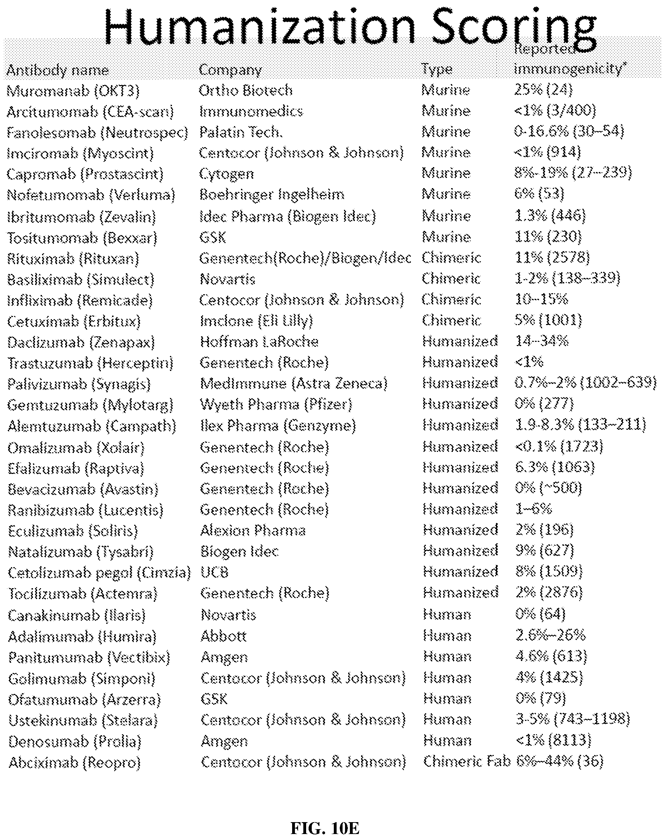

As a correlative analysis, information regarding FDA approved antibody therapeutics was assessed and the level of reported immunogenicity observed in patients from prescribing information in the public domain. As summarized in FIG. 10E, higher levels of humanization tend to be correlated with decreased instances of reported immunotoxicity in patients.

As an example of humanization scoring, T20 scores for the V_{L} and V_{H} sequences were evaluated before and after the humanization process for mCD19CAR vs hCD19CAR (FIGS. 10F-G). Indeed, higher T20 scores were observed for both processed fragments (humanized V_{L} and V_{H}). Since the workflow can be iterative, the CAR humanization process can be used to produce CARs with lower immunogenicity, better persistence, and robust function.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### REFERENCES

The following references, to the extent that they provide exemplary procedural or other details supplementary to those set forth herein, are specifically incorporated herein by reference.
Altschul, S.F., et al., Nucleic Acids Res, 25, 3389-3402, 1997.
Arnold, K., et al., Bioinformatics, 22, 195-201, 2006.
Benkert, P., et al., Bioinformatics, 27, 343-350, 2011.
Fedorov et al., Sci. Transl. Medicine, 5(215), 2013.
Gao SH, et al., BMC Biotechnology. 13:55, 2013.
Guex, N. et al., Electrophoresis, 18, 2714-2723, 1997.
Marco Biasini et al., Nucleic Acids Research, 42 (W1): W252-W258, 2014.
Remmert, M., et al., Nat Methods, 9, 173-175, 2012.
Sela-Culang et al., Frontiers in Immunology, 4:302, 2013.
Shah et al., PLoS One, 8:e776781, 2013.
Singh et al., Cancer Research, 68:2961-2971, 2008.
Singh et al., Cancer Research, 71:3516-3527, 2011.
U.S. Patent 7,109,304

### FURTHER ASPECTS OF THE DISCLOSURE:

1. A chimeric antigen receptor (CAR) comprising a truncated EGFRvIII (Ev3) in the hinge of said CAR, wherein said Ev3 hinge links an extracellular domain and at least one intracellular signaling domain.
2. The CAR of aspect 1, wherein the Ev3 hinge comprises a transmembrane domain and a non-functional ectodomain of EGFRvIII.
3. The CAR of aspect 1, wherein the Ev3 hinge does not comprise EGFRvIII endodomain.
4. The CAR of aspect 2, wherein the non-functional ectodomain of EGFRvIII has essentially no binding to epidermal growth factor (EGF).
5. The CAR of any one of aspects 1-4, wherein the Ev3 hinge comprises a truncated domain 1, truncated domain 2, domain 3, and domain 4 of EGFR.
6. The CAR of aspect 5, wherein the domain 3 and domain 4 of EGFR are not truncated.
7. The CAR of any one of aspects 1-4, wherein the Ev3 hinge comprises an amino acid sequence with at least 80% sequence identity to SEQ ID NO:2.
8. The CAR of any one of aspects 1-4, wherein the Ev3 hinge comprises an amino acid sequence with at least 95% sequence identity to SEQ ID NO:2.
9. The CAR of any one of aspects 1-4, wherein the Ev3 hinge comprises an amino acid sequence of SEQ ID NO:2.
10. The CAR of any one of aspects 1-4, wherein the Ev3 hinge is encoded by a nucleotide sequence with at least 80% sequence identity to SEQ ID NO:1.
11. The CAR of any one of aspects 1-4, wherein the Ev3 hinge is encoded by a nucleotide sequence with at least 95% sequence identity to SEQ ID NO:1.
12. The CAR of any one of aspects 1-4, wherein the Ev3 hinge is encoded by a nucleotide sequence of SEQ ID NO:1.
13. The CAR of aspect 1, wherein the extracellular domain of the CAR comprises an antigen-binding domain selected from the group consisting of F(ab')2, Fab', Fab, Fv, and scFv.
14. The CAR of aspect 13, wherein the antigen-binding domain comprises a scFv.
15. The CAR of aspect 14, wherein the scFv is further defined as a humanized scFv.
16. The CAR of aspect any one of aspects 13-15, wherein the antigen binding region of the CAR binds one or more tumor associated antigens.
17. The CAR of aspect 16, wherein the one or more tumor associated antigens are selected from the group consisting of CD19, CD319 (CS1), ROR1, CD20, carcinoembryonic antigen, alphafetoprotein, CA-125, MUC-1, epithelial tumor antigen, melanoma-associated antigen, mutated p53, mutated ras, HER2/Neu, ERBB2, folate binding protein, HIV-1 envelope glycoprotein gp120, HIV-1 envelope glycoprotein gp41, GD2, CD5, CD123, CD23, CD30, CD56, c-Met, mesothelin, GD3, HERV-K, IL-11Ralpha, kappa chain, lambda chain, CSPG4, ERBB2, WT-1, TRAIL/DR4, VEGFR2, CD33, CD47, CLL-1, U5snRNP200, CD200, BAFF-R, BCMA, and CD99.
18. The CAR of aspect 16, wherein the one or more tumor-associated antigens are CD19, CD319, CD123, CD5, ROR1, CD33, CD99, CLL-1, and/or mesothelin.
19. The CAR of any one of aspects 13-15, wherein the scFV does not comprise an EGFR binding domain.
20. The CAR of aspect 1, wherein the at least one signaling domain comprises CD3ξ, CD28, OX40/CD134, 4-1BB/CD137, FcεRIγ, ICOS/CD278, ILRB/CD122, IL-2RG/CD132, DAP12, CD70, CD40, or a combination thereof.
21. The CAR of aspect 1, wherein the at least one signaling domain comprises DAP12.
22. The CAR of aspect 1, wherein the CAR comprises IL-15.
23. The CAR of aspect 1, wherein the CAR comprises CD3ζ, CD28, DAP12, and IL-15.
24. The CAR of aspect 1, wherein the CAR further comprises a suicide gene.
25. The CAR of aspect 24, wherein the suicide gene is inducible caspase 9.
26. An isolated antigen-specific humanized single chain variable fragment (scFv) comprising a light chain variable region (V_{L}) and a heavy chain variable region (V_{H}), wherein the scFv is:
   (a) CD19-specific and wherein the V_{L} comprises an amino acid sequence of SEQ ID NO:7 and the V_{H} comprises an amino acid sequence of SEQ ID NO:8;
   (b) CD123-specific and wherein the V_{L} comprises an amino acid sequence of SEQ ID NO:13 and the V_{H} comprises an amino acid sequence of SEQ ID NO:14;
   (c) mesothelin-specific and wherein the V_{L} comprises an amino acid sequence of SEQ ID NO:19 and the V_{H} comprises an amino acid sequence of SEQ ID NO:20;
   (d) ROR1-specific and wherein the V_{L} comprises an amino acid sequence of SEQ ID NO:25 and the V_{H} comprises an amino acid sequence of SEQ ID NO:26;
   (e) CD5-specific and wherein the V_{L} comprises an amino acid sequence of SEQ ID NO:31 and the V_{H} comprises an amino acid sequence of SEQ ID NO:32;
   (f) CLL-1-specific and wherein the V_{L} comprises an amino acid sequence of SEQ ID NO:59 and the V_{H} comprises an amino acid sequence of SEQ ID NO:60;
   (g) CD99-specific and wherein the V_{L} comprises an amino acid sequence of SEQ ID NO:63 and the V_{H} comprises an amino acid sequence of SEQ ID NO:64 or
   (h) a sequence with 90% sequence identity to framework regions of any one of (a)-(g).
27. The humanized scFv of aspect 26, wherein the scFv is CD19-specific and wherein the V_{L} comprises an amino acid sequence of SEQ ID NO:7 and the V_{H} comprises an amino acid sequence of SEQ ID NO:8.
28. The humanized scFv of aspect 27, wherein the CD19-specific scFv comprises an amino acid sequence of SEQ ID NO:6.
29. The humanized scFv of aspect 26, wherein the scFv is CD123-specific and wherein the V_{L} comprises an amino acid sequence of SEQ ID NO:13 and the V_{H} comprises an amino acid sequence of SEQ ID NO:14.
30. The humanized scFv of aspect 29, wherein the CD123-specific scFv comprises an amino acid sequence of SEQ ID NO:12.
31. The humanized scFv of aspect 26, wherein the scFv is mesothelin-specific and wherein the V_{L} comprises an amino acid sequence of SEQ ID NO:19 and the V_{H} comprises an amino acid sequence of SEQ ID NO:20.
32. The humanized scFv of aspect 31, wherein the mesothelin-specific scFv comprises an amino acid sequence of SEQ ID NO:18.
33. The humanized scFv of aspect 26, wherein the scFv is ROR1-specific and wherein the V_{L} comprises an amino acid sequence of SEQ ID NO:25 and the V_{H} comprises an amino acid sequence of SEQ ID NO:26.
34. The humanized scFv of aspect 33, wherein the ROR1-specific comprises an amino acid sequence of SEQ ID NO:24.
35. The humanized scFv of aspect 26, wherein the scFv is CD5-specific and wherein the V_{L} comprises an amino acid sequence of SEQ ID NO:31 and the V_{H} comprises an amino acid sequence of SEQ ID NO:32.
36. The humanized scFv of aspect 35, wherein the CD5-specific comprises an amino acid sequence of SEQ ID NO:30.
37. The humanized scFv of aspect 26, wherein the scFv is CD99-specific and wherein the V_{L} comprises an amino acid sequence of SEQ ID NO:63 and the V_{H} comprises an amino acid sequence of SEQ ID NO:64.
38. The humanized scFv of aspect 35, wherein the CD99-specific comprises an amino acid sequence of SEQ ID NO:62.
39. The humanized scFv of any one of aspects 26-38, wherein the scFv comprises an amino acid sequence with at least 95% sequence identity to framework regions an amino acid sequence of SEQ ID NO:6, 12, 18, 24, or 30.
40. The humanized scFv of any one of aspects 26-38, wherein the scFv comprises an amino acid sequence with at least 98% sequence identity to framework regions an amino acid sequence of SEQ ID NO:6, 12, 18, 24, or 30.
41. An isolated polynucleotide encoding an isolated humanized scFv of any one of aspects 26-38.
42. The isolated polynucleotide of aspect 41, wherein the polynucleotide comprises SEQ ID NO:3, 9, 15, 21, or 27.
43. An expression vector comprising an isolated polynucleotide of aspect 41.
44. The vector of aspect 43, wherein the vector is further defined as a viral vector.
45. The CAR of aspect 15, wherein the humanized scFv is a scFv according to any one of aspects 26-38.
46. An isolated nucleic acid comprising a nucleotide sequence encoding the CAR according to aspects 1-25 and 45.
47. A host cell engineered to express a CAR comprising a humanized scFv according to any one of aspects 26-38 and/or a truncated EGFRvIII (Ev3) in the hinge region of said CAR.
48. The cell of aspect 47, wherein said CAR is according to any one of aspects 1-25.
49. The cell of aspect 47, wherein the host cell is further defined as a CAR immune cell.
50. The cell of aspect 49, wherein the immune cell is a T cell, peripheral blood lymphocyte, NK cell, invariant NK cell, NKT cell, or stem cell.
51. The cell of aspect 49, wherein the immune cell is a T cell or a NK cell.
52. The cell of aspect 50, wherein the stem cell is a mesenchymal stem cell (MSC) or an induced pluripotent stem (iPS) cell.
53. The cell of aspect 49, wherein the immune cell is derived from an iPS cell.
54. The cell of aspect 50, wherein the T cell is a CD8⁺ T cell, CD4⁺ T cell, or gamma-delta T cell.
55. The cell of aspect 50, wherein the T cell is a cytotoxic T lymphocyte (CTL).
56. The cell of aspect 49, wherein the immune cell is allogeneic.
57. The cell of aspect 49, wherein the immune cell is autologous.
58. The cell of aspect 49, wherein the immune cell is isolated from peripheral blood, cord blood, or bone marrow.
59. The cell of aspect 49, wherein the immune cell is isolated from cord blood.
60. The cell of aspect 59, wherein the cord blood is pooled from 2 or more individual cord blood units.
61. The cell of aspect 47, wherein DNA encoding the CAR is integrated into the genome of the cell.
62. A pharmaceutical composition comprising a population of cells according to any one of aspects 47-61.
63. A composition comprising a population of cells of any one of aspects 47-61 for use in the treatment of an immune-related disorder
64. A method of treating an immune-related disorder in a subject comprising administering an effective amount of cells of any one of aspects 47-61 to the subject.
65. The method of aspect 64, wherein the immune-related disorder is a cancer, autoimmune disorder, graft versus host disease, allograft rejection, or inflammatory condition.
66. The method of aspect 64, wherein the immune-related disorder is an inflammatory condition and the immune cells have essentially no expression of glucocorticoid receptor.
67. The method of aspect 64, wherein the immune cells are autologous.
68. The method of aspect 64, wherein the immune cells are allogeneic.
69. The method of aspect 64, wherein the immune-related disorder is a cancer.
70. The method of aspect 69, wherein the cancer is a solid cancer or a hematologic malignancy.
71. The method of aspect 64, further comprising administering at least a second therapeutic agent.
72. The method of aspect 71, wherein the at least a second therapeutic agent comprises chemotherapy, immunotherapy, surgery, radiotherapy, or biotherapy.
73. The method of aspect 71, wherein the immune cells and/or the at least a second therapeutic agent are administered intravenously, intraperitoneally, intratracheally, intratumorally, intramuscularly, endoscopically, intralesionally, percutaneously, subcutaneously, regionally, or by direct injection or perfusion.
74. The method of aspect 64, further comprising administering an anti-EGFR antibody.
75. The method of aspect 74, wherein the anti-EGFR antibody is a monoclonal antibody.
76. The method of aspect 75, wherein the anti-EGFR antibody is cetuximab or panitumumab.
77. The method of any one of aspects 74-76, wherein the anti-EGFR antibody selectively ablates Ev3-CAR immune cells through antibody-dependent cell-mediated cytotoxicity (ADCC).
78. The method of any one of aspects 74-76, wherein the anti-EGFR antibody is fused to a detectable tag and/or a cytotoxic agent.
79. The method of aspect 78, further comprising imaging the detectable tag, thereby detecting the Ev3-CAR immune cells.
80. The method of any one of aspects 74-76, wherein the anti-EGFR antibody is fused to a cytotoxic agent.
81. The method of aspect 79, wherein the cytotoxic agent induces activation of the suicide gene in the Ev3-CAR immune cells.
82. The method of aspect 78, wherein the cytotoxic agent results in the ablation of the Ev3-CAR immune cells.

## Claims

1. A polynucleotide, encoding:
a) a chimeric antigen receptor (CAR) comprising a CD5-specific single chain variable fragment (scFv) comprising a light chain variable region (VL) and a heavy chain variable region (VH); and
b) IL-15.

2. The polynucleotide of claim 1, wherein the V_{L} comprises an amino acid sequence of SEQ ID NO:31 or a sequence having at least 85% sequence identity to framework regions of SEQ ID NO:31.

3. The polynucleotide of claim 1 or claim 2, wherein the V_{H} comprises an amino acid sequence of SEQ ID NO:32 or a sequence with at least 85% sequence identity to framework regions of SEQ ID NO:32.

4. The polynucleotide of any one of claims 1-3, wherein the V_{L} comprises an amino acid sequence of SEQ ID NO:31 and wherein the V_{H} comprises an amino acid sequence of SEQ ID NO:32.

5. The polynucleotide of any one of claims 1-4, wherein the scFv comprises the sequence set forth in SEQ ID NO:30 or an amino acid sequence having at least 85% sequence identity thereto.

6. The polynucleotide of any one of claims 1-5, wherein the scFV comprises the CDRs of the scFv sequence set forth in SEQ ID NO: 30.

7. The polynucleotide of any one of claims 1-6, wherein the polynucleotide comprises the nucleic acid sequence of SEQ ID NO: 46.

8. The polynucleotide of any one of claims 1-7, wherein the IL-15 comprises the amino acid sequence of SEQ ID NO: 56.

9. The polynucleotide of any one of claims 1-8, wherein the CAR comprises:
(i) a CD3ζ, CD28, and DAP12; or
(ii) CD3ζ, CD28, and Ev3.

10. The polynucleotide of any one of claims 1-9, wherein the CAR further comprises a suicide gene, optionally wherein the suicide gene is inducible caspase 9.

11. An expression vector comprising the polynucleotide of any one of claims 1-10, optionally wherein the expression vector is a viral vector.

12. A host cell engineered to express the polynucleotide of any one of claims 1-10.

13. The host cell of claim 12, wherein the host cell is an immune cell.

14. The host cell of claim 13, wherein the immune cell is a T cell or an NK cell.

15. A pharmaceutical composition comprising a population of cells according to any one of claims 12-14.

16. The cells according to any one of claims 12-14 for use in a method of treating an immune-related disorder in a subject, the method comprising administering an effective amount of the immune cells to the subject.
